# EUROPEAN PATENT SPECIFICATION

(11) **EP 4 514 813 B1**
(45) Date of publication and mention of the grant of the patent: **05.08.2026**
(21) Application number: 23722096.7
(22) Date of filing: 28.04.2023
(51) Int. Cl.: C07H 15/207, C07H 15/26, A61P 35/00, A61K 31/7042

(54) **PSEUDO-DISACCHARIDE COMPOUNDS**
PSEUDO-DISACCHARID-VERBINDUNGEN
COMPOSÉS DE PSEUDO-DISACCHARIDE

(30) Priority: 29.04.2022 NL 2031742
(43) Date of publication of application: 05.03.2025
(73) Proprietor: Universiteit Leiden, 2311 EZ Leiden (NL); University of York, York YO10 5DD (GB)
(72) Inventor: OVERKLEEFT, Herman, 2311 EZ Leiden (NL); LIT, Vincent, 2311 EZ Leiden (NL); DE BOER, Casper, 2311 EZ Leiden (NL); SCHRÖDER, Sybrin, 2311 EZ Leiden (NL); DAVIES, Gideon, York Yorkshire YO10 5DD (GB); WU, Liang, York Yorkshire YO10 5DD (GB); BORLANDELLI, Valentina, 2311 EZ Leiden (NL)
(74) Representative: HGF
(86) International application number: PCT/NL2023/050231
(87) International publication number: WO 2023/211280

(56) References cited:
- CHEN YURONG ET AL: "Activity-Based Protein Profiling of Retaining [alpha]-Amylases in Complex Biological Samples", vol. 143, no. 5, 10 February 2021 (2021-02-10), pages 2423 - 2432, XP093013815, ISSN: 0002-7863, Retrieved from the Internet <URL:http://pubs.acs.org/doi/pdf/10.1021/jacs.0c13059> DOI: 10.1021/jacs.0c13059
- SCHRÖDER SYBRIN P. ET AL: "Dynamic and Functional Profiling of Xylan-Degrading Enzymes in Aspergillus Secretomes Using Activity-Based Probes", vol. 5, no. 6, 26 June 2019 (2019-06-26), pages 1067 - 1078, XP093013820, ISSN: 2374-7943, Retrieved from the Internet <URL:http://pubs.acs.org/doi/pdf/10.1021/acscentsci.9b00221> DOI: 10.1021/acscentsci.9b00221
- J. P. RITCHIE ET AL: "SST0001, a Chemically Modified Heparin, Inhibits Myeloma Growth and Angiogenesis via Disruption of the Heparanase/Syndecan-1 Axis", CLINICAL CANCER RESEARCH, vol. 17, no. 6, 21 January 2011 (2011-01-21), US, pages 1382 - 1393, XP055267176, ISSN: 1078-0432, DOI: 10.1158/1078-0432.CCR-10-2476
- VITO FERRO ET AL: "Discovery of PG545: A Highly Potent and Simultaneous Inhibitor of Angiogenesis, Tumor Growth, and Metastasis", JOURNAL OF MEDICINAL CHEMISTRY, vol. 55, no. 8, 13 April 2012 (2012-04-13), US, pages 3804 - 3813, XP055493094, ISSN: 0022-2623, DOI: 10.1021/jm201708h

## Description

The invention is defined in the appended claims. Any disclosure going beyond the scope of said claims is only intended for illustrative purposes and should not be construed as being part of the invention. Furthermore, any reference to a method of treatment should be interpreted as compound for use in a method of treatment.

This invention relates to compounds comprising pseudo-disaccharide compounds, as well as a pharmaceutically acceptable salts, stereoisomers, or solvates thereof. The compounds of the invention may be substrates for, or inhibitors of, heparanase (HPSE). Also provided are pharmaceutical compositions comprising the compounds of the invention, as well as various medical and non-medical uses of the compounds and compositions.

### BACKGROUND

Cancer progression is accompanied by extensive changes to the tumor microenvironment, which supports the proliferation and dissemination of malignant cells. Extracellular matrix (ECM) remodeling by cancer cells and their associated stromal cells utilizes a host of enzymes that act upon both proteins and carbohydrates within the ECM, resulting in profound compositional changes that drive growth of the primary tumor, and prime distant sites for metastasis (Winkler et al., Nat Commun 11, 5120 (2020)).

Heparan sulfate proteoglycans (HSPGs) are a fundamental class of ECM constituent, comprised of pericellular and extracellular core proteins conjugated to one or more chains of the glycosaminoglycan polysaccharide heparan sulfate (HS) (Sarrazin et al., Cold Spring Harb Perspect Biol 3 (2011)). HSPGs mediate essential interactions throughout the extracellular matrix (ECM), providing signals that regulate cellular growth and development.

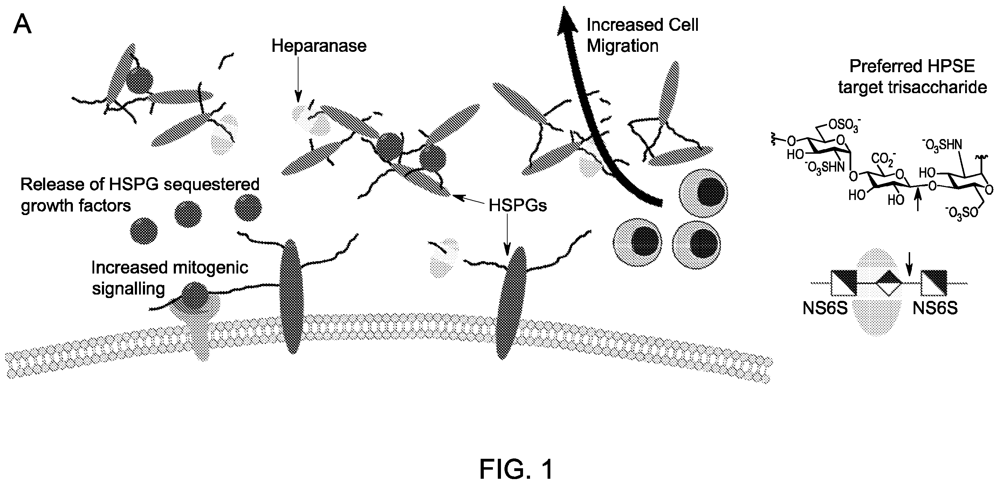

Heparanase (HPSE) is a mammalian *endo*-β-D-glucuronidase that plays a key role in HS polysaccharide degradation (Wu et al., Nat Struct Mol Biol 22, 1016-1022 (2015)). Initially produced as a proenzyme (proHPSE), HPSE is matured within lysosomes by proteolysis of a linker peptide that obstructs its active site. Mature HPSE remains active within the lysosomes, but can also be secreted into the extracellular space, where it aids ECM remodeling during tissue development and homeostasis. In contrast to its normal role in ECM maintenance, pathological HPSE overexpression strongly drives the growth of aggressive metastatic cancers (Jayatilleke et al., J Transl Med 18, 453 (2020)); excessive HSPG degradation within the ECM directly facilitates cancer cell migration to and from the vasculature (Nakajima et al., Science 220, 611-3 (1983); Vlodavsky et al., Cancer Res 43, 2704-11 (1983)), whilst growth factors and cytokines released upon HSPG degradation stimulate proliferation and angiogenesis (Vlodavsky et al., J Clin Invest 108, 341-7 (2001); Elkin et al., FASEB J 15, 1661-3 (2001)) (Fig 1a).

HPSE is the sole human enzyme responsible for extracellular HS polysaccharide degradation. Accordingly, there is intense interest in its inhibition as an anticancer strategy.

Current best-in-class HPSE inhibitors are typically polyanionic oligo-/polysaccharides that mimic the physicochemical properties of the natural HPSE inhibitor heparin, a glycosaminoglycan closely related to HS (Ritchie et al., Clin Cancer Res 17, 1382-93 (2011); Zhou et al., PLoS One 6, e21106 (2011); Parish et al., Cancer Res 59, 3433-3441 (1999); Ferro et al. J Med Chem 55, 3804-13 (2012)). The heparin-like properties of these inhibitors, along with their structural heterogeneity, results in their targeting of multiple proteins *in vivo*; this leads to unwanted pleiotropic effects such as anticoagulation and growth factor binding, thereby complicate the clinical use of these compounds.

There is therefore a need to develop compounds which demonstrate high potency and selectivity for HPSE as anticancer agents.

### BRIEF SUMMARY OF THE DISCLOSURE

The invention provides compounds comprising a 'cyclophellitol-inspired' pseudo-disaccharide, as well as a pharmaceutically acceptable salt, stereoisomer, or solvate thereof. The compounds of may be substrates for the heparanase (HPSE).

Without wishing to be bound by theory, it is thought that the current lack of effective HPSE inhibitors is due to the extensive active site cleft of this enzyme, which facilitates multiple interactions with structurally complex HS polysaccharide substrates. The extended binding surface presents a challenging target for pharmacological intervention; known compounds typically comprise a polyanionic saccharide structure similar to that of HS itself, which often target multiple proteins *in vivo,* resulting in undesirable pleiotropic effects.

In contrast, pseudo-disaccharide compounds of the invention, inspired by the naturally occurring molecule cyclophellitol, are believed to irreversibly inhibit the enzyme HPSE with exemplary such compounds having nanomolar potency and high selectivity. Without wishing to be bound by theory, it is thought that modifying the cyclophellitol-derived warhead with a nonreducing α-1,4 glucosaminyl moiety (or similar) blocks inhibitor engagement with exo-acting β-glucuronidases such as GUSB, whilst reactivity with HPSE itself is enhanced. The compounds of the invention thus represent highly selective HPSE inhibitors. The inhibition of HPSE is thought to minimise HSPG degradation in the extracellular matrix, thereby reducing cancer cell migration. Thus, the compounds of the invention can represent highly selective anti-cancer agents. In particular, the compounds of the invention (typically) act to reduce cancer metastasis.

Although covalency has historically been avoided during pharmacological development for fears of off-target toxicity, this inhibition modality may offer several advantages compared to traditional competitive inhibition. For example, covalent inhibitors can effectively target challenging protein sites, by virtue of their ability to label despite poor or transient initial enzyme binding (Sutanto et al., RSC Med Chem 11, 876-884 (2020)). Improved potency is also readily achieved by time-dependent accumulation of inhibition, an effect that likely contributes to the good (e.g. nanomolar) HPSE inhibitory potencies observed for compounds of the invention. HPSE has a long cellular lifetime of >72 h (t_{1/2} ~30 h) (Gingis-Velitski et al., J Biol Chem 279, 44084-92 (2004)), and thus is well-suited for targeting by irreversible inhibitors, whose effect will persist until the protein molecule is degraded. Potential off-target effects of the compounds of the invention are minimized by their mechanism-based nature of activation: efficient inhibition requires binding to a protein site that also contains the biochemical machinery necessary to process the cyclophellitol warhead. These criteria are likely only well-satisfied by HPSE *in vivo.*

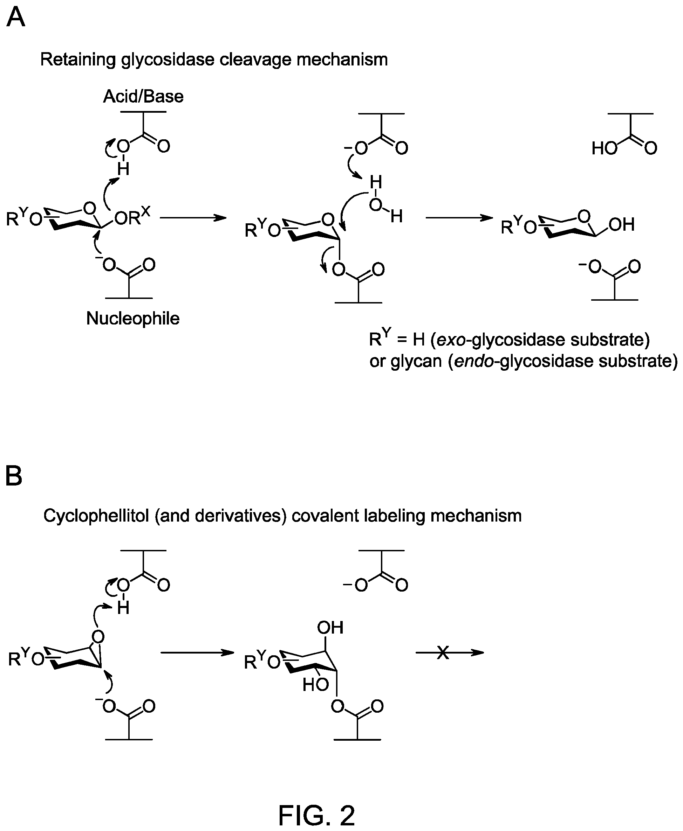

We have noted that derivatives of the naturally occurring β-glucosidase inhibitor cyclophellitol yield active mechanism-based retaining glycosidase inhibitors and probes. Following initial engagement by their target glycosidase, enzymatic attack upon the electrophilic warhead of cyclophellitol-derived molecules leads to alkylation of the catalytically essential enzyme nucleophile residue, producing covalent and irreversible inhibition (Fig. 2B).

The compounds of the invention also may achieve good metabolic stability, due to presence of the cyclophellitol-derived epoxide warhead. Indeed, several pharmaceutical and natural product inhibitors (both reversible and irreversible) contain epoxide motifs e.g. fosfomycin, scopolamine and cyclophellitol itself, indicating this functionality can be stable and well tolerated *in vivo.*

The invention provides in a first aspect a compound of formula **(I),** or a pharmaceutically acceptable salt, solvate, or prodrug thereof: 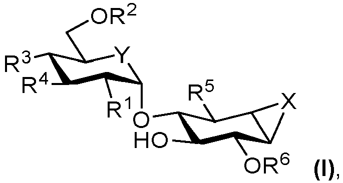 wherein
X is selected from -O-, -N(H)-, -N(R^{a})-, -N(C(O)R^{b})-, -N-L₁-fluorophore-, or -N-L₁-biotin-;
Y is selected from -O-, -(CH₂)-, or -S-;
R¹ is selected from -H, -OH, -NHC(O)CH₃, -NHSO₃⁻;
R² is selected from -H or -SO₃⁻;
R³ is selected from -H, -OH, -O-C₁₋₆ alkyl, -L₂-fluorophore, or -L₂-biotin;
R⁴ is selected from -H, -OH, -O-C₁₋₆ alkyl or -SO₃⁻;
R⁵ is selected from: -C(O)O⁻, -C(O)OC₁₋₆ alkyl, -C(O)NH(C₁₋₆ alkyl), -OPO₃²⁻, -PO₃²⁻;
R⁶ is selected from -H or -SO₃⁻;
R^{a} is selected from C₁₋₆ alkyl, or C₁₋₆ alkyl interrupted by one or more ether linkages;
R^{b} is selected from C₁₋₆ alkyl, or C₁₋₆ alkyl interrupted by one or more ether linkages;
L₁ is a linker; and
L₂ is a linker,
wherein when X is -N-L₁-fluorophore-, or -N-L₁-biotin-, R³ is selected from -H, -OH, or -O-C₁₋₆ alkyl; and
wherein when R³ is -L₂-fluorophore, or -L₂-biotin, X is selected from -O-, -N(H)-, -N(R^{a})-, or-N(C(O)R^{b})-.

A second aspect of the invention provides a compound of formula (XXIVa) or (XXIVb), or a pharmaceutically acceptable salt, solvate, or prodrug thereof: 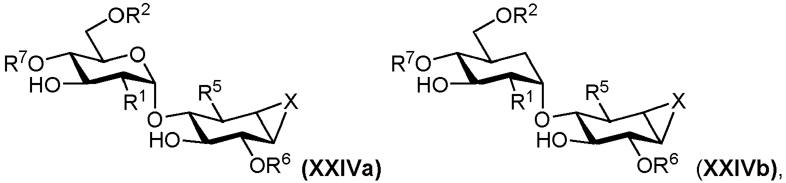 wherein
R¹ is selected from -H, -OH, -NHC(O)CH₃, -NHSO₃⁻;
R² is selected from -H or -SO₃⁻;
R⁵ is selected from: -C(O)O⁻, -C(O)OC₁₋₆ alkyl, -C(O)NH(C₁₋₆ alkyl), -OPO₃²⁻, -PO₃²⁻;
R⁶ is selected from -H or -SO₃⁻;
R⁷ is selected from -H, -C₁₋₆ alkyl, -L₃-fluorophore, or -L₃-biotin;
X is selected from -O-, -N(H)-, -N(R^{a})-, -N(C(O)R^{b})-, -N-L₁-fluorophore-, or -N-L₁-biotin-;
R^{a} is selected from C₁₋₆ alkyl, or C₁₋₆ alkyl interrupted by one or more ether linkages; and
R^{b} is selected from C₁₋₆ alkyl, or C₁₋₆ alkyl interrupted by one or more ether linkages;
wherein at least one of R⁷ and X comprises a fluorophore or a biotin.

An embodiment of the second aspect of the invention provides a compound of formula **(XXIVa),** or a pharmaceutically acceptable salt, solvate, or prodrug thereof: 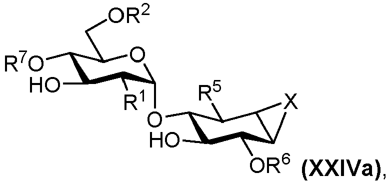 wherein
R¹ is selected from -H, -OH, -NHC(O)CH₃, -NHSO₃⁻;
R² is selected from -H or -SO₃⁻;
R⁵ is selected from: -C(O)O⁻, -C(O)OC₁₋₆ alkyl, -C(O)NH(C₁₋₆ alkyl), -OPO₃²⁻, -PO₃²⁻;
R⁶ is selected from -H or -SO₃⁻;
R⁷ is selected from -H, -C₁₋₆ alkyl, -L₃-fluorophore, or -L₃-biotin;
X is selected from -O-, -N(H)-, -N(R^{a})-, -N(C(O)R^{b})-, -N-L₁-fluorophore-, or -N-L₁-biotin-;
R^{a} is selected from C₁₋₆ alkyl, or C₁₋₆ alkyl interrupted by one or more ether linkages; and
R^{b} is selected from C₁₋₆ alkyl, or C₁₋₆ alkyl interrupted by one or more ether linkages;
wherein at least one of R⁷ and X comprises a fluorophore or a biotin.

A third aspect of the invention provides a pharmaceutical composition comprising a compound of the first aspect or second aspect. The pharmaceutical composition may further comprise a pharmaceutically acceptable excipient.

A fourth aspect of the invention provides a compound of the first or second aspect, or a composition of the third aspect, for use as a medicament.

A fifth aspect of the invention provides a compound of the first or second aspect, or a composition of the third aspect, for use in the treatment of a condition which is modulated by heparanase (HPSE).

A sixth aspect of the invention provides a method of inhibiting heparanase (HPSE), comprising administration to a cell of an effective amount of a compound of the first or second aspect, or a composition of the third aspect. The administration is *in vitro.*

A seventh aspect provides a use of a compound of the first or second aspect, or a composition of the third aspect, for the inhibition of heparanase (HPSE).

An eighth aspect provides a use of a compound of the first or second aspect, or a composition of the third aspect, for the enzymatic labelling of heparanase (HPSE), wherein said compound comprises a fluorophore or a biotin.

A ninth aspect provides a method of treating a condition selected from: cancer, inflammation, inflammatory lung injury, rheumatoid arthritis, chronic colitis, neuroinflammation, sepsis-associated lung injury, inflammatory bowel disease, diabetes, diabetic nephropathy, bone osteolysis, thrombosis, artherosclerosis, inflammatory kidney disease, acute kidney injury, and viral infection (e.g. viral infection caused by herpes simplex virus, dengue virus, human papillomavirus, respiratory syncytial virus, adenovirus, hepatitis C virus, porcine respiratory virus, reproductive syncytial virus, or retroviruses); the method comprising administering a pharmaceutically effective amount of a compound of the first or second aspect, or a composition of the third aspect, to a patient in need thereof.

A tenth aspect provides a method of labelling heparanase (HPSE) *in vitro* or *in vivo,* the method comprising administering to a cell or a patient an effective amount of a compound of the first or second aspect, or a composition of the third aspect, wherein said compound comprises a fluorophore or a biotin.

### BRIEF DESCRIPTION OF THE DRAWINGS

Embodiments of the invention are further described hereinafter with reference to the accompanying drawings, in which:

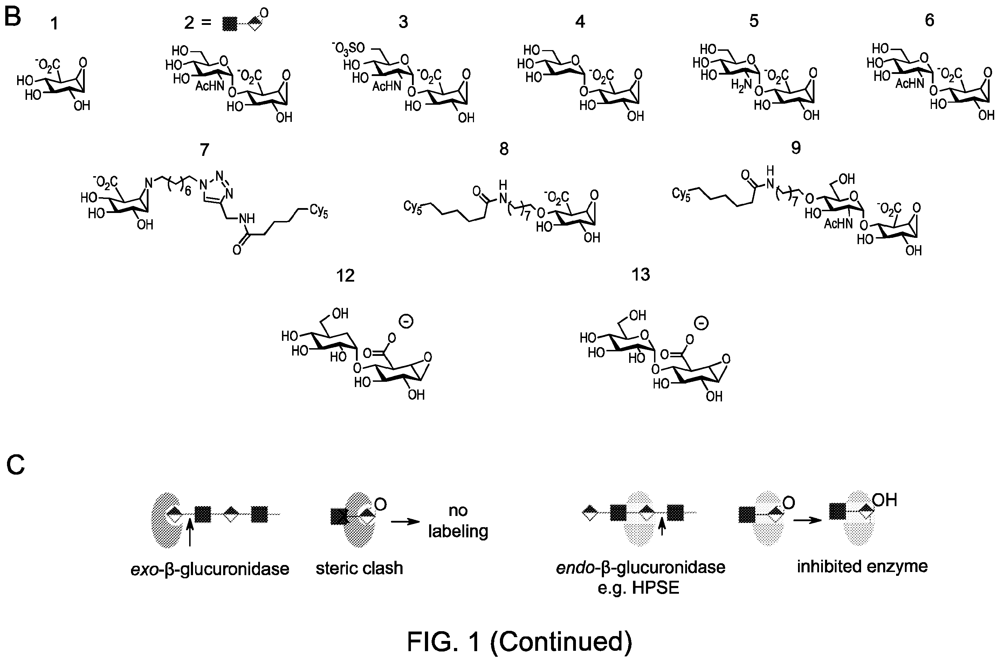

Figure 1 shows the design and development of HS-configured cyclophellitol pseudo-disaccharides. A) Biological effects of HPSE overexpression in the extracellular space. Excessive degradation of HSPG networks in basement membranes facilitates cell migration to and from the vasculature. Release of HSPG sequestered growth factors also stimulates cell proliferation. Right - preferred HPSE target trisaccharide sequence, comprising a GlcA residue flanked by two sulfated glucosamine residues. B) Compounds 2-4, 9, 12 and 13 of the invention, and comparative compounds 1 and 5-8. C) Principle of pseudo-disaccharide HPSE selectivity via steric occlusion of exo-β-glucuronidase binding.
Figure 2 shows A). Double displacement mechanism used by typical retaining glycoside hydrolases during substrate cleavage. B). Mechanism-based covalent labelling of retaining glycoside hydrolases by cyclophellitol and derivatives.

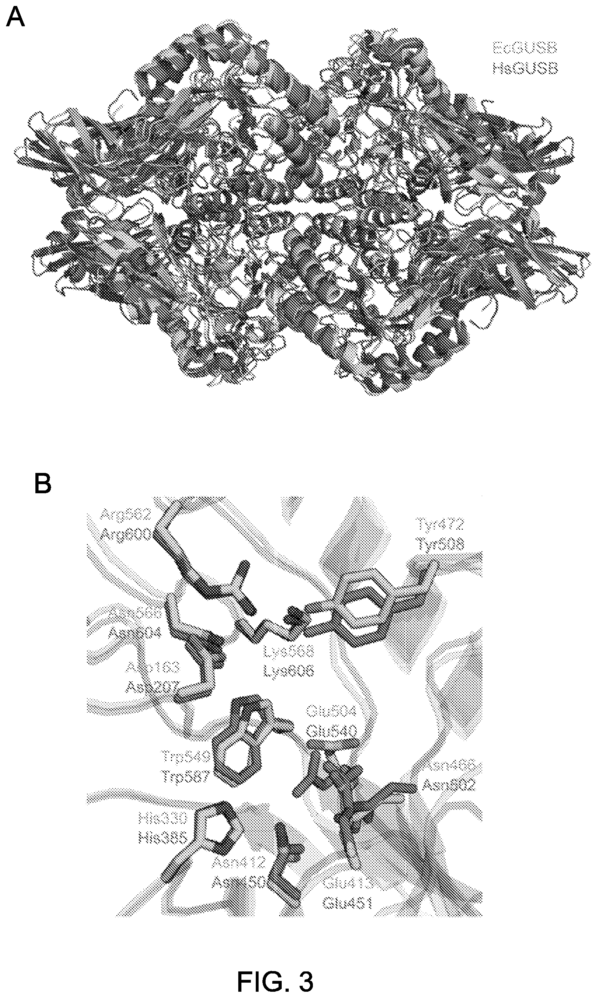

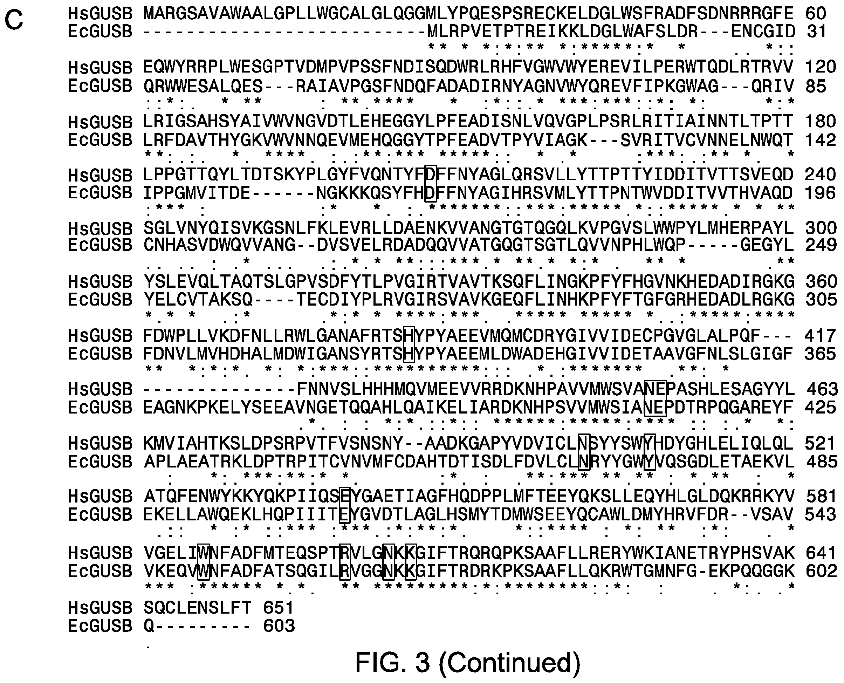

Figure 3 shows the Homology between CAZy GH2 enzymes HsGUSB and EcGUSB. A) Superposed EcGUSB and HsGUSB tertiary/quaternary structures, showing the close similarity between these two enzymes. B) Overlay of active sites of EcGUSB and HsGUSB, showing full conservation of active site residues. C) Sequence homlogy between HsGUSB and EcGUSB. Active site residues are highlighted.

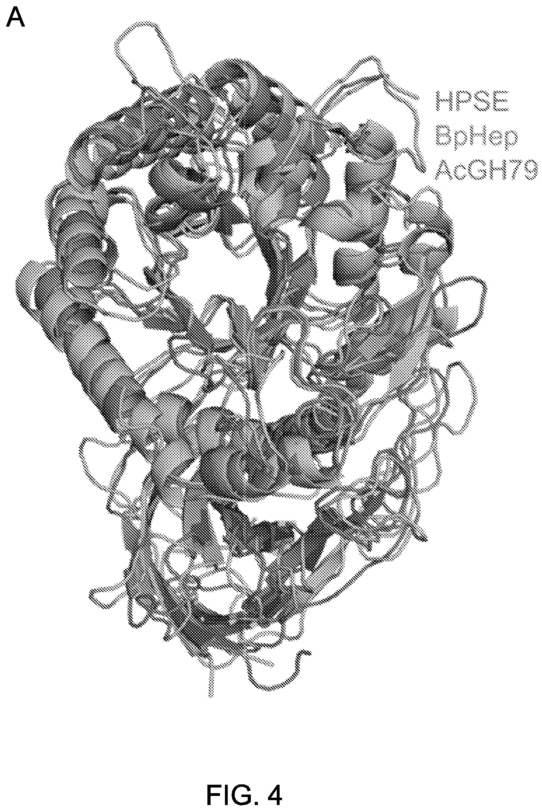

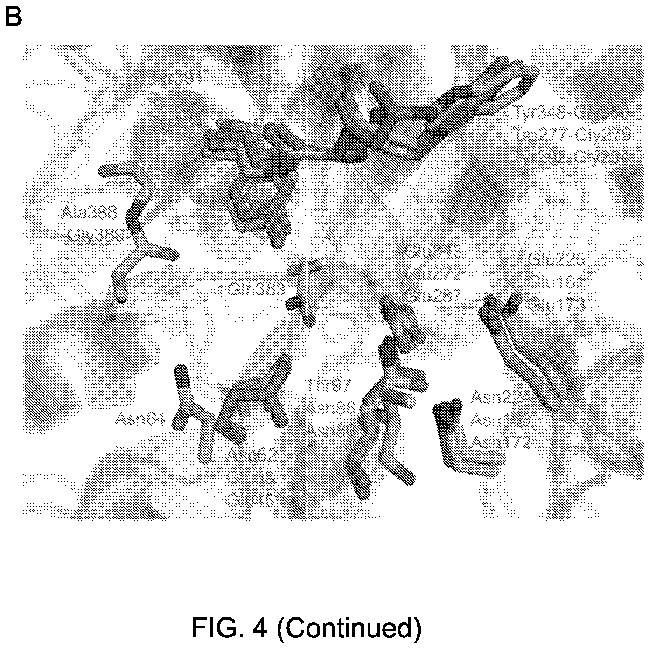

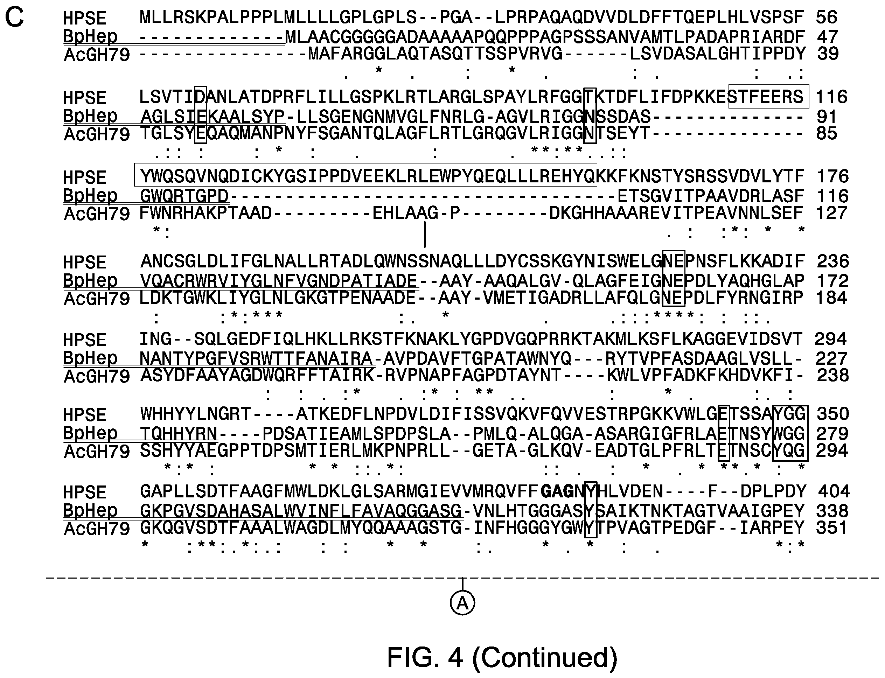

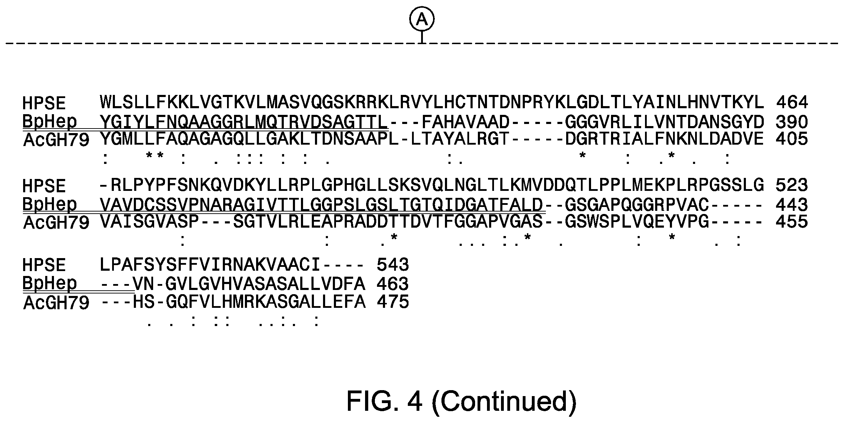

Figure 4 shows the Homology between CAZy GH79 enzymes HPSE, BpHep and AcGH79. A) Superposed enzyme tertiary structures. B) Overlay of active site residues between HPSE, BpHep and AcGH79. Some residues involved in substrate interaction are unique to HPSE and have no clear structural homology in the other enzymes. C) Sequence homlogy between HPSE, BpHep and AcGH79. Conserved active site residues are highlighted in yellow. HPSE unique active site residues are in bold (N64, Q383, A388, G389). The 6-kDa linker peptide of HPSE (S110-Q157) is annotated by a box.

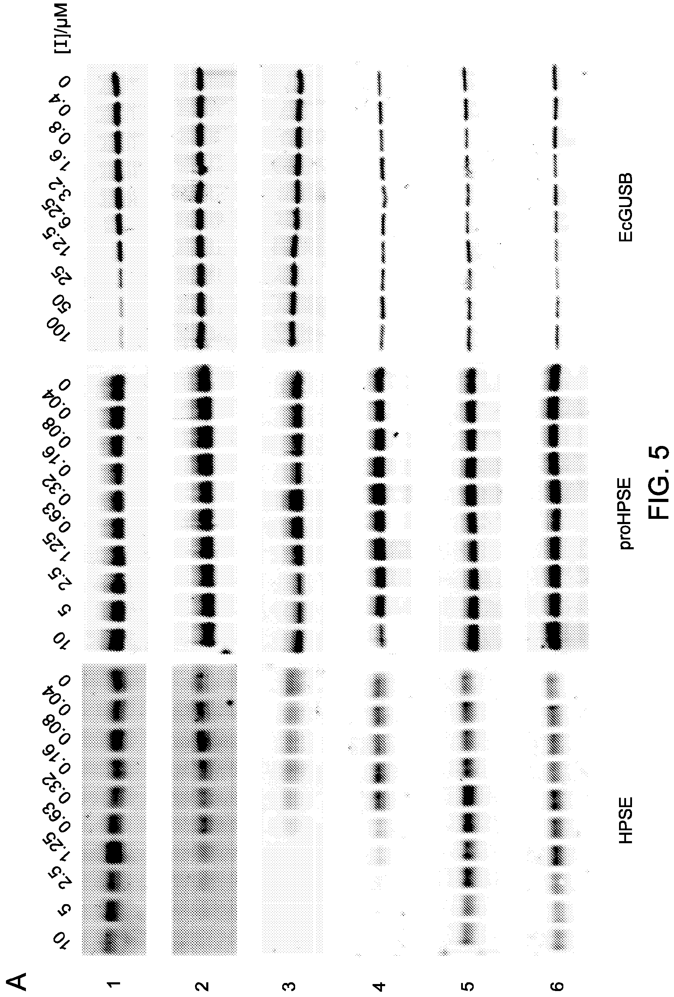

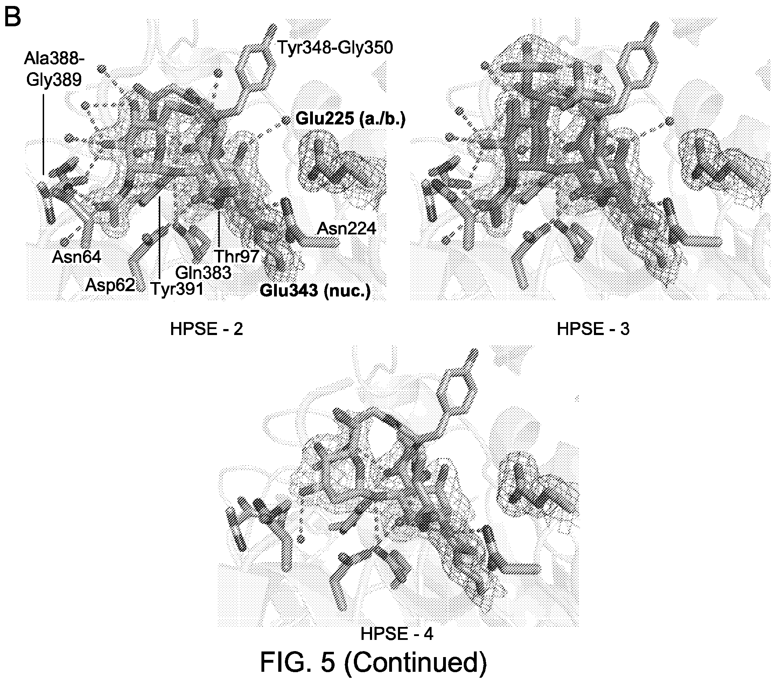

Figure 5 shows In vitro HPSE inhibition by HS configured cyclophellitol-inspired inhibitors. A) cABPP gels for HPSE, proHPSE and EcGUSB inhibition by 1-6. Pseudo-disaccharides **2-4** inhibit HPSE labeling with nanomolar potency. Only **1** inhibits EcGUSB. No inhibitors were effective against proHPSE. B) Active site views from crystal structures of HPSE in complex with **2-4,** showing covalent labeling of the HPSE catalytic nucleophile. Electron density for sidechains is REFMAC σ_{A}-weighted 2mFo-DFc, contoured to 1σ (0.23-0.26 e⁻.Å⁻³). Electron density for ligands is REFMAC σ_{A}-weighted mFo-DFc, contoured to 3σ (0.25-0.27 e⁻.Å⁻³).

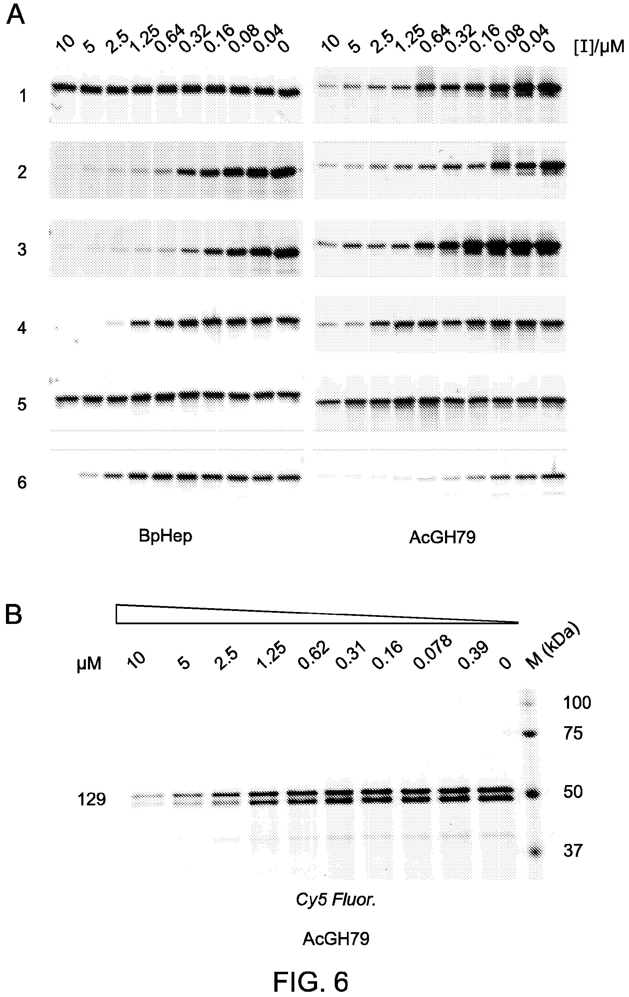

Figure 6 shows cABPP gels for BpHep and AcGH79 inhibition by compounds of the invention. (A) cABPP gels for BpHep and AcGH79 inhibition by **1-6.** (B) cABPP gels for AcGH79 inhibition by **12.**

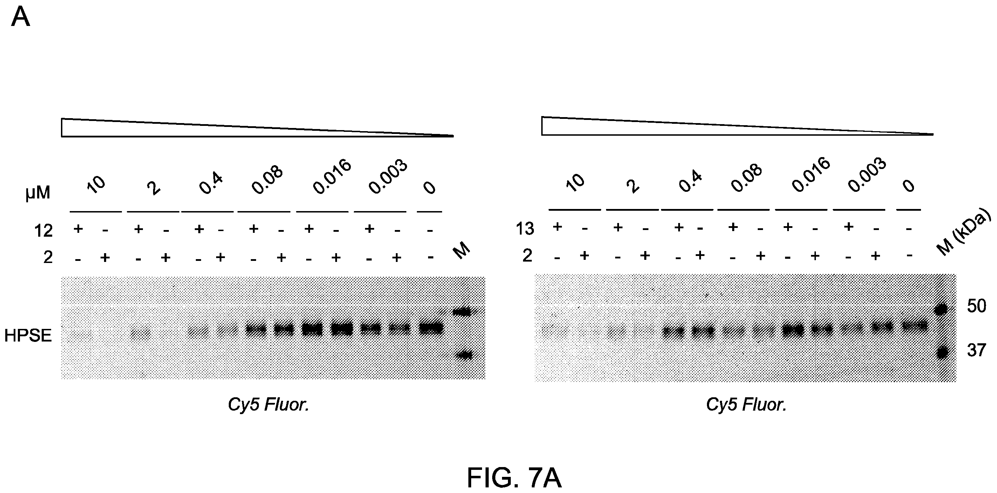

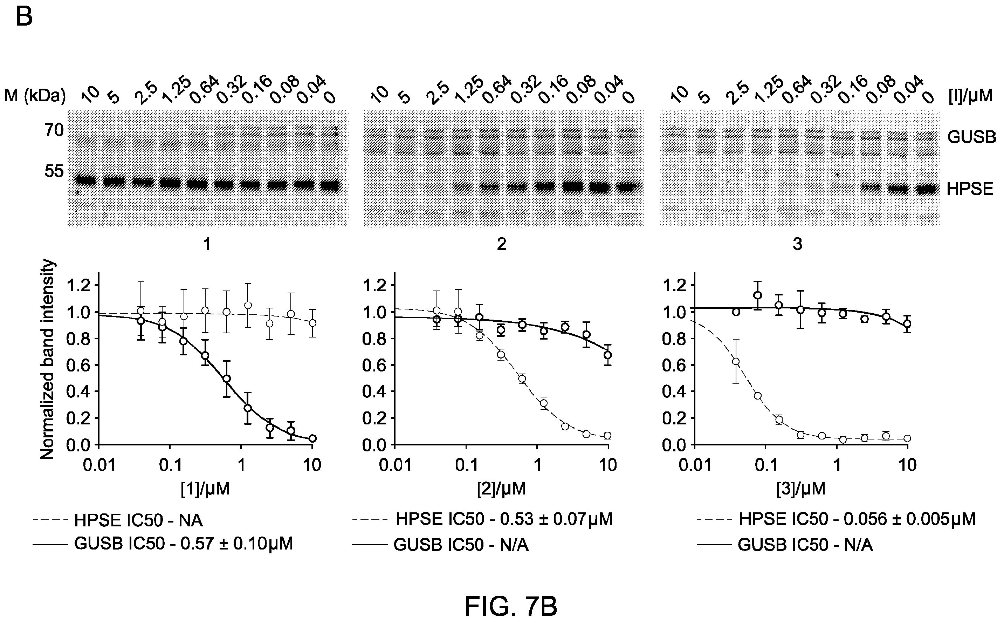

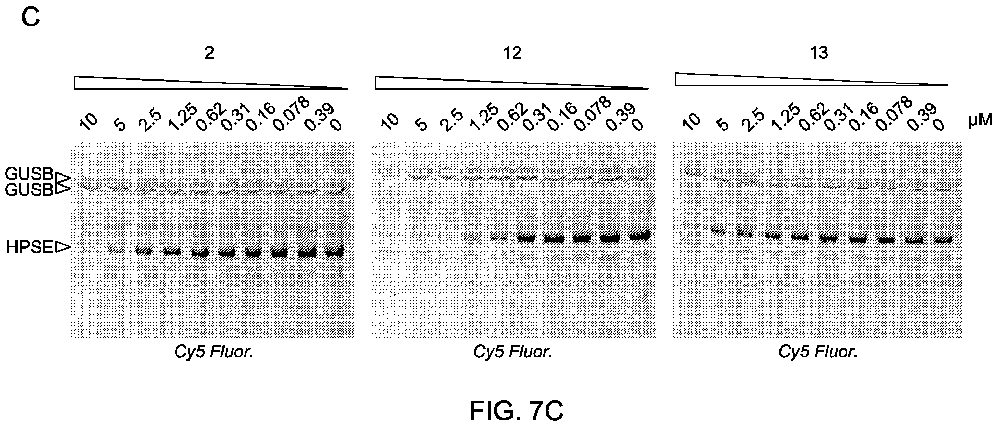

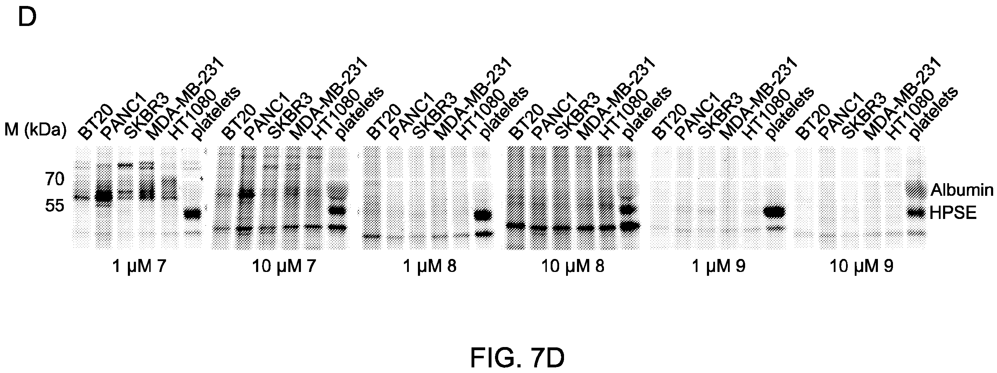

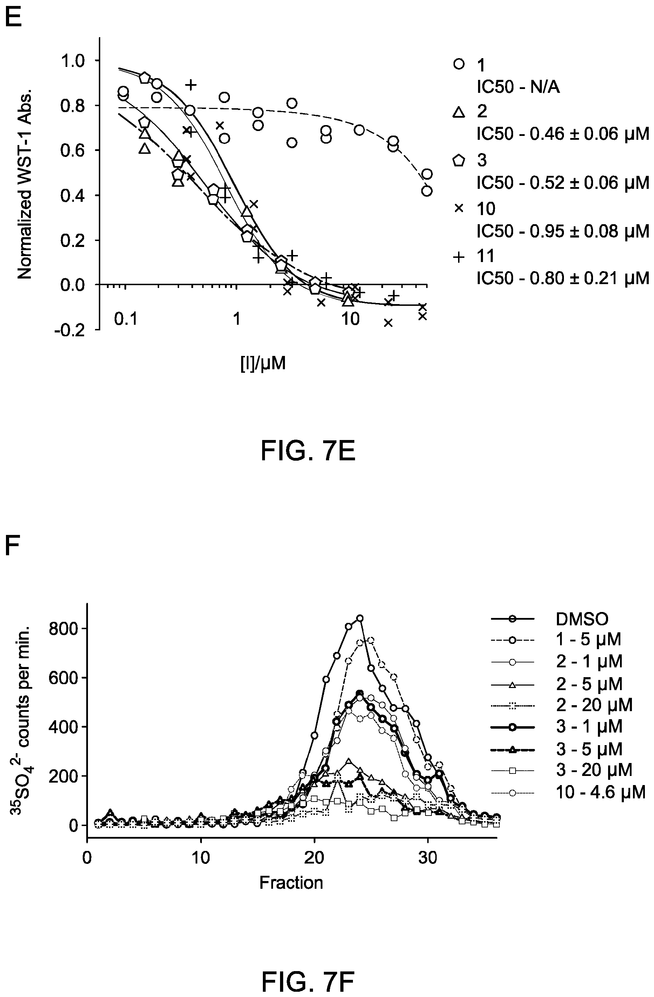

Figure 7 shows HPSE inhibition in physiologically representative scenarios. A) Assessment of inhibitory potency of **2, 12** and **13** by Cy5-tagged β-D-glucuronidase ABP-based in-gel competitive profiling against recombinant HPSE. B) Cyclophellitol pseudo-disaccharides **2** and **3** selectively inhibit endogenous HPSE in platelet lysates, which contain both GUSB and HPSE β-glucuronidase activities. Representative gels, along with graphs showing normalized quantitated band intensities vs inhibitor concentration. Datapoints are mean ± standard deviation (N = 3). C). HPSE selectivity by **2, 12** and **13** in platelet lysates as determined by competitive ABPP in blood platelet lysate. Fluorescent labelling of HPSE by β-D-glucuronidase ABP can be abrogated by pre-incubation with the tested inhibitors without altering GUSB signal, thus demonstrating their innate selectivity for human HPSE in the presence of exo-acting β-D-glucuronidase. In particular, **2** and **13** scored comparable apparent HPSE IC₅₀ values. In contrast, **12** exhibited higher HPSE inhibitory potency. D) Cy5 fluorescent ABP **7, 8** and **9** labelling of cell lysates. Pseudo-disaccharide **9** shows superior HPSE specificity compared to **7** and **8.** E) **2** and **3** inhibit HPSE mediated cleavage of the synthetic HS pentasaccharide fondaparinux, with greater potency than reference inhibitors **10** and **11.** Individual assay datapoints (N=2) plotted. F) **2** and **3** inhibit the HPSE mediated liberation of H³⁵S fragments from basement membrane HSPGs, with greater potency than reference inhibitor **10.**

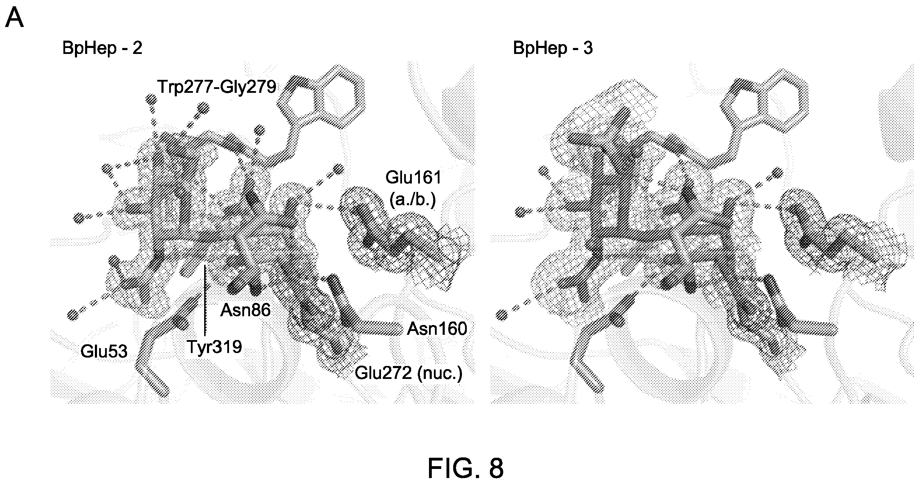

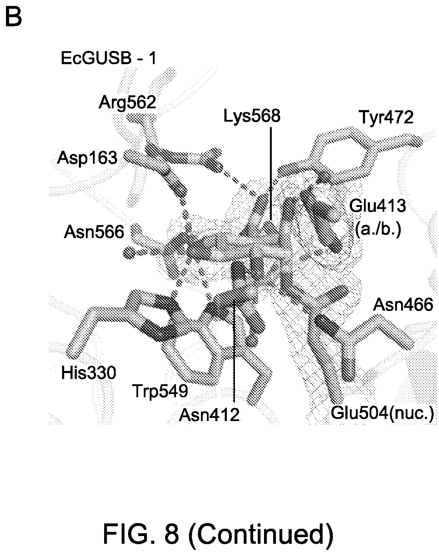

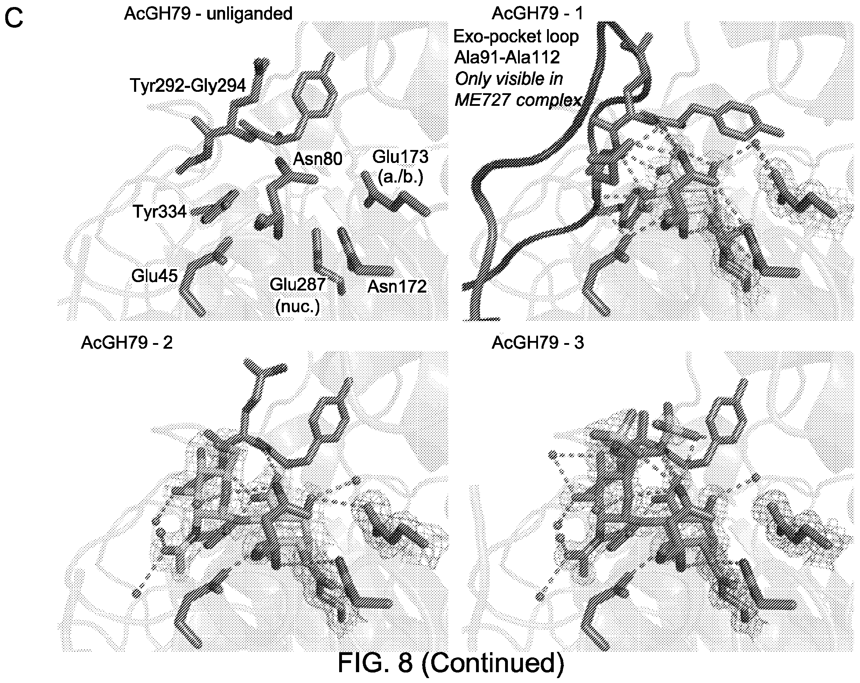

Figure 8 shows Active-site views of BpHep (A), EcGUSB (B), and AcGH79 (C) in complex with cyclophellitol-derived inhibitors. Electron density for sidechains is REFMAC σ_{A}-weighted 2mFo-DFc, contoured to 1s (0.21-0.51 e-.Å-3). Electron density for ligands is REFMAC σ_{A}-weighted mFo-DFc, contoured to 3σ (0.2-0.54 e⁻.Å⁻³). An otherwise unstructured loop in the AcGH79 active site is only visible in the complex with pseudo-monosaccharide **1.**

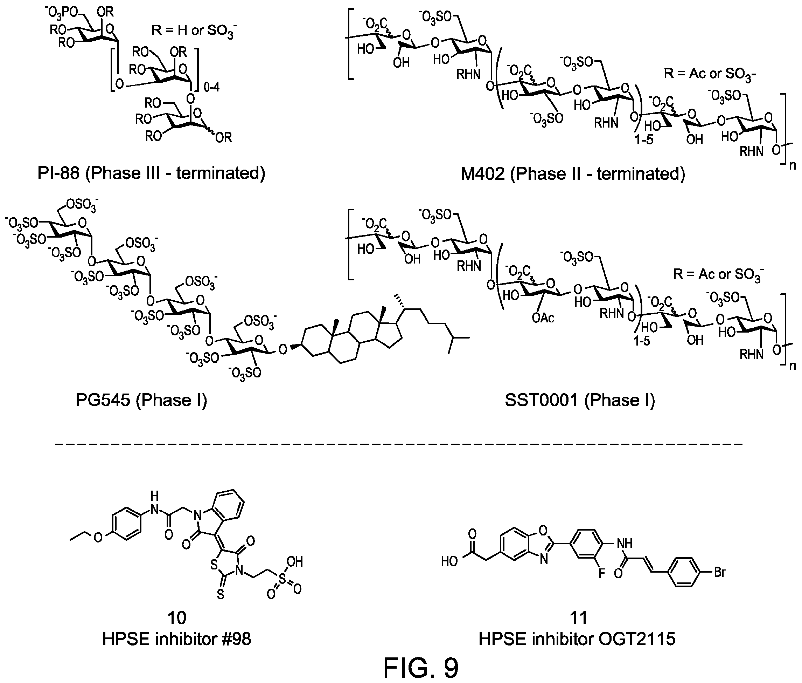

Figure 9 shows HPSE inhibitors that have been tested in clinical trials to date and additional HPSE inhibitors **10** and **11** used in this study, which are non-cyclophellitol related molecules used as comparators.

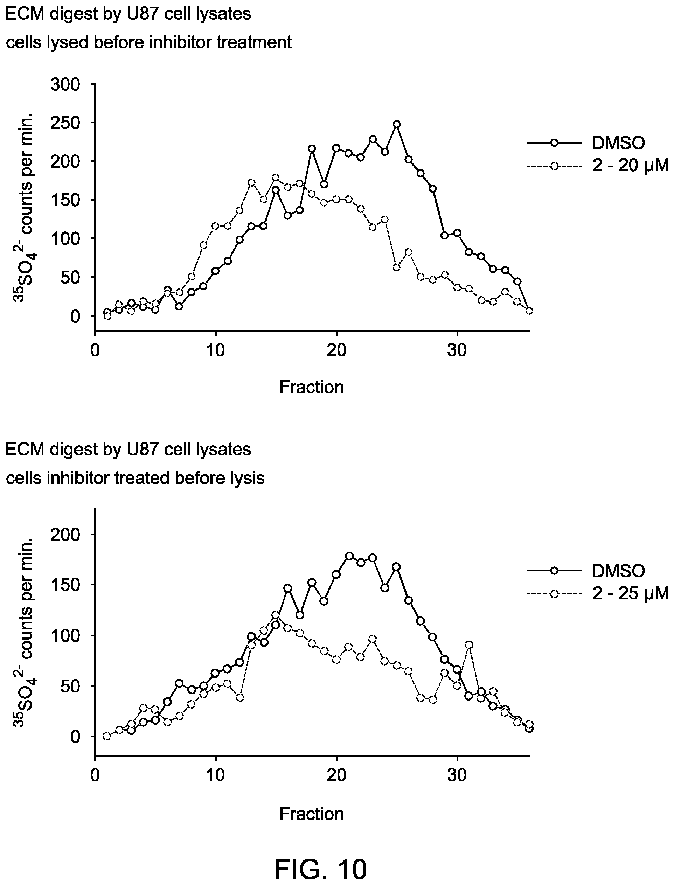

Figure 10 shows radiolabelled basement membrane H³⁵SPG digests by HPSE expressing U87 cell lysates, which show comparable profiles of H³⁵S solubilization whether inhibitor **2** is applied before or after cell lysis. These data indicate that **2** can penetrate cells to inhibit the intracellular HPSE pool.

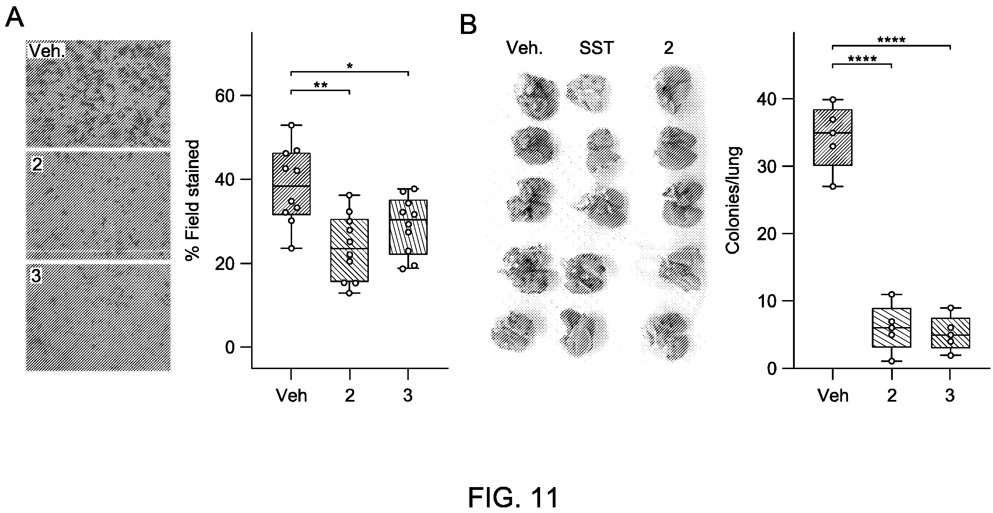

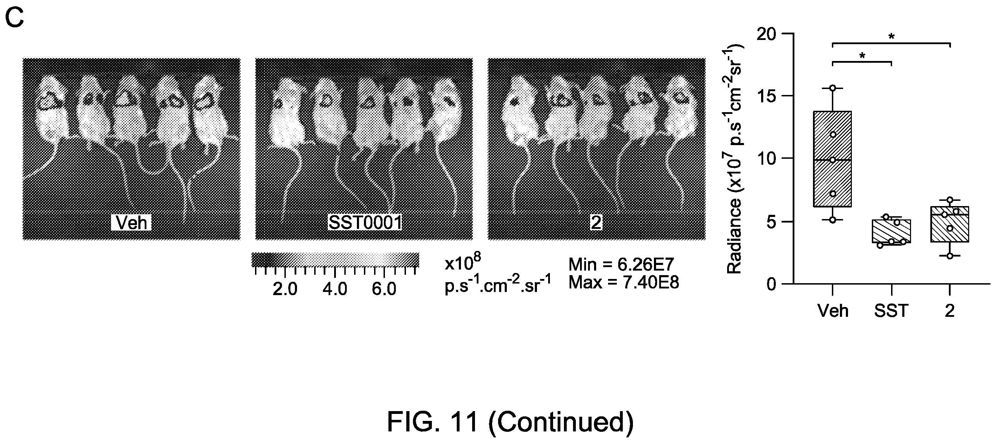

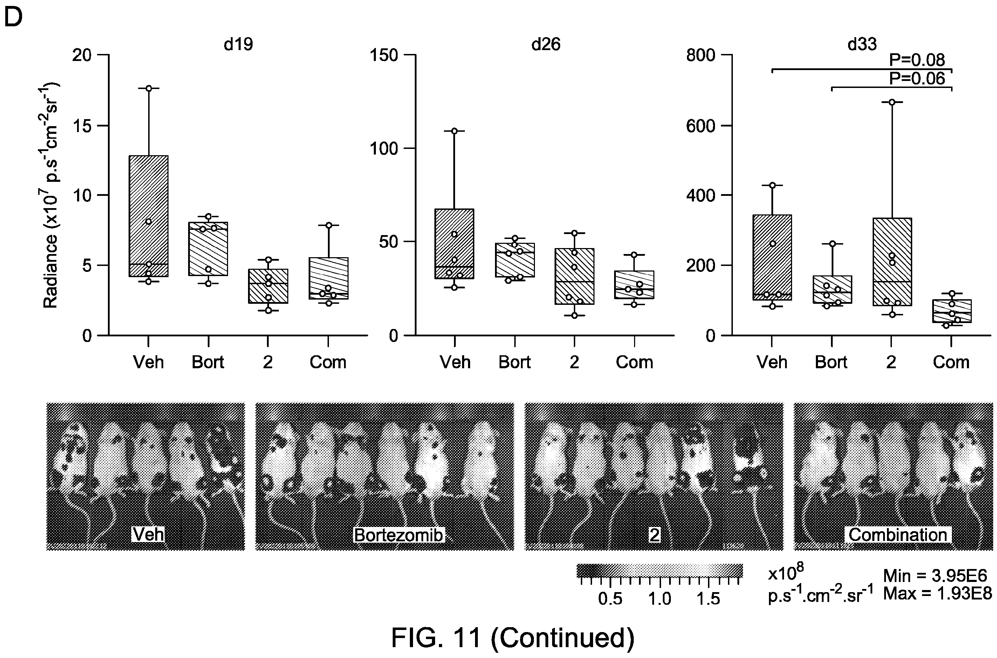

Figure 11 shows the effects of cyclophellitol pseudo-disaccharides on metastasis *in cellulo* and *in vivo.* **A)** Effect of inhibitors **2** and **3** on U87 cell invasion through a Matrigel coated transwell. Representative fields-of-view of migrated cells (left), alongside quantitation of invasion (% field stained) from 10 randomly selected fields-of-view (right). **B)** Effect of inhibitor **2** on the formation of lung metastases by B16 melanoma cells, on par with HPSE inhibitor SST0001. Images of lungs showing metastatic B16 foci (left), alongside quantitation of foci on lungs from 5 replicates (right). **C)** Effect of inhibitor **2** on the formation of lung metastases formed by 4T1 breast cancer cells, with efficacy on par with SST0001. IVIS images of luciferase expressing 4T1 cell bioluminescence in lungs at the 14 day timepoint (left) alongside quantitation of bioluminescent flux from 5 replicates (right). **D)** Effect of inhibitor **2** on the formation of bone metastases formed by CAG myeloma cancer cells, with efficacy on par with Bortezomib, and synergistic efficacy at longer timepoints. IVIS images of luciferase expressing CAG cell bioluminescence (bottom) alongside quantitation of bioluminescent flux from 5 or 6 replicates (top). All box and whisker plots show mean, interquartile range, maxima and minima. Statistical comparisons are two-tailed Student's t-tests: * - P < 0.05; ** - P < 0.01; *** - P < 0.001; **** - P < 0.0001.

### DETAILED DESCRIPTION

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

### DEFINITIONS

The following explanations of terms and methods are provided to better describe the present disclosure and to guide those of ordinary skill in the art in the practice of the present disclosure.

The invention concerns amongst other things the treatment of a disease. The term "treatment", and the therapies encompassed by this invention, include the following and combinations thereof: (1) hindering, e.g. delaying initiation and/or progression of, an event, state, disorder or condition, for example arresting, reducing or delaying the development of the event, state, disorder or condition, or a relapse thereof in case of maintenance treatment or secondary prophylaxis, or of at least one clinical or subclinical symptom thereof; (2) preventing or delaying the appearance of clinical symptoms of an event, state, disorder or condition developing in an animal (e.g. human) that may be afflicted with or predisposed to the state, disorder or condition but does not yet experience or display clinical or subclinical symptoms of the state, disorder or condition; and/or (3) relieving and/or curing an event, state, disorder or condition (e.g., causing regression of the event, state, disorder or condition or at least one of its clinical or subclinical symptoms, curing a patient or putting a patient into remission). The benefit to a patient to be treated may be either statistically significant or at least perceptible to the patient or to the physician. It will be understood that a medicament will not necessarily produce a clinical effect in each patient to whom it is administered; thus, in any individual patient or even in a particular patient population, a treatment may fail or be successful only in part, and the meanings of the terms "treatment" and "prophylaxis" and of cognate terms are to be understood accordingly. The compositions and methods described herein are of use for therapy and/or prophylaxis of the mentioned conditions.

The term "prophylaxis" includes reference to treatment therapies for the purpose of preserving health or inhibiting or delaying the initiation and/or progression of an event, state, disorder or condition, for example for the purpose of reducing the chance of an event, state, disorder or condition occurring. The outcome of the prophylaxis may be, for example, preservation of health or delaying the initiation and/or progression of an event, state, disorder or condition. It will be recalled that, in any individual patient or even in a particular patient population, a treatment may fail, and this paragraph is to be understood accordingly.

The term "alkyl" as used herein includes reference to a straight or branched chain alkyl moiety having up to 20 (e.g. 1, 2, 3, 4, 5, 6, 7, 8, 9, 10, 11, 12, 13, 14, 15, 16, 17, 18, 19 or 20) carbon atoms. The term includes reference to, for example, methyl, ethyl, propyl (n-propyl or isopropyl), butyl (n-butyl, sec-butyl or tert-butyl), pentyl, hexyl and the like. In particular, alkyl may be a "C1-C8 alkyl", i.e. an alkyl having 1, 2, 3, 4, 5, 6, 7 or 8 carbon atoms; "C1-C6 alkyl", i.e. an alkyl having 1, 2, 3, 4, 5 or 6 carbon atoms; "C1-C4 alkyl", i.e. an alkyl having 1, 2, 3 or 4 carbon atoms; or a "C1-C3 alkyl", i.e. an alkyl having 1, 2 or 3 carbon atoms. The term "lower alkyl" includes reference to alkyl groups having 1, 2, 3 or 4 carbon atoms.

The term "alkenyl" as used herein includes reference to a straight or branched chain alkenyl moiety having up to 20 (e.g. 2, 3, 4, 5, 6, 7, 8, 9, 10, 11, 12, 13, 14, 15, 16, 17, 18, 19 or 20) carbon atoms. The term includes reference to, for example, ethenyl, propenyl, butenyl, pentenyl, hexenyl and the like. In particular, alkenyl may be a "C3-C8 alkenyl", i.e. an alkenyl having 3, 4, 5, 6, 7 or 8 carbon atoms; "C3-C6 alkenyl", i.e. an alkenyl having 3, 4, 5 or 6 carbon atoms; "C3-C4 alkenyl", i.e. an alkenyl having 3 or 4 carbon atoms; The term "lower alkenyl" includes reference to alkenyl groups having 2, 3 or 4 carbon atoms. The alkenyl may be monounsaturated (i.e. comprise a single carbon carbon double bond) or polyunsaturated (i.e. comprise a two or more carbon carbon double bonds, e.g. 2, 3 or 4 carbon carbon double bonds). For example, an alkenyl may be an alkadienyl, alkatrienyl, etc..

The term "cycloalkyl" as used herein includes reference to an alicyclic moiety having 3, 4, 5, 6, 7, 8, or 9 carbon atoms. The group may be a bridged or polycyclic ring system. More often cycloalkyl groups are monocyclic. This term includes reference to groups such as cyclopropyl, cyclobutyl, cyclopentyl, cyclohexyl and the like.

The term "heteroalkyl," by itself or in combination with another term, means, unless otherwise stated, a stable straight or branched chain, or cyclic hydrocarbon radical, or combinations thereof, consisting of at least one carbon atoms and at least one heteroatom selected from the group consisting of O, N, P, Si and S, and wherein the nitrogen, sulfur and phosphorus atoms may optionally be oxidized and the nitrogen heteroatom may optionally be quaternized. The heteroatom(s) O, N, P, S and Si may be placed at any interior position of the heteroalkyl group or at the position at which the alkyl group is attached to the remainder of the molecule. Examples include, but are not limited to, -CH2-CH2-O-CH3, -CH2-CH2-NH-CH3,-CH2-CH2-N(CH3)-CH3, -CH2-S-CH2-CH3, -CH2-CH2,-S(O)-CH3, -CH2-CH2-S(O)2-CH3,-CH=CH-O-CH3, -Si(CH3)3, -CH2-CH=N-OCH3, -CH=CH-N(CH3)-CH3, O-CH3, -O-CH2-CH3, and -CN. Up to two heteroatoms may be consecutive, such as, for example, -CH2-NH-OCH3 and -CH2-O-Si(CH3)3. Similarly, the term "heteroalkylene" by itself or as part of another substituent means a divalent radical derived from heteroalkyl, as exemplified, but not limited by, -CH2-CH2-S-CH2-CH2- and -CH2-S-CH2-CH2-NH-CH2-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)2R'- represents both -C(O)2R'- and -R'C(O)2-. As described above, heteroalkyl groups, as used herein, include those groups that are attached to the remainder of the molecule through a heteroatom, such as -C(O)R', -C(O)NR', -NR'R", -OR', -SR', and/or -SO2R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The term "heterocycloalkyl" as used herein includes reference to a saturated heterocyclic moiety having 3, 4, 5, 6 or 7 ring carbon atoms and 1, 2, 3, 4 or 5 ring heteroatoms selected from nitrogen, oxygen, phosphorus and sulphur. For example, a heterocycloalkyl may comprise 3, 4, or 5 ring carbon atoms and 1 or 2 ring heteroatoms selected from nitrogen and oxygen. The group may be a polycyclic ring system but more often is monocyclic. This term includes reference to groups such as azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolizidinyl and the like.

The terms "halo" or "halogen" as used herein includes reference to F, Cl, Br or I, for example F, Cl or Br. In a particular class of embodiments, halogen is F or Cl, of which F is more common.

The terms "halo" or "halogen," by themselves or as part of another substituent, mean, unless otherwise stated, a fluorine, chlorine, bromine, or iodine atom; for example a halo may be a fluorine, chlorine or bromine. Additionally, terms such as "haloalkyl," are meant to include monohaloalkyl and polyhaloalkyl. For example, the term "haloalkyl" refers to an alkyl group where one or more hydrogen atoms are substituted by a corresponding number of halogens. For example, the term "halo(C1-C4)alkyl" is mean to include, but not be limited to, trifluoromethyl, 2,2,2-trifluoroethyl, 4-chlorobutyl, 3-bromopropyl, and the like.

The term "alkoxy" as used herein include reference to -O-alkyl, wherein alkyl is straight or branched chain and comprises 1, 2, 3, 4, 5, 6, 7 or 8 carbon atoms. In one class of embodiments, alkoxy has 1, 2, 3 or 4 carbon atoms, e.g. 1, 2 or 3 carbon atoms. This term includes reference to, for example, methoxy, ethoxy, propoxy, isopropoxy, butoxy, tert-butoxy, pentoxy, hexoxy and the like. The term "lower alkoxy" includes reference to alkoxy groups having 1, 2, 3 or 4 carbon atoms.

The term "haloalkoxy" as used herein refers to an alkoxy group where one or more hydrogen atoms are substituted by a corresponding number of halogens.

Each of the above terms (e.g., "alkyl," "cycloalkyl," "heteroalkyl," and "alkoxyl"), unless otherwise noted, are meant to include both substituted and unsubstituted forms of the indicated radical. Where a substituent is R-substituted (e.g. an Rx-substituted alkyl, where "x" is an integer), the substituent may be substituted with one or more R groups as allowed by chemical valency rules where each R group is optionally different (e.g. an Rx-substituted alkyl may include multiple Rx groups wherein each Rx group is optionally different). Certain examples of substituents for each type of radical are provided below.

The term "substituted" as used herein in reference to a moiety means that one or more, especially 1 to 5, more especially 1, 2 or 3, (e.g. 1 or 2) of the hydrogen atoms in said moiety are replaced independently of each other by the corresponding number of the described substituents. Unless otherwise specified, exemplary substituents include -OH, -CN, -NH₂,-NH(C1-C6 alkyl), -N(C1-C4 alkyl)2, =O, -halo, -C1-C6 alkyl, -C2-C6 alkenyl, -C1-C6 haloalkyl,-C1-C6 haloalkoxy and-C2-C6 haloalkenyl, -C1-C6 alkylcarboxylic acid (e.g. -CH₃COOH or-COOH). Where the substituent is a -C1-C6 alkyl or -C1-C6 haloalkyl, the C1-C6 chain is optionally interrupted by an ether linkage (-O-) or an ester linkage (-C(O)O-). Exemplary substituents for a substituted alkyl may include -OH, -CN, -NH₂, =O, -halo, -CO2H, -C1-C6 haloalkyl, -C1-C6 haloalkoxy and-C2-C6haloalkenyl, -C1-C6 alkylcarboxylic acid (e.g.-CH3COOH or -COOH). For example, exemplary substituents for an alkyl may include -OH,-CN, -NH₂, =O, -halo.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

Where steric issues determine placement of substituents on a group, the isomer having the lowest conformational energy may be preferred.

Where a compound, moiety, process or product is described as "optionally" having a feature, the disclosure includes such a compound, moiety, process or product having that feature and also such a compound, moiety, process or product not having that feature. Thus, when a moiety is described as "optionally substituted", the disclosure comprises the unsubstituted moiety and the substituted moiety.

Where two or more moieties are described as being "independently" or "each independently" selected from a list of atoms or groups, this means that the moieties may be the same or different. The identity of each moiety is therefore independent of the identities of the one or more other moieties.

The term "pharmaceutically acceptable" as used herein includes reference to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio. This term includes acceptability for both human and veterinary purposes.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like (see, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centres) or double bonds; the racemates, tautomers, geometric isomers and individual isomers are encompassed within the scope of the present invention. The compounds of the present invention do not include those which are known in the art to be too unstable to synthesize and/or isolate.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). In addition, the compounds may be labeled with stable isotopes that have a relatively low natural abundance, such as deuterium (²H), carbon-13 (¹³C), or nitrogen-15 (¹⁵N). All isotopic variations of the compounds of the present invention, whether radioactive or not, are encompassed within the scope of the present invention.

The term "prodrug" as used herein represents compounds which are transformed *in vivo* to the parent compound or other active compound, for example, by hydrolysis in blood. An example of such a prodrug is a pharmaceutically acceptable ester of a carboxylic acid. A thorough discussion is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987; H Bundgaard, ed, Design of Prodrugs, Elsevier, 1985; and Judkins, et al. Synthetic Communications, 26(23), 4351-4367 (1996); and The organic chemistry of drug design and drug action by Richard B Silverman in particular pages 497 to 546.

The term "pharmaceutical composition" as used herein includes reference to a composition comprising at least one active compound (or prodrug) and optionally one or more additional pharmaceutically acceptable ingredients, for example a pharmaceutically acceptable carrier. Unless the context indicates otherwise, all references to a "composition" herein are references to a pharmaceutical formulation.

The term "HPSE" as used herein refers to the enzyme heparanase. The enzyme is an *endo*-β-D-glucuronidase that is known to catalyse heparan sulfate (HS) polysaccharide degradation, which facilitates cancer cell migration to and from the vasculature. Compounds provided herein covalently inhibit HPSE, thereby reducing levels of HS degradation *in vivo* and, in turn, minimising cancer metastasis.

The term "probe" refers to a compound comprising a label.

The term "label" refers to a detectable or therapeutic moiety.

The label may be a detectable moiety that is a chemical moiety or protein that is directly or indirectly detectable (e.g. due to its spectral properties, conformation or activity) when attached to a target or compound and used in the present methods, including reporter molecules and carrier molecules. The label may be directly detectable (e.g. a dye, such as a fluorophore) or indirectly detectable (e.g. hapten or enzyme). Such labels include, but are not limited to, radiolabels that can be measured with radiation-counting devices; pigments, dyes or other chromogens that can be visually observed or measured with a spectrophotometer; spin labels that can be measured with a spin label analyzer; and fluorescent labels (fluorophores), where the output signal is generated by the excitation of a suitable molecular adduct and that can be visualized by excitation with light that is absorbed by the dye or can be measured with standard fluorometers or imaging systems, for example. The label can be a chemiluminescent substance, where the output signal is generated by chemical modification of the signal compound; a metal-containing substance; or an enzyme, where there occurs an enzyme-dependent secondary generation of signal, such as the formation of a coloured product from a colourless substrate. The term label can also refer to a "tag" or hapten that can bind selectively to a conjugated molecule such that the conjugated molecule, when added subsequently along with a substrate, is used to generate a detectable signal. For example, one can use biotin as a tag and then use an avidin or streptavidin conjugate of horseradish peroxidate (HRP) to bind to the tag, and then use a colorimetric substrate (e.g., tetramethylbenzidine (TMB)) or a fluorogenic substrate such as Amplex Red reagent (Molecular Probes, Inc.) to detect the presence of HRP. Numerous labels are known by those of skill in the art and include, but are not limited to, particles, fluorophores, haptens, enzymes and their colorimetric, fluorogenic and chemiluminescent substrates and other labels that are described in The Molecular Probes Handbook: A Guide to Fluorescent Probes and Labeling Technologies (11th edition, 2010, ThermoFisher Scientific, available at thermofisher.com/probes). Examples of labels include fluorophore, hapten, nucleic acid tags.

The label may be a therapeutic moiety, such as a such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thiotepa chlorambucil, melphalan, carmustine (BCNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). The label may comprise a chelator and a chelated radioactive metal ion. The term label can also refer to a "tag" or hapten that can bind selectively to a conjugated molecule such that the conjugated molecule may also comprise the cytotoxin, therapeutic agent or radioactive metal ion. For example, one can use biotin as a tag and then use an avidin or streptavidin conjugate of a cytotoxin, therapeutic agent or radioactive metal ion.

The term "enzymatic labelling" refers to the incorporation of a substate comprising a label to another species via an enzyme catalysed reaction. In the context of the present disclosure, the substrate may be a compound of the disclosure comprising a label and the another species may be HPSE, and the HPSE may catalyse the reaction.

The term "linker" refers to a chemical spacer group employed to attach the HPSE inhibitor compounds of the invention to an additional moiety (e.g. a label) that is not involved in binding to the HPSE active site. Typically, the linker has a chain length that is sufficiently long such that the additional moiety cannot interact with the binding site. The linker may be introduced via click chemistry, e.g. using azide-alkyne cycloaddition. Alternatively, the linker may be introduced by amide coupling reactions. For example, the linker may comprise a C₂₋₂₄-alkyl, optionally interrupted by one, two, or three of -NC(O)-, -NH-, -O-, -C(O)-, -C(OH)-, -C(O)O-, and heterocyclic ring system comprising 1,2,3-triazole.

The term "cyclophellitol" relates to a compound with the IUPAC nomenclature name (1R,2R,3R,4S,5R,6S)-2-(hydroxymethyl)-7-oxabicyclo[4.1.0]heptane-3,4,5-triol.

The term "cyclophellitol-inspired" implies compounds with a cyclohexyl ring and various substituents which may be derived chemically from the cyclophellitol, or synthesized differently, whereby the stereochemistry of the substituents at the carbon atoms of the cyclohexene ring may be varied in line with the required or desired conformation.

The term "HPSE" refers to an endoglycosidase that cleaves heparan sulfate proteoglycans (HSPGs) into heparan sulfate side chains and core proteoglycans. Participates in extracellular matrix (ECM) degradation and remodeling. An example of an HPSE is HPSE_HUMAN (UniProtKB - Q9Y251).

### COMPOUNDS

In one aspect, the invention provides compounds of formula **(I)** as previously described or a pharmaceutically acceptable salt, stereoisomer, solvate or prodrug thereof.

In embodiments, the compound is a compound of formula (**IIa**) or (**IIb**), or a pharmaceutically acceptable salt or solvate thereof: 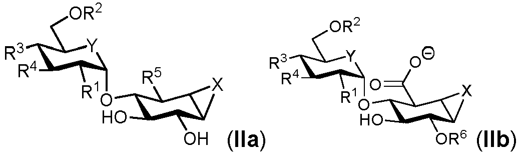 wherein X, Y, R¹, R², R³, R⁴, R⁵ and R⁶ are as defined herein.

In embodiments, the compound is a compound of formula (**III**) or a pharmaceutically acceptable salt or solvate thereof: 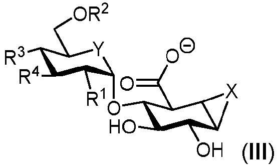 wherein X, Y, R¹, R², R³ and R⁴ are as defined herein.

In embodiments, the compound is a compound of formula (IVa), (IVb) or (IVc), or a pharmaceutically acceptable salt or solvate thereof: 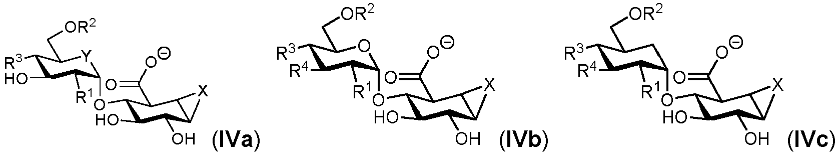 wherein X, Y, R¹, R², R³ and R⁴ are as defined herein.

In embodiments, the compound is a compound of formula (Va) or (Vb), or a pharmaceutically acceptable salt or solvate thereof: 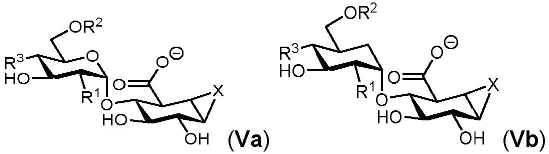 wherein X, R¹, R² and R³ are as defined herein.

In embodiments, the compound is a compound of formula (**VI**), (**VIa**) or (**VIb**)**,** or a pharmaceutically acceptable salt or solvate thereof: 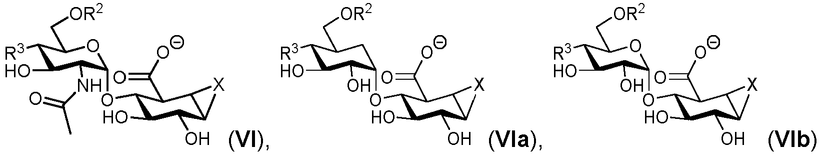 wherein X, R² and R³ are as defined herein.

In embodiments, the compound is a compound of formula (**VIIa**), (**VIIb**) or (**VIIc**) or a pharmaceutically acceptable salt or solvate thereof: 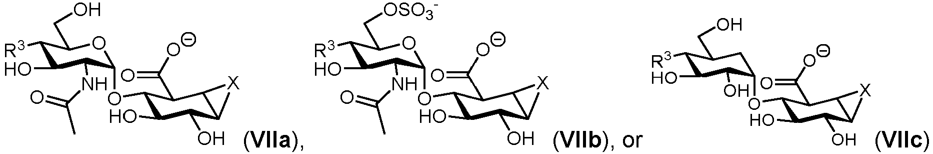 wherein X and R³ are as defined herein.

In embodiments, the compound is a compound of formula (**VIIIa**) or (**VIIIb**), or a pharmaceutically acceptable salt or solvate thereof: 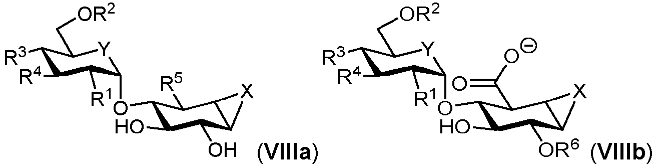 wherein X is selected from -O-, -N(H)-, -N(R^{a})-, and -N(C(O)R^{b})-; R³ is selected from -H, -OH, and -O-C₁₋₆ alkyl; and Y, R¹, R², R⁴, R⁵, R⁶, R^{a} and R^{b} are as defined herein.

In embodiments, the compound is a compound of formula (**IX**) or a pharmaceutically acceptable salt or solvate thereof: 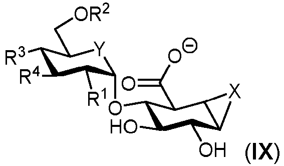 wherein X is selected from -O-, -N(H)-, -N(R^{a})-, and -N(C(O)R^{b})-; R³ is selected from -H, -OH, and -O-C₁₋₆ alkyl; and Y, R¹, R², R⁴, R^{a} and R^{b} are as defined herein.

In embodiments, the compound is a compound of formula (**Xa**), (**Xb**) or (**Xc**) or a pharmaceutically acceptable salt or solvate thereof: 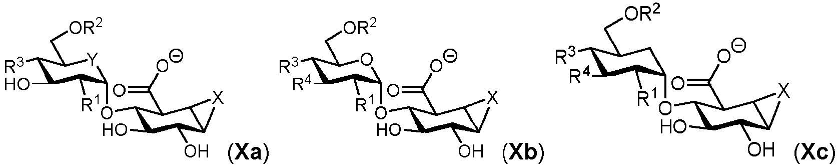 wherein X is selected from -O-, -N(H)-, -N(R^{a})-, and -N(C(O)R^{b})-; R³ is selected from -H, -OH, and -O-C₁₋₆ alkyl; and Y, R¹, R², R⁴, R^{a} and R^{b} are as defined herein.

In embodiments, the compound is a compound of formula (**XIa**), (**XIb**) or a pharmaceutically acceptable salt or solvate thereof: 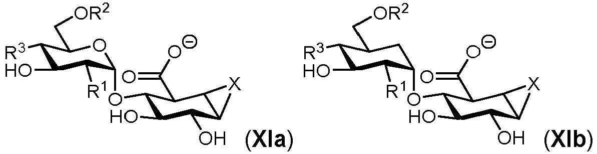 wherein X is selected from -O-, -N(H)-, -N(R^{a})-, and -N(C(O)R^{b})-; R³ is selected from -H, -OH, and -O-C₁₋₆ alkyl; and R¹, R², R^{a} and R^{b} are as defined herein.

In embodiments, the compound is a compound of formula (**XIIa**) or (**XIIb**) or a pharmaceutically acceptable salt or solvate thereof: 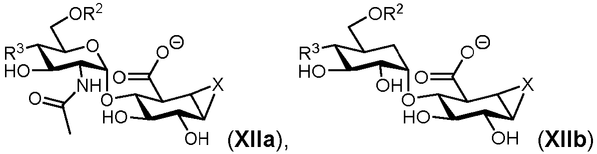 wherein X is selected from -O-, -N(H)-, -N(R^{a})-, and -N(C(O)R^{b})-; R³ is selected from -H, -OH, and -O-C₁₋₆ alkyl; and R², R^{a} and R^{b} are as defined herein.

In embodiments, the compound is a compound of formula (**XIIIa**), (**XIIIb)** or (**XIIIc**) or a pharmaceutically acceptable salt or solvate thereof: 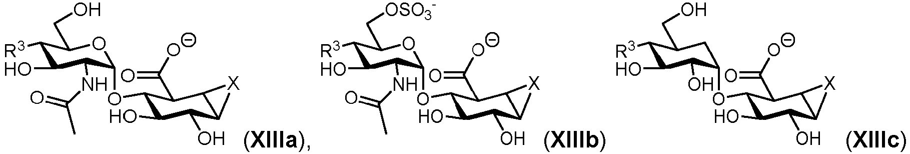 wherein X is selected from -O-, -N(H)-, -N(R^{a})-, and -N(C(O)R^{b})-; R³ is selected from -H, -OH, and -O-C₁₋₆ alkyl; and R^{a} and R^{b} are as defined herein.

In embodiments, the compound is a compound of formula (**XIVa**), (**XIVb**) or (**XIVc**) or a pharmaceutically acceptable salt or solvate thereof: 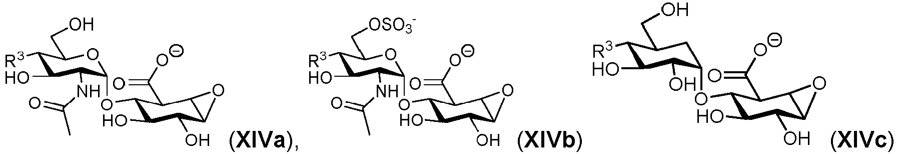 wherein R³ is selected from -H, -OH, and -O-C₁₋₆ alkyl.

In embodiments, the compound is a compound of formula (**XVa**), (**XVb**) or (**XVc**) or a pharmaceutically acceptable salt or solvate thereof: 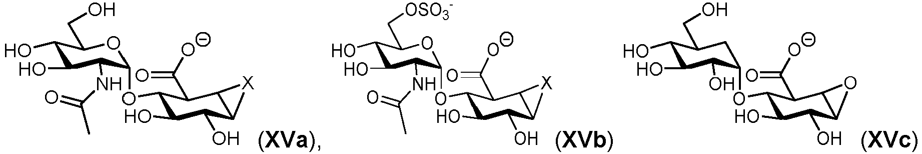 wherein X is selected from -O-, -N(H)-, -N(R^{a})-, and -N(C(O)R^{b})-; and R^{a} and R^{b} are as defined herein.

In embodiments, the compound is a compound of formula (**XVIa**) or (**XVIb**), or a pharmaceutically acceptable salt or solvate thereof: 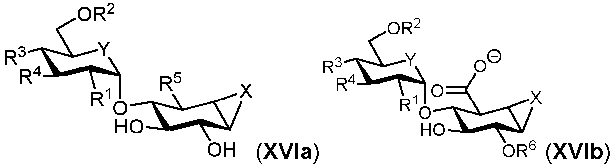 wherein X is -N-L₁-fluorophore- or -N-L₁-biotin-, and R³ is selected from -H, -OH, -O-C₁₋₆ or-OR⁷, where R⁷ is -H or -C₁₋₆ alkyl; or X is selected from -O-, -N(H)-, -N(R^{a})-, or -N(C(O)R^{b})-, and R³ is -L₂-fluorophore, -L₂-biotin or -OR⁷, where R⁷ is -L₃-fluorophore, or -L₃-biotin. It may be that X is -N-L₁-fluorophore- or -N-L₁-biotin-, and R³ is -OR⁷, where R⁷ is -H or -C₁₋₆ alkyl; or X is selected from -O-, -N(H)-, -N(R^{a})-, or -N(C(O)R^{b})-, and R³ is -OR⁷, where R⁷ is -L₃-fluorophore, or -L₃-biotin. Y, R¹, R², R⁴, R⁵, R⁶, R^{a}, R^{b}, -L₁-, -L₂- and -L₃- are as defined herein.

In embodiments, the compound is a compound of formula (**XVII**) or a pharmaceutically acceptable salt or solvate thereof: 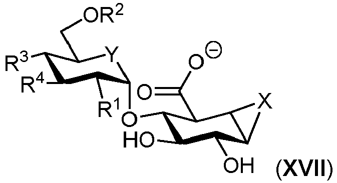 wherein X is -N-L₁-fluorophore- or -N-L₁-biotin-, and R³ is selected from -H, -OH, -O-C₁₋₆ or-OR⁷, where R⁷ is -H or -C₁₋₆ alkyl; or X is selected from -O-, -N(H)-, -N(R^{a})-, or -N(C(O)R^{b})-, and R³ is -L₂-fluorophore, -L₂-biotin or -OR⁷, where R⁷ is -L₃-fluorophore, or -L₃-biotin. It may be that X is -N-L₁-fluorophore- or -N-L₁-biotin-, and R³ is -OR⁷, where R⁷ is -H or -C₁₋₆ alkyl; or X is selected from -O-, -N(H)-, -N(R^{a})-, or -N(C(O)R^{b})-, and R³ is -OR⁷, where R⁷ is -L₃-fluorophore, or -L₃-biotin. Y, R¹, R², R⁴, R^{a}, R^{b}, -L₁-, -L₂- and -L₃- are as defined herein.

In embodiments, the compound is a compound of formula (**XVIIIa**), (**XVIIIb**) or (**XVIIIc**) or a pharmaceutically acceptable salt or solvate thereof: 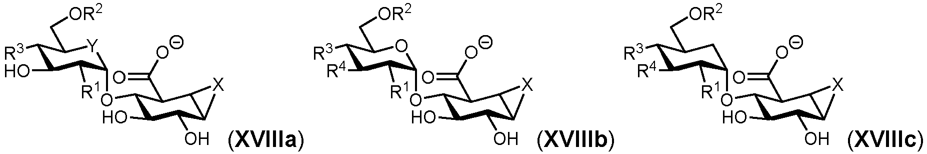 wherein X is -N-L₁-fluorophore- or -N-L₁-biotin-, and R³ is selected from -H, -OH, -O-C₁₋₆ or-OR⁷, where R⁷ is -H or -C₁₋₆ alkyl; or X is selected from -O-, -N(H)-, -N(R^{a})-, or -N(C(O)R^{b})-, and R³ is -L₂-fluorophore, -L₂-biotin or -OR⁷, where R⁷ is -L₃-fluorophore, or -L₃-biotin. It may be that X is -N-L₁-fluorophore- or -N-L₁-biotin-, and R³ is -OR⁷, where R⁷ is -H or -C₁₋₆ alkyl; or X is selected from -O-, -N(H)-, -N(R^{a})-, or -N(C(O)R^{b})-, and R³ is -OR⁷, where R⁷ is -L₃-fluorophore, or -L₃-biotin. Y, R¹, R², R⁴, R^{a}, R^{b}, -L₁-, -L₂- and -L₃- are as defined herein.

In embodiments, the compound is a compound of formula (**XIXa**), (**XIXb**) or a pharmaceutically acceptable salt or solvate thereof: 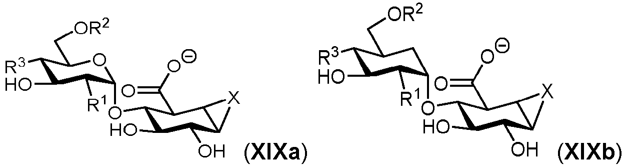 wherein X is -N-L₁-fluorophore- or -N-L₁-biotin-, and R³ is selected from -H, -OH, -O-C₁₋₆ or-OR⁷, where R⁷ is -H or -C₁₋₆ alkyl; or X is selected from -O-, -N(H)-, -N(R^{a})-, or -N(C(O)R^{b})-, and R³ is -L₂-fluorophore, -L₂-biotin or -OR⁷, where R⁷ is -L₃-fluorophore, or -L₃-biotin. It may be that X is -N-L₁-fluorophore- or -N-L₁-biotin-, and R³ is -OR⁷, where R⁷ is -H or -C₁₋₆ alkyl; or X is selected from -O-, -N(H)-, -N(R^{a})-, or -N(C(O)R^{b})-, and R³ is -OR⁷, where R⁷ is -L₃-fluorophore, or -L₃-biotin. R¹, R², R^{a}, R^{b}, -L₁-, -L₂- and -L₃- are as defined herein.

In embodiments, the compound is a compound of formula (**XXa**), (**XXb**) or a pharmaceutically acceptable salt or solvate thereof: 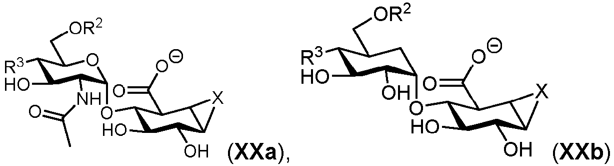 wherein X is -N-L₁-fluorophore- or -N-L₁-biotin-, and R³ is selected from -H, -OH, -O-C₁₋₆ or-OR⁷, where R⁷ is -H or -C₁₋₆ alkyl; or X is selected from -O-, -N(H)-, -N(R^{a})-, or -N(C(O)R^{b})-, and R³ is -L₂-fluorophore, -L₂-biotin or -OR⁷, where R⁷ is -L₃-fluorophore, or -L₃-biotin. It may be that X is -N-L₁-fluorophore- or -N-L₁-biotin-, and R³ is -OR⁷, where R⁷ is -H or -C₁₋₆ alkyl; or X is selected from -O-, -N(H)-, -N(R^{a})-, or -N(C(O)R^{b})-, and R³ is -OR⁷, where R⁷ is -L₃-fluorophore, or -L₃-biotin. R², R^{a}, R^{b}, -L₁-, -L₂- and -L₃- are as defined herein.

In embodiments, the compound is a compound of formula (**XXIa**), (**XXIb**), (**XXIc**) or a pharmaceutically acceptable salt or solvate thereof: 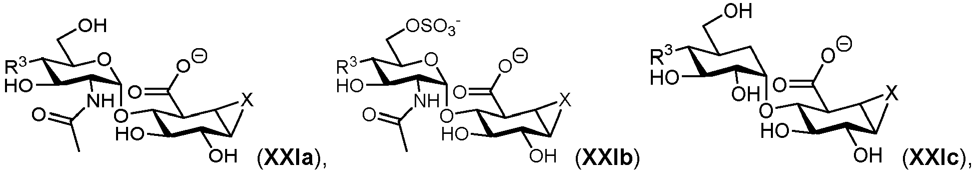 wherein X is -N-L₁-fluorophore- or -N-L₁-biotin-, and R³ is selected from -H, -OH, -O-C₁₋₆ or-OR⁷, where R⁷ is -H or -C₁₋₆ alkyl; or X is selected from -O-, -N(H)-, -N(R^{a})-, or -N(C(O)R^{b})-, and R³ is -L₂-fluorophore, -L₂-biotin or -OR⁷, where R⁷ is -L₃-fluorophore or -L₃-biotin. It may be that X is -N-L₁-fluorophore- or -N-L₁-biotin-, and R³ is -OR⁷, where R⁷ is -H or -C₁₋₆ alkyl; or X is selected from -O-, -N(H)-, -N(R^{a})-, or -N(C(O)R^{b})-, and R³ is -OR⁷, where R⁷ is -L₃-fluorophore, or -L₃-biotin. R^{a}, R^{b}, -L₁-, -L₂- and -L₃- are as defined herein.

In embodiments, the compound is a compound of formula (**XXIIa**), (**XXIIb**) or (**XXIIc**), or a pharmaceutically acceptable salt or solvate thereof: 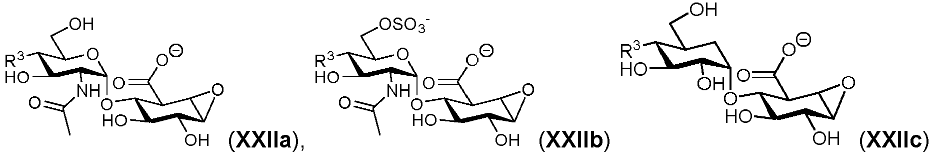 wherein R³ is -L₂-fluorophore, -L₂-biotin or -OR⁷, where R⁷ is -L₃-fluorophore or -L₃-biotin. R^{a}, R^{b}, -L₁-, -L₂- and -L₃- are as defined herein.

In embodiments, the compound is a compound of formula (**XXIIIa**), (**XXIIIb**) or (**XXIIIc**), or a pharmaceutically acceptable salt or solvate thereof: 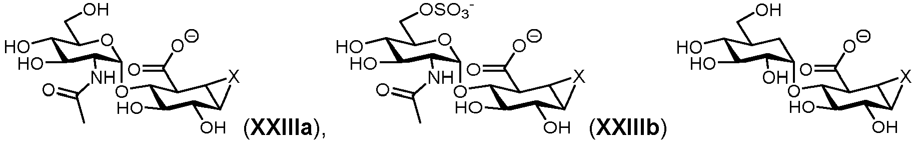 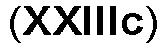 wherein X is -N-L₁-fluorophore- or -N-L₁-biotin-; and R^{a}, R^{b}, -L₁- and -L₂- are as defined herein.

In embodiments of the first aspect, one or more of X, Y, R¹, R², R³, R⁴, R⁵, R⁶, R⁷ R^{a}, R^{b}, L₁, L₂ and L₃ are as described in the following paragraphs:
X may be selected from -O-, -N(H)-, -N(R^{a})- and -N(C(O)R^{b})-. X may be selected from-O-, -N-L₁-fluorophore-, or -N-L₁-biotin. X may be selected from -N-L₁-fluorophore-, or -N-L₁-biotin. X may be -O-. X may be -N(H)-. X may be -N(R^{a})-. X may be -N(C(O)R^{b})-. X may be -N-L₁-fluorophore-. X may be -N-L₁-biotin.

Y may be selected from -O- and -(CH₂)-. Y may be -O-. Y may be -(CH₂)-.

R¹ may be selected from -H, -OH, and -NHC(O)CH₃. R¹ may be -H. R¹ may be -OH. R¹ may be -NHC(O)CH₃. R¹ may be -NHSO₃⁻.

R² may be -H. R² may be -SO₃⁻.

R³ may be selected from -H, -OH, and -O-C₁₋₆ alkyl (e.g. -O-CH₃, -O-CH₂CH₃, -O-CH(CH₃)₂). R³ may be -H. R³ may be -OH. R³ may be -O-C₁₋₆ alkyl (e.g. -O-CH₃, -O-CH₂CH₃,-O-CH(CH₃)₂).

R³ may be selected from -L₂-fluorophore and -L₂-biotin. R³ may be -L₂-fluorophore.

R³ may be -L₂-biotin.

X may be selected from -O-, -N(H)-, -N(R^{a})-, and -N(C(O)R^{b})-; and R³ may be selected from -H, -OH, -O-C₁₋₆ alkyl. X may be -O- and R³ may be selected from -H, -OH, -O-C₁₋₆ alkyl. X may be -O- and R³ may be -OH.

When R³ is -L₂-fluorophore, or -L₂-biotin, X is selected from -O-, -N(H)-, -N(R^{a})-, or-N(C(O)R^{b})-. X may be -O- and R³ may be -L₂-fluorophore or -L₂-biotin.

When X is -N-L₁-fluorophore- or -N-L₁-biotin-, R³ is selected from -H, -OH, or -O-C₁₋₆ alkyl. X may be -N-L₁-fluorophore- or -N-L₁-biotin-; and R³ may be -OH.

R⁴ may be -H. R⁴ may be -OH. R⁴ may be -O-C₁₋₆ alkyl (e.g. -O-CH₃, -O-CH₂CH₃, -O-CH(CH₃)₂). R⁴ may be -SO₃⁻. Preferably, R⁴ is -OH.

R⁵ may be -C(O)O⁻. R⁵ may be -C(O)OC₁₋₆ alkyl (e.g. -C(O)OCH₃, -C(O)OCH₂CH₃,-C(O)OCH(CH₃)₂). R⁵ may be -C(O)NH-C₁₋₆ alkyl (e.g. -C(O)NHCH₃, -C(O)NHCH₂CH₃,-C(O)NHCH(CH₃)₂). R⁵ may be -OPO₃⁻. R⁵ may be -PO₃⁻.

R⁶ may be -H. R⁶ may be -SO₃⁻.

R^{a} may be selected from C₁₋₆ alkyl (e.g. -CH₃, -CH₂CH₃, -CH(CH₃)₂), or C₁₋₆ alkyl interrupted by one or more ether linkages (e.g. -OCH₃, -OCH₂CH₃, -OCH₂CH₂OCH₃). R^{a} may be C₁₋₆ alkyl (e.g. -CH₃, -CH₂CH₃, -CH(CH₃)₂). R^{a} may be C₁₋₆ alkyl interrupted by one or more ether linkages (e.g. -OCH₃, -OCH₂CH₃, -OCH₂CH₂OCH₃).

R^{b} may be selected from C₁₋₆ alkyl (e.g. -CH₃, -CH₂CH₃, -CH(CH₃)₂), or C₁₋₆ alkyl interrupted by one or more ether linkages (e.g. -OCH₃, -OCH₂CH₃, -OCH₂CH₂OCH₃). R^{b} may be C₁₋₆ alkyl (e.g. -CH₃, -CH₂CH₃, -CH(CH₃)₂). R^{b} may be C₁₋₆ alkyl interrupted by one or more ether linkages (e.g. -OCH₃, -OCH₂CH₃, -OCH₂CH₂OCH₃).

-L₁- may comprise a -O-C₂₋₂₄-alkyl (e.g. -O-C₄₋₂₄-alkyl or -O-C₆₋₂₄-alkyl) or C₂₋₂₄-alkyl (e.g. C₄₋₂₄-alkyl or C₆₋₂₄-alkyl), optionally interrupted by one, two, or three of: -NC(O)-, -NH-, -O-, -C(O)-, -C(OH)-, -C(O)O- and heterocyclic ring system comprising 1,2,3-triazole. -L₁- may comprise a C₄₋₂₄-alkyl interrupted by one or two of -NC(O)-, -NH-, -O-, -C(O)-, -C(OH)-, -C(O)O-, or heterocyclic ring system comprising 1,2,3-triazole. -L₁- may comprise -O-C₄₋₂₄-alkyl or -C₄₋₂₄-alkyl interrupted by one of -NC(O)-, -O-, -C(O)O-, or heterocyclic ring system comprising 1,2,3-triazole. -L₁- may comprise a C₄₋₂₄-alkyl interrupted by two of -NC(O)-, -O-, -C(O)O-, or heterocyclic ring system comprising 1,2,3-triazole. -L₁- may comprise a C₄₋₂₄-alkyl interrupted by -NC(O)-. -L₁- may comprise a C₄₋₂₄-alkyl interrupted by -NC(O)- and-O-. -L₁- may comprise a C₄₋₂₄-alkyl interrupted by -NC(O)- and 1,2,3-triazole.

When present, -L₁-fluorophore may be selected from -O-(CH₂)ₙ-NC(O)-fluorophore, or -O-(CH₂)ₘ-NC(O)-(CH₂)ₚ-fluorophore, wherein each of n, m and p may be independently selected from 4, 5, 6, 7 or 8. n may be 4. m may be 7. p may be 5. -L₁-fluorophore may be -O-(CH₂)₄-NC(O)-fluorophore or -O-(CH₂)₇-NC(O)-(CH₂)₅-fluorophore.

-L₂- may comprise a -O-C₂₋₂₄-alkyl (e.g. -O-C₄₋₂₄-alkyl or -O-C₆₋₂₄-alkyl) or C₂₋₂₄-alkyl (e.g. C₄₋₂₄-alkyl or C₆₋₂₄-alkyl), optionally interrupted by one, two, or three of: -NC(O)-, -NH-, -O-, -C(O)-, -C(OH)-, -C(O)O- and heterocyclic ring system comprising 1,2,3-triazole. -L₂- may comprise a -O-C₄₋₂₄-alkyl or C₄₋₂₄-alkyl interrupted by one or two of -NC(O)-, -NH-, -O-, -C(O)-, - C(OH)-, -C(O)O-, or heterocyclic ring system comprising 1,2,3-triazole. -L₂- may comprise a -O-C₄₋₂₄-alkyl interrupted by one of -NC(O)-, -O-, -C(O)O-, or heterocyclic ring system comprising 1,2,3-triazole. -L₂- may comprise a C₄₋₂₄-alkyl interrupted by one of -NC(O)-, -O-, -C(O)O-, or heterocyclic ring system comprising 1,2,3-triazole. -L₂- may comprise a C₄₋₂₄-alkyl interrupted by two of -NC(O)-, -O-, -C(O)O-, or heterocyclic ring system comprising 1,2,3-triazole. -L₂- may comprise a -O-C₄₋₂₄-alkyl interrupted by -NC(O)-. -L₂- may comprise a -O-C₄₋₂₄-alkyl interrupted by -NC(O)- and -O-. -L₂- may comprise a C₄₋₂₄-alkyl interrupted by -NC(O)-. -L₂- may comprise a C₄₋₂₄-alkyl interrupted by -NC(O)- and -O-. -L₂- may comprise a C₄₋₂₄-alkyl interrupted by-NC(O)- and 1,2,3-triazole.

When present, -L₂-fluorophore may be selected from -O-(CH₂)ₙ-NC(O)-fluorophore, or -O-(CH₂)ₘ-NC(O)-(CH₂)ₚ-fluorophore, wherein each of n, m and p may be independently selected from 4, 5, 6, 7 or 8. n may be 4. m may be 7. p may be 5. -L₂-fluorophore may be-O-(CH₂)₄-NC(O)-fluorophore or -O-(CH₂)₇-NC(O)-(CH₂)₅-fluorophore.

When present, each occurrence of fluorophore may be independently selected from cyanine 2, cyanine 3, cyanine 5, BODIPY, fluorescein, tetramethylrhodamine. The fluorophore may be cyanine 5.

The compound may be selected from the following, or a pharmaceutically acceptable salt, solvate, or prodrug thereof:

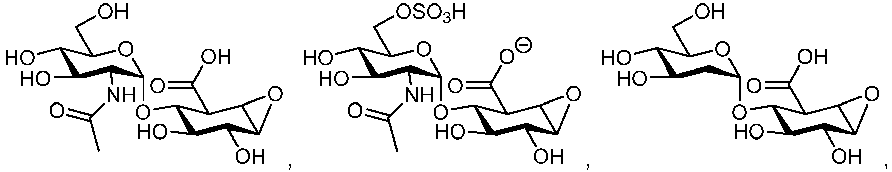

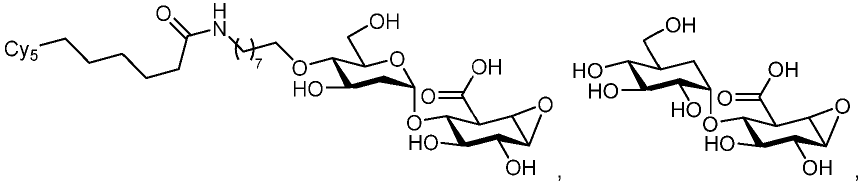

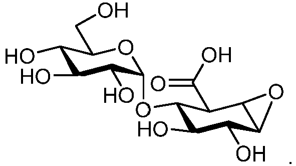

The compound may be selected from:

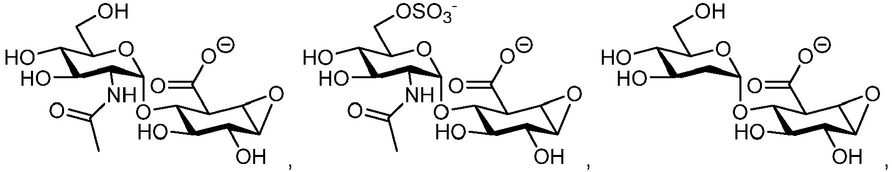

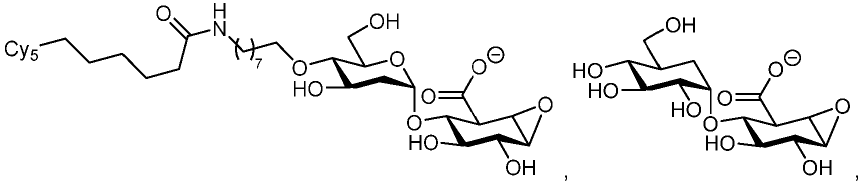

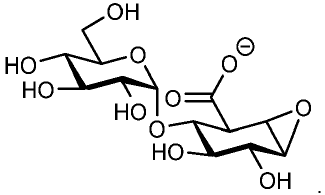

The compound may be selected from:

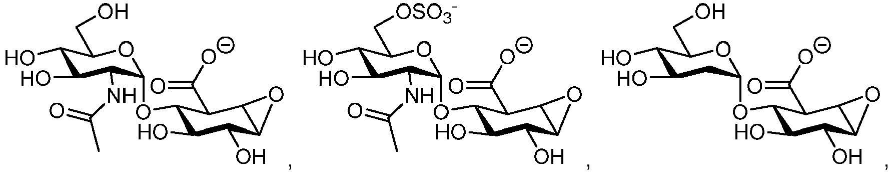

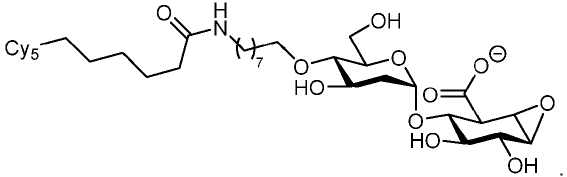

In another aspect, the invention provides compounds of formula **(XXIV)** as previously described, or a pharmaceutically acceptable salt, stereoisomer, solvate or prodrug thereof.

In embodiments of the second aspect, one or more of X, R¹, R², R⁵, R⁶, R⁷ R^{a}, R^{b}, L₁ and L₃ are as described in the following paragraphs:
X may be selected from -O-, -N-L₁-fluorophore-, or -N-L₁-biotin. X may be selected from -N-L₁-fluorophore-, or -N-L₁-biotin. X may be -O. X may be -N-L₁-fluorophore-. X may be -N-L₁-biotin.

R¹ may be selected from -H, -OH, and -NHC(O)CH₃. R¹ may be -H. R¹ may be -OH. R¹ may be -NHC(O)CH₃. R¹ may be -NHSO₃⁻.

R² may be -H. R² may be -SO₃⁻.

R⁵ may be -C(O)O⁻. R⁵ may be -C(O)OC₁₋₆ alkyl (e.g. -C(O)OCH₃, -C(O)OCH₂CH₃,-C(O)OCH(CH₃)₂). R⁵ may be -C(O)NH-C₁₋₆ alkyl (e.g. -C(O)NHCH₃, -C(O)NHCH₂CH₃,-C(O)NHCH(CH₃)₂). R⁵ may be -OPO₃⁻. R⁵ may be -PO₃⁻.

R⁶ may be -H. R⁶ may be -SO₃⁻.

R⁷ may be selected from -H and -C₁₋₆ alkyl (e.g. -CH₃, -CH₂CH₃, -CH(CH₃)₂). R⁷ may be H. R⁷ may be -C₁₋₆ alkyl (e.g. -CH₃, -CH₂CH₃, -CH(CH₃)₂). R⁷ may be selected from -L₃-fluorophore and -L₃-biotin. R⁷ may be -L₃-fluorophore. R⁷ may be -L₃-biotin.

When R⁷ is -L₃-fluorophore or -L₃-biotin, X is selected from -O-, -N(H)-, -N(R^{a})-, or-N(C(O)R^{b})-. When R⁷ is -L₃-fluorophore or -L₃-biotin, X may be -O-.

When X is -N-L₁-fluorophore- or -N-L₁-biotin-, R⁷ is selected from -H, -C₁₋₆ alkyl. When X is -N-L₁-fluorophore- or -N-L₁-biotin-, R⁷ may be -H.

-L₁- may comprise a -O-C₂₋₂₄-alkyl (e.g. -O-C₄₋₂₄-alkyl or -O-C₆₋₂₄-alkyl) or C₂₋₂₄-alkyl (e.g. C₄₋₂₄-alkyl or C₆₋₂₄-alkyl), optionally interrupted by one, two, or three of: -NC(O)-, -NH-, -O-, -C(O)-, -C(OH)-, -C(O)O- and heterocyclic ring system comprising 1,2,3-triazole. -L₁- may comprise a C₄₋₂₄-alkyl interrupted by one or two of -NC(O)-, -NH-, -O-, -C(O)-, -C(OH)-, -C(O)O-, or heterocyclic ring system comprising 1,2,3-triazole. -L₁- may comprise -O-C₄₋₂₄-alkyl or -C₄₋₂₄-alkyl interrupted by one of -NC(O)-, -O-, -C(O)O-, or heterocyclic ring system comprising 1,2,3-triazole. -L₁- may comprise a C₄₋₂₄-alkyl interrupted by two of -NC(O)-, -O-, -C(O)O-, or heterocyclic ring system comprising 1,2,3-triazole. -L₁- may comprise a C₄₋₂₄-alkyl interrupted by -NC(O)-. -L₁- may comprise a C₄₋₂₄-alkyl interrupted by -NC(O)- and-O-. -L₁- may comprise a C₄₋₂₄-alkyl interrupted by -NC(O)- and 1,2,3-triazole.

When present, -L₁-fluorophore may be selected from -O-(CH₂)ₙ-NC(O)-fluorophore, or -O-(CH₂)ₘ-NC(O)-(CH₂)ₚ-fluorophore, wherein each of n, m and p may be independently selected from 4, 5, 6, 7 or 8. n may be 4. m may be 7. p may be 5. -L₁-fluorophore may be -O-(CH₂)₄-NC(O)-fluorophore or -O-(CH₂)₇-NC(O)-(CH₂)₅-fluorophore.

-L₃- may comprise a C₂₋₂₄-alkyl (e.g. C₄₋₂₄-alkyl or C₆₋₂₄-alkyl), optionally interrupted by one, two, or three of: -NC(O)-, -NH-, -O-, -C(O)-, -C(OH)-, -C(O)O- and heterocyclic ring system comprising 1,2,3-triazole. -L₃- may comprise a C₄₋₂₄-alkyl interrupted by one or two of -NC(O)-,-NH-, -O-, -C(O)-, -C(OH)-, -C(O)O-, or heterocyclic ring system comprising 1,2,3-triazole. -L₃- may comprise a C₄₋₂₄-alkyl interrupted by one of -NC(O)-, -O-, -C(O)O-, or heterocyclic ring system comprising 1,2,3-triazole. -L₃- may comprise a C₄₋₂₄-alkyl interrupted by two of -NC(O)-, -O-, -C(O)O-, or heterocyclic ring system comprising 1,2,3-triazole. -L₃- may comprise a C₄₋₂₄-alkyl interrupted by -NC(O)-. -L₃- may comprise a C₄₋₂₄-alkyl interrupted by -NC(O)- and 1,2,3-triazole. L₃- may comprise a C₄₋₂₄-alkyl interrupted by -NC(O)- and -O-.

When present, -L₃-fluorophore may be selected from -(CH₂)ₙ-NC(O)-fluorophore, or-(CH₂)ₘ-NC(O)-(CH₂)ₚ-fluorophore, wherein each of n, m and p may be independently selected from 4, 5, 6, 7 or 8. n may be 4. m may be 7. p may be 5. -L₃-fluorophore may be -(CH₂)₄-NC(O)-fluorophore or -(CH₂)₇-NC(O)-(CH₂)₅-fluorophore.

When present, each occurrence of fluorophore may be independently selected from cyanine 2, cyanine 3, cyanine 5, BODIPY, fluorescein, tetramethylrhodamine. The fluorophore may be cyanine 5.

The compounds of the first or second aspects may be substrates for heparanase (HPSE).

Without wishing to be bound by theory, it is thought that elaboration of a cyclophellitol-derived warhead with a α-1,4 glucosaminyl type (or related) moiety in the compounds of the invention blocks engagement with exo-acting β-glucuronidases such as GUSB, due to steric clashes with their 'pocket-like' active sites, whilst simultaneously enhancing reactivity with HPSE itself, whose more open substrate binding cleft has additional interacting subsites that bind HS like compounds.

The compounds of the second aspect may be enzymatic labels for heparanase (HPSE).

### COMPOSITIONS AND ADMINISTRATION

According to a further aspect of the invention, there is provided a pharmaceutical composition comprising a compound of the invention. For example. The pharmaceutical composition may comprise a compound of any of formulae **(I)** to **(XXIV)**. The pharmaceutical composition may comprise a compound of any of formulae **(VIII)** to **(XV)**. The pharmaceutical composition may comprise a compound of any of formulae **(XVI)** to **(XXIII)**. The composition may further comprise a pharmaceutically acceptable excipient. The composition may provide the compound in admixture with at least one pharmaceutically acceptable adjuvant, carrier, or diluent.

Compounds or compositions of the invention may be administered orally, topically, intravenously, subcutaneously, buccally, rectally, dermally, nasally, tracheally, bronchially, by any other parenteral route, as an oral or nasal spray or via inhalation. Preferably, the compounds or compositions are formulated for non-oral administration. The compounds may be administered in the form of pharmaceutical preparations comprising the compound either as a free compound or, for example, a pharmaceutically acceptable non-toxic organic or inorganic acid or base addition salt, in a pharmaceutically acceptable dosage form. Depending upon the disorder and patient to be treated and the route of administration, the compositions may be administered at varying doses.

Actual dosage levels of active ingredients in the pharmaceutical formulations and pharmaceutical compositions of this invention may be varied so as to obtain an amount of the active compound(s) that is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved. For example, a compound of the invention could be started at a dose of about 5 µmol per kg (e.g. by injection) and dosage could be gradually increased until the desired effect is achieved.

Pharmaceutical compositions of this invention for parenteral (e.g. intravenous) injection may comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters, such as ethyl oleate. Proper fluidity can be maintained, for example, by the use of coating materials, such as lecithin, by the maintenance of the required particle size in the case of dispersions and by the use of surfactants.

These compositions may also contain adjuvants such as preservative, wetting agents, emulsifying agents and dispersing agents. Inhibition of the action of microorganisms may be ensured by the inclusion of various antibacterial and antifungal agents, for example, paraben, chlorobutanol or phenol sorbic acid. It may also be desirable to include isotonic agents, such as sugars or sodium chloride, for example. Prolonged absorption of the injectable pharmaceutical form may be brought about by the inclusion of agents (for example, aluminium monostearate and gelatine) which delay absorption.

Solid dosage forms for oral administration include capsules, tablets, pills, powders and granules. In such solid dosage forms, the active compound is typically mixed with at least one inert, pharmaceutically acceptable excipient or carrier, such as sodium citrate or dicalcium phosphate and/or one or more: a) fillers or extenders, such as starches, lactose, sucrose, glucose, mannitol and silicic acid; b) binders, such as carboxymethylcellulose, alginates, gelatine, polyvinylpyrrolidone, sucrose and acacia; c) humectants, such as glycerol; d) disintegrating agents, such as agar-agar, calcium carbonate, potato or tapioca starch, alginic acid, certain silicates and sodium carbonate; e) solution retarding agents, such as paraffin; f) absorption accelerators, such as quaternary ammonium compounds; g) wetting agents, such as acetyl alcohol and glycerol monostearate; h) absorbents, such as kaolin and bentonite clay and i) lubricants, such as talc, calcium stearate, magnesium stearate, solid polyethylene glycols, sodium lauryl sulfate and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Oral formulations may contain a dissolution aid. Examples of dissolution aids include non-ionic surface active agents, such as sucrose fatty acid esters, glycerol fatty acid esters, sorbitan fatty acid esters (e.g. sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkyamine oxides; bile acid and salts thereof (e.g. chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsufonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract, and/or in delayed fashion. Examples of embedding compositions include polymeric substances and waxes.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavoring and perfuming agents. Suspensions, in addition to the active compounds, may contain suspending agents such as ethoxylated isostearyl alcohols, polyoxyethylene sorbitol and sorbitan esters, microcrystalline cellulose, aluminium metahydroxide, bentonite, agaragar, and traganacanth and mixtures thereof.

Compositions for rectal or vaginal administration may be in the form of suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, sprays, creams, foams, gels, ointments and inhalants. The active compound is mixed under sterile conditions with a pharmaceutically acceptable carrier and any needed preservatives, buffers or propellants which may be required. Ophthalmic formulations, eye ointments, powders and solutions are also contemplated as being within the scope of this invention.

The compositions according to the present subject matter may also contain inactive components. Suitable inactive components are well known in the art and are described in standard textbooks, such as Goodman and Gillman's: The Pharmacological Bases of Therapeutics, 13thEd., Brunton et al., Eds. McGraw-Hill Education (2017), and Remington's Pharmaceutical Sciences, 17th Ed., Mack Publishing Co., Easton, Pa. (1990).

The compositions may be used in combination with an additional pharmaceutical dosage form to enhance their effectiveness in treating any of the disorders described herein. For example, the compositions may be used in combination with bortezomib. In this regard, the present formulations may be administered as part of a regimen additionally including any other pharmaceutical and/or pharmaceutical dosage form known in the art as effective for the treatment of any of these disorders.

### USES AND METHODS

The compounds of the invention are inhibitors of heparanase (HPSE). HPSE plays a key role in the degradation of heparan sulfate proteoglycans (HSPGs). Excessive HSPG degradation facilitates cancer cell migration to and from the vasculature, whilst growth factors and cytokines released upon HSPG degradation stimulate proliferation and angiogenesis. Thus, overexpression of HPSE (e.g. by cancer cells) strongly drives HSPG degradation, thereby resulting in aggressive metastatic cancers. Thus, by inhibiting HPSE, the compounds of the invention have the therapeutic effect of minimizing HSPG degradation, thereby reducing cancer aggression.

In addition, as HPSE is often overexpressed by aggressive metastatic cancers, compounds of the invention that comprise a therapeutic moiety may provide targeting of the therapeutic moiety to said aggressive metastatic cancers.

An aspect of the invention provides a compound of the invention or a pharmaceutical composition of the invention, for use as a medicament. The compound may, for example, be a compound of any of formulae **(I)** to **(XXIV).** The composition may comprise a compound of any of formulae **(I)** to **(XXIV).** Optionally, the compound may be a compound of any of formulae **(VIII)** to **(XV).**

An aspect of the invention provides a compound of the invention or a pharmaceutical composition of the invention, for use in the treatment of a condition which is modulated by heparanase (HPSE). The compound may, for example, be a compound of any of formulae **(I)** to **(XXIV).** The composition may comprise a compound of any of formulae **(I)** to **(XXIV).** Optionally, the compound may be a compound of any of formulae **(VIII)** to **(XV).**

Another aspect of the invention provides a compound of the invention, or a pharmaceutical composition of the invention, for use in the treatment of a condition selected from: cancer, inflammation, inflammatory lung injury, rheumatoid arthritis, chronic colitis, neuroinflammation, sepsis-associated lung injury, inflammatory bowel disease, diabetes, diabetic nephropathy, bone osteolysis, thrombosis, artherosclerosis, inflammatory kidney disease, acute kidney injury, and viral infection (e.g. viral infection caused by herpes simplex virus, dengue virus, human papillomavirus, respiratory syncytial virus, adenovirus, hepatitis C virus, porcine respiratory virus, reproductive syncytial virus, or retroviruses). The compound may, for example, be a compound of any of formulae **(I)** to **(XXIV).** The composition may comprise a compound of any of formulae **(I)** to **(XXIV).** Optionally, the compound may be a compound of any of formulae **(VIII)** to **(XV).**

The condition may be cancer. Exemplary types of cancer include: breast cancer, lung cancer, pancreatic cancer, colorectal cancer, oesophageal cancer, head and neck cancers, cervical cancer, prostate cancer, oral cancer, gastric cancer, thyroid cancer, liver cancer, bladder cancer, melanoma, lymphoma, leukaemia, and myeloma.

Another aspect of the invention provides a method of inhibiting heparanase (HPSE), comprising administration to a cell of an effective amount of a compound of the invention, or a pharmaceutical composition of the invention. The compound may, for example, be a compound of any of formulae **(I)** to **(XXIV).** The composition may comprise a compound of any of formulae **(I)** to **(XXIV).**

The administration is *in vitro.*

Another aspect of the invention provides use of a compound of the invention, or a pharmaceutical composition of the invention, for the inhibition of heparanase (HPSE).

The use is an *in vitro* use. The compound may, for example, be a compound of any of formulae **(I)** to **(XXIV).** The composition may comprise a compound of any of formulae **(I)** to **(XXIV).**

Another aspect of the invention provides use of a compound of the invention, or a pharmaceutical composition of the invention, for the enzymatic labelling of heparanase (HPSE), wherein said compound comprises a detectable moiety. The detectable moiety may be a fluorophore or a biotin. The fluorophore may be as defined herein. The biotin may be conjugated to an avidin or streptavidin conjugate as defined herein. The compound may, for example, be a compound of any of formulae **(I)** to **(XXIV).** The composition may comprise a compound of any of formulae **(I)** to **(XXIV).** Optionally, the compound may be a compound of any of formulae **(XVI)** to **(XXIII).**

Another aspect of the invention provides a method of treating a condition selected from: cancer, inflammation, inflammatory lung injury, rheumatoid arthritis, chronic colitis, neuroinflammation, sepsis-associated lung injury, inflammatory bowel disease, diabetes, diabetic nephropathy, bone osteolysis, thrombosis, artherosclerosis, inflammatory kidney disease, acute kidney injury, and viral infection (e.g. viral infection caused by herpes simplex virus, dengue virus, human papillomavirus, respiratory syncytial virus, adenovirus, hepatitis C virus, porcine respiratory virus, reproductive syncytial virus, or retroviruses); the method comprising administering a pharmaceutically effective amount of a compound of the invention, or a pharmaceutical composition of the invention, to a patient in need thereof. The compound may, for example, be a compound of any of formulae **(I)** to **(XXIV).** The composition may comprise a compound of any of formulae **(I)** to **(XXIV).**

The condition may be cancer. Exemplary types of cancer include: breast cancer, lung cancer, pancreatic cancer, colorectal cancer, oesophageal cancer, head and neck cancers, cervical cancer, prostate cancer, oral cancer, gastric cancer, thyroid cancer, liver cancer, bladder cancer, melanoma, lymphoma, leukaemia, and myeloma.

Another aspect of the invention provides a method of labelling heparanase (HPSE) *in vitro* or *in vivo,* the method comprising administering to a cell or a patient an effective amount of a compound of the invention, or of a pharmaceutical composition of the invention, wherein said compound comprises a fluorophore or a biotin. The fluorophore may be as defined herein. The biotin may be conjugated to an avidin or streptavidin conjugate as defined herein.

The method may be an *in vitro* method. The cells may be cancer cells. The cells may be in a cell culture. The compound may, for example, be a compound of any of formulae **(I)** to **(XXIV)**; optionally, the compound may be a compound of any of formulae **(XVI)** to **(XXIII)**. The composition may comprise a compound of any of formulae **(I)** to **(XXIV)**; optionally, the composition may comprise a compound of any of formulae **(XVI)** to **(XXIII).**

### ASSAYS

Compounds of the invention can be assessed for biological activity using any suitable assay that would be known to the person skilled in the art. Exemplary assays that are useful for the assessment of compounds of the invention are provided in the following paragraphs.

Initial evaluations of the selectivity and potency of the compounds of the invention were performed using a panel of recombinant β-glucuronidase enzymes. Humans express two distinct and unrelated β-glucuronidases: *endo*-β-glucuronidase HPSE falls under the GH79 family of the sequence-based Carbohydrate Active enzymes (CAZy) classification (Poulain et al., Development,142, 3456-67 (2015)), whilst *exo*-β-glucuronidase GUSB is a CAZy GH2 family enzyme involved in lysosomal mucopolysaccharide breakdown. Recombinant human HPSE was prepared for inhibitor testing, along with its precursor proHPSE, the active site of which is occluded by a linker peptide, and should thus be unreactive to pseudo-disaccharides. Due to the intractability of producing human GUSB, a GH2 ortholog from *E. coli* - EcGUSB was employed, which shares 47.6% sequence identity with the human enzyme, including full conservation of active site residues (Fig 3). In additional, bacterial GH79 orthologs BpHep from *B. pseudomallei* and AcGH79 from *A. capsulatum* were assessed, which share 20.1% and 20.8% sequence identity with HPSE respectively (Fig 4).

In the assays described below, unless the context requires otherwise, compounds mentioned by number are the corresponding compounds illustrated in Figure 1B.

### Competitive Activity-Based Protein Profiling (cABPP) Assay

### cABPP Assay 1

ABP labelling reactions were carried out in Mcllvaine buffer (pH 5.5), 300 mM NaCl. For recombinant proteins 200 fmol of EcGH2, BpHep, AcGH79, HPSE or proHPSE was used (20 nM in 10 µl final reaction volume). For labeling cell lysates, 4-20 µg total protein was used. Labeling reactions were initiated by the addition of ABP compounds 7, 8 or 9 to a final concentration of 100 nM (unless otherwise specified), before incubation for 30 min at 37°C with shaking. Following labeling, samples were denatured by boiling with Laemmli buffer for 5 min and resolved by SDS-PAGE. Gels were scanned using Typhoon-5 laser scanner platform (Cytiva), using the λEX 635 nm laser and 670BP30 emission filter. For competitive ABPP (cABPP) experiments, protein mixtures were preincubated with inhibitor (or buffer only control) for 1 h at 37°C with shaking, before the addition of 7 to a final concentration of 100 nM, and subsequent incubation for 30 min at 37°C with shaking. SDS-PAGE and analysis steps were carried out as above. For quantitative measurements, fluorescently labeled band intensities were calculated using Imagequant (Cytiva), and normalized to the corresponding band in the inhibitor free control lane. cABPP plots were prepared and analyzed using SigmaPlot 14.0 (Systat).

### cABPP Assay 2

For further competitive ABPP studies, a working protein mixture was prepared by mixing the platelet lysates (87.5 µL), buffer (pH 5.0, 150 mM, 157.5 µL) and 3 M NaCl solution (35 µL). The enzymatic reactions were carried out at a constant concentration of read-out 7 (100 nM, final). For each inhibitor 2, 12 and 13, triplicate sets of ten 0.5 mL low protein binding Eppendorf tubes were prepared. Working solutions of each inhibitor (1% DMSO (w/v), 5 µL) in a range of ten concentrations (100, 50, 25, 12.5, 6.3, 3.1, 1.6, 0.8, 0.4, 0 µM) were prepared with MilliQ water and kept on ice. The working protein mixture (8 µL) was added in each protein low binding 0.5 mL Eppendorf tube, and 1 µL of inhibitor solution was added while on ice.

### Platelet lysates

Further cABPP studies were undertaken using platelet lysates to provide a more physiologically realistic environment. Platelet lysates contain complex mixtures of overlapping enzyme activities, including both *endo-* and *exo*-β-glucuronidases HPSE and GUSB respectively, thus provide a challenging environment to test inhibitor specificity.

### Whole cell lysates

To estimate the broader proteome-wide specificity of our inhibitors, we also applied fluorescent ABPs 7, 8 and 9 at 1 µM and 10 µM concentration to whole cell lysates (4-20 µg total protein per reaction) from a panel of human cancer cell lines: U87, PANC1, SKBR3, MDA-MB-231, HT1080, as well as platelet lysates.

### X-Ray Crystallography

Xray diffraction data were collected at 100 K at beamlines i03 (HPSE-**2**, HPSE-**3**, HPSE-**4**, EcGUSB-**1**, BpHep-**3**), i04 (AcGH79-**2**, BpHep-**2**) and i04-1 (Apo-AcGH79, AcGH79-**1**, AcGH79-**3**) of the Diamond Light Source UK. Reflections were autoprocessed using the XIA2 pipeline. Complexes were solved by directly refining against their unliganded structures where isomorphous, or else solved by molecular replacement with PHASER (search model PDB accessions: 5E98 (HPSE), 3VNY (AcGH79), 3K46 (EcGUSB), 5BWI (BpHep)). Solved structures were iteratively improved by alternating rounds of manual model building and maximum-likelihood refinement using COOT and REFMAC5 respectively. Ligand coordinates were built using jLigand. Diagrams were generated using PyMOL.

### Fondaparinux assay

Fondaparinux cleavage was performed essentially as previously described in Anal Biochem 396, 112-6 (2010). Briefly, assays were carried out in 96 well plates (Nunc) pretreated with 4% BSA in TBS-T (20 mM Tris pH 7.4, 150 mM NaCl, 0.1% (v/v) Tween 20). 100 µL reactions were set up in 40 mM NaOAc pH 5.0 buffer, containing final concentrations of 200 ng/mL HPSE, 100 µM Fondaparinux (Arixtra) and specified concentrations of inhibitors. Reactions were incubated at 37°C for 5 h, then quenched with 100 µL 0.1 M NaOH supplemented with 1.7 mM WST-1 tetrazolium salt, and incubated at 60°C for 1 h. Absorbance at 584 nm was read using an ELISA plate reader (BIO-TEK Instruments). All reactions were carried out in technical duplicate. Data were plotted and analyzed using Sigmaplot (Systat).

### HSPG digest assay

ECM coated 35 mm culture dishes containing ³⁵S labelled HS were prepared as previously described (Vlodavsky et al., Curr. Protoc. Cell. Biol., Chapter 10, Unit 10, 14). Briefly, bovine corneal endothelial cells were plated at an initial density of 2x105 cells/ml, with 4% dextran T-40 (Sigma) included in the growth medium. Na₂³⁵SO₄ (25 µCi/ml) was added to the incubation medium on days 2 and 5 after seeding. On day 12, subendothelial ECM is exposed by dissolving the cells with PBS containing 0.5% Triton X-100 and 20 mM NH₄OH, followed by four washes with PBS. For HS-degradation assays, ECM coated dishes were incubated with 200 ng recombinant HPSE in 1 mL reaction mixture (10 mM buffer Phosphate-Citrate pH 6.0, 50 mM NaCl, 1 mM DTT, 1 mM CaCl₂) containing HPSE inhibitors or DMSO control. Plates were incubated at 37°C for 5 h, then assay medium collected and applied to a 0.9×30 cm Sepharose 6B size exclusion column. The Sepharose 6B column was eluted with PBS, and ³⁵S radioactivity of 0.2 mL fractions quantitated using a liquid scintillation analyzer (Packard BioScience). Digest assays with U87 cell lysates were carried out in a similar fashion, except plates were incubated for 18 h with cell extracts prepared from 1x10⁶ U87 glioma cells lysed by 3x freeze/thaw cycles.

### Matrigel Invasion Assay

The Matrigel invasion assay was used to assess the effects of the compounds of the invention on cancer cell aggression. This is an *in cellulo* model of cell invasion through a HSPG rich basement membrane. The ability of cells to migrate through transwell inserts coated with Matrigel (an ECM secretion from Engelbreth-Holm-Swarm mouse sarcoma cells) correlates well with their *in vivo* invasive potential (Hall et al., Methods Mol Biol, 1070, 1-11 (2014)).

1x10⁵ U87 cells in 200 µL DMEM media (without serum) were seeded onto the top chamber of a Matrigel-coated Boyden transwell filter, alongside 20 µM HPSE inhibitor (or PBS control). Cells in the upper chamber were separated by Matrigel from a lower chamber containing 600 µL DMEM, supplemented with 10% FBS. Plates were incubated at 37 °C for 4 h, before fixing cells in 4% (v/v) paraformaldehyde, and staining with 0.5% (w/v) crystal violet. Matrigel and non-migrating cells in the upper side of the Boyden filter were removed by swabbing, then invading cells on the bottom side of the transwell filter imaged for analysis. 10 randomly selected microscopic fields were recorded, and cell invasion calculated using ImageJ, by quantitating the percentage of the field stained by crystal violet. Graphs were plotted using RStudio.

### In vivo murine models of cancer metastasis

*B16 melanoma* - Twenty minutes before tumor cell inoculation, C57BL/J6 mice (n=5) were injected intraperitoneally with vehicle alone (PBS), compound **2** (300 nmol/mouse), or SST0001 (150 µg/mouse). Mice were subsequently intravenously inoculated with 1.5x10⁵ B16-BL6 melanoma cells/mouse in a volume of 100 µL, alongside 20 µM **2** or 10 µg SST0001. Mice were sacrificed after 14 days and the lungs were dissected, photographed and evaluated for the number of metastatic foci/lung.

4T1 breast cancer - Twenty minutes before tumor cell inoculation, Balb/c mice (n=5) were injected intraperitoneally with vehicle alone (PBS), compound **2** (300 nmol/mouse), or SST0001 (150 µg/mouse). Mice were subsequently intravenously inoculated with 1.5x10⁵ luciferase tagged 4T1 mouse mammary carcinoma cells/mouse in a volume of 100 µL, alongside 20 µM 2 or 10 µg SST0001.

Bioluminescence imaging of lung metastases produced by luciferase-expressing 4T1 cells was carried out using a highly sensitive, cooled charge-coupled device camera assembled in a specimen box (IVIS; Xenogen Corp, Hopkinton, MA). For this, the tumor-bearing mice were intraperitoneally administered with a luciferase substrate D-luciferin at a dose of 150 mg/kg body weight. The mice were anesthetized and kept in a camera box followed by continuous exposure to isoflurane (EZAnesthesia, Palmer, PA, USA). The tumor burden was quantified using Living Image software (Xenogen) (U. Barash et al., Journal of the National Cancer Institute 110, 1102-1114, 2018; M. Weissmann et al., Proc Natl Acad Sci, 113, 704-709, 2016)

*CAG myeloma* - Luciferase-labeled CAG human myeloma cells (3x10⁶) were injected into the tail vein of NOD/SCID mice. 3-5 days after cell inoculation, mice were randomly assigned to 4 cohorts (6 mice each) receiving: (a) vehicle, (b) compound **2** (i.p. 300 nmol/day), (c) bortezomib (0.5 mg/kg twice weekly) or (d) **2** plus bortezomib (dosing as for individual compounds). Tumor development was inspected (every 7 days) by IVIS imaging as detailed above. Tumor burden was quantified using Living Image software. Plots and statistical analyses were carried out in Prism 9 (Graphpad software).

### Materials and Methods

Chemicals were purchased from Acros, Sigma Aldrich, Biosolve, VWR, Fluka, Merck, Carbosynth and Fisher Scientific. All chemicals were used as received unless stated otherwise.

Tetrahydrofuran (THF), dichloromethane (DCM), N,N-dimethylformamide (DMF) and toluene were stored over molecular sieves before use. Traces of water from reagents were removed by co-evaporation with toluene in reactions that required anhydrous conditions.

All reactions were performed under a nitrogen atmosphere unless stated otherwise.

TLC analysis was conducted using Merck aluminum sheets (Silica gel 60 F₂₅₄) with detection by UV absorption (254 nm), by spraying with a solution of (NH₄)₆Mo₇O₂₄·4H₂O (25 g/L) and (NH₄)4Ce(SO₄)₄·2H₂O (10 g/L) in 10% sulfuric acid or a solution of KMnO₄ (20 g/L) and K₂CO₃ (10 g/L) in water, followed by charring at ~150 °C.

Column chromatography was performed using Screening Device b.v. silica gel (particle size of 40 - 63 µm, pore diameter of 60 A) with the indicated eluents. For reversed-phase HPLC purifications an Agilent Technologies 1200 series instrument equipped with a semi-preparative column (Gemini C18, 250 x 10 mm, 5 µm particle size, Phenomenex) was used.

LC/MS analysis was performed on a Surveyor HPLC system (Thermo Finnigan) equipped with a C18 column (Gemini, 4.6 mm x 50 mm, 5 µm particle size, Phenomenex), coupled to a LCQ Advantage Max (Thermo Finnigan) ion-trap spectrometer (ESI+). The applied buffers were H₂O, MeCN and 1% aqueous TFA. Reversed-phase HPLC purification was performed on an Agilent Technologies 1200 series instrument equipped with a HILIC column (VP 250/10 NUCLEODUR HILIC, 5 µm particle size, Macherey-Nagel). The applied buffers were: (A) 1% AcOH in H₂O, and (B) CH₃CN.

¹H NMR and ¹³C NMR spectra were recorded on a Bruker DPX-300 (300 and 75 MHz respectively), Bruker AV-400 (400 and 101 MHz respectively), a Bruker AV-I-500 (500 and 126 MHz respectively), a Brüker DMX-600 (600 and 151 MHz), or a Brüker AV-850 (850 and 214 MHz) spectrometer in the given solvent. Chemical shifts are given in ppm (δ) relative to the residual solvent peak or tetramethylsilane (0 ppm) as internal standard. Coupling constants are given in Hz. All given ¹³C-NMR spectra are proton decoupled. The following abbreviations are used to describe peak patterns when appropriate: s (singlet), d (doublet), t (triplet), q (quartet), m (multiplet), Ar (aromatic), C_{q} (quarternary carbon). 2D NMR experiments (COSY, HSQC) were carried out to assign protons and carbons of the new structures.

High resolution mass spectrometry (HRMS) analysis was performed with a LTQ Orbitrap mass spectrometer (Thermo Finnigan), equipped with an electronspray ion source in positive mode (source voltage 3.5kV, sheath gas flow 10 mL/min, capillary temperature 250 °C) with resolution R = 60000 at m/z 400 (mass range m/z = 150 - 2000) and dioctyl phthalate (m/z = 391.28428) as a "lock mass". The high resolution mass spectrometer was calibrated prior to measurements with a calibration mixture (Thermo Finnigan).

### Protein Crystallisation

HPSE - Well diffracting crystals of HPSE were obtained by the sitting-drop vapor-diffusion method at 20 °C using a well solution containing 0.1 M MES pH 5.5, 0.1 M MgCl₂, 17 % (w/v) PEG 3350, and a protein to well solution ratio of 200 nl: 500 nl. Crystals typically appeared after 1 week. Inhibitor ligand complexes were obtained by transferring HPSE crystals to drops of well solution supplemented with 25 % (v/v) ethylene glycol and 1-5 mM inhibitor. Crystals were incubated with ligand for ~2-4 h, then directly harvested and flash-cooled in LN2 for data collection.

AcGH79 - Well diffracting crystals of AcGH79 were obtained by the sitting-drop vapor-diffusion method at 20 °C using a well solution containing 0.5 M ammonium sulfate, 1 M lithium sulfate, 0.1 M trisodium citrate, and a protein to well solution ratio of 500 nl: 500 nl. Crystals typically appeared after 1 week. Inhibitor complexes were obtained by adding inhibitor at 1 mM concentration directly to crystallization droplets of pre-formed crystals. Crystals were incubated for 24 hours with the inhibitors, then cryo-protected using 2 M lithium sulfate, harvested and flash-cooled in LN2 for data collection.

BpHep - Well diffracting crystals of BpHep were obtained by the sitting-drop vapor-diffusion method at 20 °C using a well solution containing 0.1 M sodium citrate pH 5.0, 14% (w/v) PEG 6000, and a protein to well solution ratio of 300 nl: 500nl. Crystals typically appeared after 3 days. Inhibitor ligand complexes were obtained by transferring BpHep crystals to drops of well solution supplemented with 25 % (v/v) ethylene glycol and 1-5 mM inhibitor. Crystals were incubated with ligand for ~2-4 h, then directly harvested and flash-cooled in LN2 for data collection.

EcGUSB - Initial crystals of EcGUSB were obtained by the sitting-drop vapor-diffusion method at 20 °C using a well solution containing 0.1 M Bis-Tris propane pH 7.5, 20% (w/v) PEG 3350, 0.2 M NaNO₃. These initial crystals were used to prepare a microseed stock using Seed Beads (Hampton), which was then used to seed optimized crystals of EcGUSB in the same well conditions, at a protein to seed to well solution ratio of 500 nl: 200 nl: 1000 nL. Inhibitor ligand complexes were obtained by transferring EcGUSB crystals to drops of well solution supplemented with 25 % (v/v) ethylene glycol and 1-5 mM inhibitor. Crystals were incubated with ligand for ~2-4 h, then directly harvested and flash-cooled in LN2 for data collection.

### EXAMPLES

### Example 1: Synthesis of intermediates

### Intermediate 1: 2-Azido-2-deoxy-3,4-di-O-benzyl-6-O-tert-butyldiphenylsilyl-D-glucopyranoside

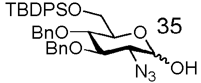

5-azido-3,4-bis(benzyloxy)-6-(phenylthio)tetrahydro-2H-pyran-2-yl)methoxy)(tert-butyl)diphenylsilane (*Lourenco and Ventura, 2016*) (3.58 g, 5.00 mmol) was dissolved in acetone/H₂O (50 ml, 9/1, v/v). The mixture was cooled to 0°C and NIS (2.25 g, 10.0 mmol) was added. The reaction was stirred for 4 hours. Upon completion Na₂S₂O₃ (aq. sat.) was added and the reaction turned colorless. The mixture was concentrated under reduced pressure, the residue was dissolved in EtOAc, washed with NaHCO₃ (aq. sat.) and brine, dried over MgSO₄, filtered and concentrated under reduced pressure. The product was obtained after chromatography (Et₂O/pentane, 1/19 -> 2/8, v/v) as a colorless oil (2.13 g, 3.41 mmol, 68%). ¹H NMR (400 MHz, CDCl₃) δ 7.79 - 7.59 (m, 7H), 7.42 - 7.24 (m, 23H), 7.23 - 7.12 (m, 3H), 5.23 (t, *J* = 3.4 Hz, 1H), 4.99 - 4.78 (m, 5H), 4.74 (d, *J* = 10.9 Hz, 1H), 4.66 (d, *J* = 10.8 Hz, 1H), 4.43 (dd, *J* = 7.8, 5.1 Hz, 1H), 4.15 - 3.78 (m, 7H), 3.78 - 3.66 (m, 1H), 3.50 - 3.26 (m, 4H), 1.06 (d, *J* = 1.6 Hz, 14H). ¹³C NMR (101 MHz, CDCl₃) δ 138.1, 137.9, 137.9, 137.8, 136.0, 135.9, 135.7, 135.7, 133.6, 133.6, 133.1, 133.0, 129.8, 129.8, 129.8, 128.6, 128.6, 128.5, 128.4, 128.3, 128.1, 127.9, 127.9, 127.8, 127.8, 127.7, 127.7, 127.7, 96.1, 92.1, 83.1, 80.2, 78.3, 77.5, 77.2, 76.8, 76.1, 75.8, 75.8, 75.2, 71.9, 67.6, 64.2, 62.8, 62.5, 60.6, 26.9, 19.4, 19.4. HRMS (ESI) m/z: [M+Na]⁺ calculated for C₃₆H₄₁N₃O₅Na 646.2708, found 646.2702

### Intermediate 2: 2-Azido-2-deoxy-3,4-di-O-benzyl-6-O-tert-butyldiphenylsilyl-D-glucopyranosyl N-phenyltrifluoroacetimidate

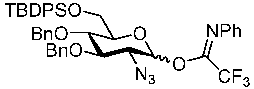

Intermediate 1 (2.10 g, 3.37 mmol) was dissolved in DCM (16.8 ml). 2,2,2-trifluoro-*N-*phenyltrifluoroacetimidoyl chloride (0.15 ml, 0.92 mmol, 1.5 eq) and Cs₂CO₃ (1.32 g, 4.04 mmol) were added and the reaction was stirred overnight at room temperature. The reaction was filtered over celite and concentrated in vacuo. Purification by flash silica column chromatography (EtOAc/pentane, 1/40 -> 1/19, v/v) yielded the product as a colorless oil (2.41 g, 3.03 mmol, 90%). ¹H NMR (400 MHz, CDCl₃) δ 7.80 - 7.58 (m, 4H), 7.51 - 7.18 (m, 19H), 7.16 - 7.00 (m, 1H), 6.81 (d, *J* = 7.8 Hz, 1H), 6.76 - 6.71 (m, 1H), 5.00 - 4.82 (m, 3H), 4.76 (dd, *J* = 10.7, 3.9 Hz, 1H), 4.08 - 3.79 (m, 4H), 3.65 (d, *J* = 10.2 Hz, 1H), 1.07 (d, *J* = 1.0 Hz, 9H). ¹³C NMR (101 MHz, CDCl₃) δ 143.5, 143.3, 137.9, 137.8, 137.7, 137.7, 136.1, 136.0, 135.7, 135.6, 133.6, 133.5, 132.9, 132.8, 130.0, 129.9, 129.9, 129.5, 128.9, 128.8, 128.7, 128.7, 128.7, 128.4, 128.3, 128.2, 128.1, 128.1, 128.0, 127.9, 127.9, 127.8, 126.5, 124.6, 124.5, 120.6, 119.5, 82.9, 80.4, 77.6, 77.5, 77.2, 77.1, 76.8, 76.5, 76.0, 75.5, 75.5, 74.6, 65.6, 63.2, 62.1, 27.0, 26.9, 19.5. HRMS (ESI) m/z: [M+Na]⁺ calculated for C₄₄H₄₅F₃N₄O₅SiNa 817.3004 found 817.3005

### Intermediate 3: 2,3-di-O-benzyl-6-O-benzoyl-cyclophellitol alkene

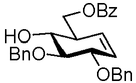

5,6-bis(benzyloxy)-2-(hydroxymethyl)cyclohex-3-en-1-ol (1.36 g, 4.00 mmol) was dissolved in DCM (20 ml). Et₃N (2.79 ml, 20.0 mmol) and benzoyl chloride (0.56 ml, 4.80 mmol) were added at -50 °C and the reaction was slowly warmed to room temperature overnight. H₂O and DCM were added and the layers were separated. The organic phase was washed with NaHCO₃ (aq. sat.) and brine, dried over MgSO₄, filtered and concentrated under reduced pressure. Column chromatography (EtOAc/pentane, 1/19 -> 1/9, v/v) afforded the product (1.45 g, 3.27 mmol, 82%). ¹H NMR (400 MHz, CDCl₃) δ 8.00 (dd, *J* = 8.3, 1.4 Hz, 2H), 7.60 - 7.49 (m, 1H), 7.49 - 7.27 (m, 12H), 5.80 (dt, *J* = 10.3, 2.2 Hz, 1H), 5.70 (dt, *J* = 10.3, 2.0 Hz, 1H), 5.04 (d, *J* = 11.2 Hz, 1H), 4.80 - 4.61 (m, 3H), 4.58 (dd, *J* = 10.9, 3.7 Hz, 1H), 4.44 (dd, *J* = 11.0, 5.5 Hz, 1H), 4.27 - 4.20 (m, 1H), 3.78 - 3.64 (m, 2H), 2.75 - 2.65 (m, 1H). ¹³C NMR (101 MHz, CDCl₃) δ 166.7, 138.9, 138.5, 138.2, 133.2, 129.7, 128.7, 128.6, 128.5, 128.1, 128.1, 128.0, 127.9, 127.8, 127.4, 83.8, 80.4, 75.2, 71.6, 69.9, 64.4, 43.4. HRMS (ESI) m/z: [M+Na]⁺ calculated for C₂₈H₂₈NaO₅ 467.1829, found 467.1832.

### Intermediate 4: 2,3-di-O-benzyl-4-O-(2-Azido-2-deoxy-3,4-di-O-benzyl-6-O-tert-butyldiphenylsilyl-D-glucopyranosyl)-6-O-benzoyl-cyclophellitol alkene

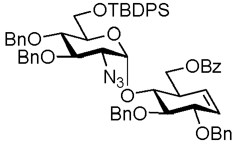

Intermediate 2 (2.41 g, 3.03 mmol) and intermediate 3 (1.04 g, 2.33 mmol) were co-evaporated with anhydrous toluene (3x). the mixture was dissolved in DCM (15 ml) and activated MS 3 Å were added. The mixture was stirred at room temperature overnight. The mixture was cooled to -78 °C. TfOH (0.04 ml, 0.45 mmol) was added and the reaction was warmed to -30°C in 70 minutes and kept at this temperature for 60 minutes. The reaction was quenched with NaHCO₃ (aq. sat.), diluted with Et₂O and washed with H₂O, NaHCO₃ (aq. sat.), and brine, dried over MgSO₄ and filtered. Volatiles were removed under reduced pressure and column chromatography (EtOAc/pentane, 1/19 -> 1/9, v/v) afforded the product (2.02 g, 1.92 mmol, 83%). ¹H NMR (400 MHz, CDCl₃) δ 7.84 - 7.80 (m, 2H), 7.61 - 7.51 (m, 4H), 7.45 - 7.13 (m, 29H), 5.83 - 5.77 (m, 1H), 5.71 (d, *J* = 3.9 Hz, 1H), 5.64 - 5.57 (m, 1H), 5.08 (d, *J* = 10.7 Hz, 1H), 4.98 (d, *J* = 10.7 Hz, 1H), 4.89 - 4.81 (m, 3H), 4.73 - 4.62 (m, 3H), 4.47 (dd, *J* = 11.1, 3.2 Hz, 1H), 4.33 - 4.24 (m, 2H), 4.05 - 3.91 (m, 3H), 3.91 - 3.81 (m, 2H), 3.80 - 3.70 (m, 2H), 3.29 (dd, *J* = 10.4, 3.9 Hz, 1H), 2.78 (br, 1H), 0.98 (s, 9H). ¹³C NMR (101 MHz, CDCl₃) δ 166.2, 138.9, 138.3, 138.2, 138.1, 135.9, 135.7, 133.6, 133.2, 133.1, 129.7, 129.7, 129.6, 128.7, 128.6, 128.6, 128.6, 128.4, 128.3, 128.0, 128.0, 127.9, 127.8, 127.8, 127.8, 127.6, 127.6, 98.2, 84.4, 81.0, 80.2, 78.1, 75.7, 75.2, 74.8, 74.4, 72.8, 71.8, 64.5, 63.6, 62.2, 43.3, 27.0, 19.4. HRMS (ESI) m/z: [M+Na]⁺ calculated for C₆₄H₆₇N₃O₉SiNa 1072.4539, found 1072.4573.

### Intermediate 5: 2,3-di-O-benzyl-4-O-(2-N-acetyl-2-deoxy-3,4-di-O-benzyl-6-O-tert-butyldiphenylsilyl-D-glucopyranosyl)-6-O-benzoyl-cyclophellitol alkene

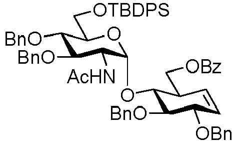

Intermediate 4 (0.22 g, 0.21 mmol) was dissolved in THF/H₂O (2.6 ml, 17/3, v/v), PPh₃ (0.14 g, 0.50 mmol) and pyridine (5 µl, 0.06 mmol) were added. The solution was stirred at 50°C for 3 hours. The reaction was concentrated under reduced pressure, dissolved in DCM and dried over MgSO₄. The solids were removed by filtration and the filtrate was concentrated under reduced pressure. The residue was co-evaporated with toluene and dissolved in DCM (2.0 ml). Acetic anhydride (0.30 ml, 3.1 mmol) and pyridine (0.25 ml, 3.1 mmol) were added and the reaction was stirred overnight. The reaction was cooled to 0 °C and quenched with H₂O. The layers were separated and the organic phase was washed with CuSO₄ (aq. sat), NaHCO₃ (aq. sat) and brine, dried over MgSO₄, filtered and concentrated under reduced pressure. Column chromatography (EtOAc/pentane, 1/9 -> 2/8, v/v) afforded the product (0.20 g, 0.19 mmol, 89%). ¹H NMR (400 MHz, CDCl₃) δ 7.97 - 7.92 (m, 2H), 7.72 - 7.63 (m, 4H), 7.42 - 7.13 (m, 29H), 6.62 (d, *J =* 9.7 Hz, 1H), 5.86 - 5.81 (m, 1H), 5.73 - 5.66 (m, 1H), 4.98 - 4.77 (m, 5H), 4.72 - 4.55 (m, 5H), 4.52 - 4.47 (m, 1H), 4.38 (td, *J =* 10.0, 3.5 Hz, 1H), 4.23 - 4.16 (m, 1H, H2), 3.99 - 3.71 (m, 7H), 2.70 - 2.63 (m, 1H), 1.46 (s, 3H), 1.03 (s, 9H). ¹³C NMR (101 MHz, CDCl₃) δ 170.4, 166.2, 138.6, 138.3, 137.9, 137.7, 136.0, 135.7, 133.7, 133.2, 133.0, 130.0, 129.8, 129.7, 129.7, 128.8, 128.7, 128.6, 128.6, 128.6, 128.5, 128.4, 128.3, 128.3, 128.2, 128.2, 128.1, 128.0, 127.9, 127.8, 127.8, 127.7, 127.7, 126.8, 100.5, 81.8, 81.4, 79.0, 77.8, 77.4, 75.4, 75.3, 74.9, 73.5, 71.6, 64.1, 62.5, 53.5, 44.0, 26.9, 22.9, 19.4. HRMS (ESI) m/z: [M+Na]⁺ calculated for C₆₆H₇₁NO₁₀SiNa 1088.4739, found 1088.4741

### Intermediate 6: 2,3-di-O-benzyl-4-O-(2-N-acetyl-2-deoxy-3,4-di-O-benzyl-6-O-tert-butyldiphenylsilyl-D-glucopyranosyl)-cyclophellitol alkene

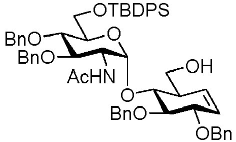

Intermediate 5 (0.52 g, 0.49 mmol) was dissolved in MeOH/DCM (13.5 ml, 4.4/1, v/v). NaOMe (5.4 M in MeOH, 0.03 ml, 0.15 mmol) was added and the reaction was stirred overnight. The reaction was quenched with NH₄Cl. Volatiles were removed under reduced pressure. The residue was dissolved in EtOAc and washed with H₂O and brine, dried over MgSO₄, filtered and concentrated under reduced pressure. Column chromatography (EtOAc/pentane, 1/9 -> 2/3, v/v) yielded the product (0.46 g, 0.47 mmol, 97%). ¹H NMR (400 MHz, CDCl₃) δ 7.70 (dd, *J =* 16.1, 7.0 Hz, 4H), 7.46 - 7.11 (m, 26H), 6.70 (d, *J =* 9.6 Hz, 1H), 5.90 - 5.82 (m, 1H), 5.67 - 5.60 (m, 1H), 4.95 - 4.89 (m, 2H), 4.83 (d, *J =* 10.6 Hz, 1H), 4.75 - 4.50 (m, 6H), 4.39 - 4.30 (m, 1H), 4.21 - 4.15 (m, 1H), 3.99 - 3.56 (m, 9H), 2.41 - 2.34 (m, 1H), 1.47 (s, 3H), 1.05 (s, 9H). ¹³C NMR (101 MHz, CDCl₃) δ 170.4, 138.5, 138.1, 137.9, 137.5, 136.0, 135.7, 133.6, 133.1, 129.8, 129.4, 128.7, 128.6, 128.6, 128.4, 128.3, 128.3, 128.2, 128.0, 127.9, 127.8, 127.8, 127.7, 127.6, 126.7, 100.2, 81.6, 81.2, 78.8, 78.0, 77.8, 75.2, 74.8, 73.5, 71.5, 62.8, 62.5, 53.3, 46.1, 26.9, 22.8, 19.3. HRMS (ESI) m/z: [M+Na]⁺ calculated for C₅₉H₆₇NO₉SiNa 984.4477, found 984.4506

### Intermediate 7: 2,3-di-O-benzyl-4-O-(2-N-acetyl-2-deoxy-3,4-di-O-benzyl-6-O-tert-butyldiphenylsilyl-D-glucopyranosyl)-6-O-tert-butyloxycarbonyl-cyclophellitol alkene

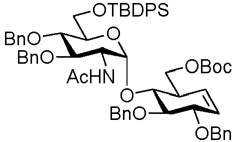

Intermediate 6 (0.72 g, 0.75 mmol) was co-evaporated three times with toluene and dissolved in dry THF (5.4 ml, 0.14 M). Boc₂O (0.19 ml, 0.81 mmol) and DMAP (9 mg, 75 µmol) were added and the reaction was stirred at room temperature. After 1h the reaction was cooled to 0°C and quenched with H₂O. The reaction mixture was further diluted with H₂O and extracted with Et₂O. The organic layer was washed with NH₄Cl (aq. sat.), NaHCO₃ (aq. sat.) and brine, dried over MgSO₄, filtered and concentrated in vacuo. Flash column chromatography (EtOAc/PE, 1/9 -> 2/3) yielded the product (0.60 g, 0.57 mmol, 76%). ¹H NMR (300 MHz, CDCl₃) δ 7.78 - 7.63 (m, 4H), 7.47 - 7.11 (m, 26H), 6.75 (d, *J =* 9.7 Hz, 1H), 5.82 (dt, *J =* 10.2, 2.5 Hz, 1H), 5.62 (ddd, *J* = 10.3, 2.6, 1.6 Hz, 1H), 4.99 - 4.84 (m, 3H), 4.80 (d, *J =* 11.1 Hz, 1H), 4.75 - 4.51 (m, 5H), 4.45 - 4.27 (m, 3H), 4.23 (dt, *J* = 6.3, 2.3 Hz, 1H), 4.04 - 3.68 (m, 7H), 2.52 (h, *J =* 3.1 Hz, 1H), 1.45 (s, 3H), 1.42 (s, 9H), 1.06 (s, 9H). ¹³C NMR (75 MHz, CDCl₃) δ 170.3, 153.4, 138.6, 138.3, 137.8, 137.6, 135.9, 135.6, 133.7, 133.0, 129.7, 128.6, 128.6, 128.5, 128.5, 128.4, 128.2, 128.2, 128.0, 128.0, 128.0, 127.9, 127.8, 127.7, 127.7, 127.7, 127.6, 127.5, 126.8, 100.5, 82.1, 81.8, 81.7, 79.3, 77.7, 75.2, 75.1, 74.9, 73.4, 71.5, 66.1, 62.5, 53.5, 43.9, 27.7, 26.9, 22.7, 19.3. HRMS (ESI) m/z: [M+H]⁺ calculated for C₆₄H₇₆NO₁₁Si 1062.5182, found 1062.5182

### Intermediate 8: 1-iodo-2,3-di-O-benzyl-4-O-(2-N-acetyl-2-deoxy-3,4-di-O-benzyl-6-O-tert-butyldiphenylsilyl-D-glucopyranosyl)-6,7-O-carbonyl-cyclophellitol alkane

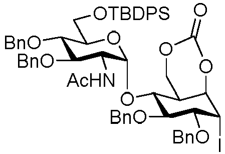

To a solution of intermediate 7 (0.22 g, 0.21 mmol) in DCM/AcOH (1.1 ml, 0.2 M, 2/1, v/v), NIS (0.09 g, 0.41 mmol) was added. After 22 hours the reaction mixture was diluted with Et₂O and quenched with Et₃N. The organic layer was washed with NH₄Cl (aq. sat.), NaHCO₃ (aq. sat.), Na₂S₂O₃ (aq. sat.) and brine, dried over MgSO₄, filtered and concentrated in vacuo. Flash column chromatography (EtOAc/PE, 1/9 -> 2/3) yielded the product (0.21 g, 0.19 mmol, 91%). ¹H NMR (400 MHz, CDCl₃) δ 7.74 - 7.62 (m, 4H), 7.48 - 7.11 (m, 26H), 6.43 (d, *J =* 9.7 Hz, 1H), 5.12 - 4.99 (m, 2H), 4.91 - 4.83 (m, 4H), 4.72 - 4.63 (m, 4H), 4.60 (d, *J =* 10.5 Hz, 1H), 4.55 (d, *J* = 11.0 Hz, 1H), 4.41 (td, *J =* 10.0, 3.3 Hz, 1H), 4.06 (t, *J =* 6.7 Hz, 1H), 4.01 - 3.82 (m, 5H), 3.82 - 3.72 (m, 2H), 3.13 (dd, *J* = 9.3, 4.0 Hz, 1H), 2.62 (dd, *J =* 10.4, 2.6 Hz, 1H), 1.28 (s, 3H), 1.06 (s, 9H). ¹³C NMR (101 MHz, CDCl₃) δ 170.4, 146.8, 138.4, 138.0, 137.0, 136.8, 135.8, 135.5, 133.6, 132.8, 129.9, 129.8, 128.7, 128.6, 128.6, 128.5, 128.5, 128.4, 128.3, 128.2, 128.1, 128.0, 127.9, 127.8, 127.7, 127.6, 101.4, 82.1, 81.6, 81.4, 77.6, 77.3, 76.7, 76.2, 75.3, 75.0, 74.0, 72.2, 68.3, 62.6, 60.4, 53.3, 35.1, 30.4, 26.9, 22.5, 19.4. HRMS (ESI) m/z: [M+H]⁺ calculated for C₆₀H₆₇INO₁₁Si 1132.3523, found 1132.3525

### Intermediate 9: 2,3-di-O-benzyl-4-O-(2-N-acetyl-2-deoxy-3,4-di-O-benzyl-6-O-tert-butyldiphenylsilyl-D-glucopyranosyl)-cyclophellitol

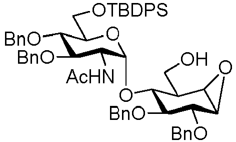

Intermediate 8 (0.12 g, 0.11 mmol) was dissolved in MeOH/DCM (1.9 ml, 0.06 M, 1.6/1, v/v), NaOMe (4.37 M in MeOH, 0.09 ml, 0.39 mmol) was added and the reaction was stirred for 20 hours. Et₃N·HCl was added and the solution was concentrated in vacuo. EtOAc was added and the solution was washed with H₂O and brine, dried over MgSO₄, filtered and concentrated in vacuo. The product was used and analyzed without further purification. (0.12 g, quantitative). ¹H NMR (400 MHz, CDCl₃) δ 7.76 - 7.66 (m, 4H), 7.47 - 7.15 (m, 26H), 6.83 (d, *J* = 9.7 Hz, 1H), 4.95 (d, *J* = 11.0 Hz, 1H), 4.88 (d, *J =* 3.3 Hz, 1H), 4.85 - 4.77 (m, 2H), 4.73 - 4.63 (m, 4H), 4.47 (d, *J* = 11.2 Hz, 1H), 4.35 (td, *J =* 9.8, 3.3 Hz, 1H), 4.12 - 4.05 (m, 1H), 4.01 - 3.93 (m, 2H), 3.93 - 3.78 (m, 4H), 3.72 - 3.52 (m, 3H), 3.34 - 3.31 (m, 1H), 3.15 (d, *J =* 3.7 Hz, 1H), 2.03 - 1.97 (m, 1H), 1.44 (s, 3H), 1.06 (s, 9H). ¹³C NMR (101 MHz, CDCl₃) δ 170.3, 138.6, 138.2, 137.5, 137.2, 136.1, 135.7, 133.7, 133.1, 129.8, 128.8, 128.7, 128.6, 128.4, 128.3, 128.2, 128.2, 127.9, 127.9, 127.7, 127.7, 127.6, 100.8, 83.1, 81.5, 80.1, 77.6, 75.9, 75.6, 75.1, 74.9, 73.7, 72.8, 62.7, 61.8, 56.6, 53.3, 52.2, 44.5, 26.9, 22.8, 19.4. HRMS (ESI) m/z: [M+Na]⁺ calculated for C₅₉H₆₇NO₁₀SiNa 1000.4426, found 1000.4452.

### Intermediate 10: 2,3-di-O-benzyl-4-O-(2-N-acetyl-2-deoxy-3,4-di-O-benzyl-6-O-tert-butyldiphenylsilyl-D-glucopyranosyl)-glucurono-cyclophellitol

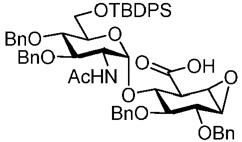

Intermedaite 9 (0.13 g, 0.13 mmol) was dissolved in *t*-BuOH/DCM/H₂O (4.9 ml, 5/4/1, v/v). and cooled down to 0°C. TEMPO (4 mg, 0.03 mmol) and BAIB (0.10 g, 0.32 mmol) were added and the reaction was stirred 23 hours. The reaction was diluted with DCM and quenched with Na₂S₂O₃ (aq. sat.). The layers were separated and the water layer was acidified with AcOH. The water layer was extracted with DCM (4x). The combined organic layers were dried over MgSO₄, filtered and concentrated under reduced pressure. Column chromatography (EtOAc/pentane 1% AcOH, 1/4 -> 1/1, v/v) afforded the product (0.10 mg, 0.10 mmol, 80%). ¹H NMR (400 MHz, CDCl₃) δ 7.76 (d, *J =* 3.4 Hz, 2H), 7.68 (d, *J =* 6.8 Hz, 2H), 7.50 - 7.13 (m, 26H), 6.04 (d, *J =* 9.6 Hz, 1H), 4.97 - 4.74 (m, 6H), 4.69 - 4.53 (m, 3H), 4.30 - 4.22 (m, 1H), 4.08 (d, *J =* 9.7 Hz, 1H), 3.99 (t, *J =* 9.5 Hz, 1H), 3.95 - 3.87 (m, 2H), 3.80 - 3.64 (m, 3H), 3.48 - 3.42 (m, 1H), 3.29 (s, 1H), 3.17 (d, *J =* 3.5 Hz, 1H), 2.79 - 2.73 (m, 1H), 1.25 (s, 3H), 1.05 (s, 9H). ¹³C NMR (101 MHz, CDCl₃) δ 170.6, 170.1, 138.6, 138.4, 137.6, 137.1, 136.6, 135.8, 133.7, 133.6, 130.0, 129.8, 128.8, 128.8, 128.7, 128.5, 128.4, 128.4, 128.2, 128.2, 128.0, 127.9, 127.8, 127.7, 100.5, 81.8, 81.4, 79.1, 77.7, 75.9, 75.6, 75.4, 75.4, 73.2, 72.9, 62.2, 54.1, 53.7, 53.4, 48.7, 27.0, 22.5, 19.3. HRMS (ESI) m/z: [M+Na]⁺ calculated for C₅₉H₆₅NO₁₁SiNa 1014.4219, found 1014.4223

### Intermediate 11: 2,3-di-O-benzyl-4-O-(2-N-acetyl-2-deoxy-3,4-di-O-benzyl-D-glucopyranosyl)-glucurono-cyclophellitol

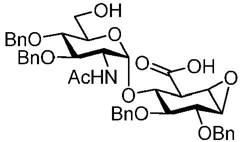

Intermediate 10 (89 mg, 0.090 mmol) was dissolved in anhydrous THF (1.9 ml). Et₃N·3HF (0.046 ml ,0.28 mmol) was added and the reaction was stirred for 20 hours. More Et₃N·3HF (0.030 ml, 0.18 mmol) was added and the reaction was stirred 23 hours. The reaction was diluted with DCM and washed with water. The water layer was extracted four times with DCM. The combined organic layers were dried over MgSO₄, filtered and concentrated under reduced pressure. Column chromatography (EtOAc/pentane 1% AcOH, 1/9 -> 1/4, v/v) yielded the product (62 mg, 0.082 mmol, 87%). ¹H NMR (400 MHz, CDCl₃) δ 7.38 - 7.15 (m, 20H), 6.35 (d, *J* = 9.4 Hz, 1H), 4.96 (d, *J =* 10.7 Hz, 1H), 4.88 - 4.74 (m, 4H), 4.74 - 4.63 (m, 2H), 4.63 - 4.49 (m, 2H), 4.39 - 4.28 (m, 1H), 4.02 - 3.89 (m, 3H), 3.87 - 3.77 (m, 3H), 3.68 - 3.61 (m, 1H), 3.49 (t, *J =* 9.3 Hz, 1H), 3.44 - 3.39 (m, 1H), 3.22 - 3.16 (m, 1H), 2.88 (d, *J* = 8.3 Hz, 1H), 1.19 (s, 3H). ¹³C NMR (101 MHz, CDCl₃) δ 173.2, 172.3, 138.6, 138.3, 137.7, 137.1, 129.1, 128.8, 128.8, 128.5, 128.4, 128.4, 128.3, 128.3, 128.3, 128.3, 128.2, 127.9, 127.6, 127.6, 125.4, 100.5, 81.8, 81.5, 79.3, 77.6, 76.7, 75.8, 75.2, 75.1, 73.3, 72.8, 61.0, 54.4, 53.6, 49.4, 22.7. HRMS (ESI) m/z: [M+Na]⁺ calculated for C₄₃H₄₇NO₁₁Na 776.3041 found 776.3058

### Intermediate 12: 2,3-di-O-benzyl-4-O-(2-N-acetyl-2-deoxy-3,4-di-O-benzyl-6-O-sulfo-D-glucopyranosyl)-glucurono-cyclophellitol

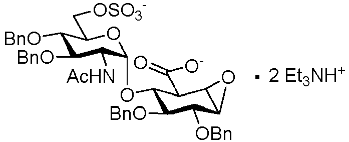

Intermediate 11 (0.062 g, 0.082 mmol) was dissolved in dry DMF (1.1 ml, 0.08 M). SO₃·Et₃N (0.031 g, 0.17 mmol) was added and the mixture was stirred for 4 hours. Et₃N (0.07 ml, 0.5 mmol) was added, the reaction mixture was diluted with MeOH (2 ml) and concentrated under reduced pressure. The crude mixture was analyzed and used without purification. ¹H NMR (400 MHz, MeOD) δ 7.45 - 7.17 (m, 20H), 5.42 (d, *J* = 3.9 Hz, 1H), 4.90 - 4.59 (m, 8H), 4.41 (d, *J =* 10.6 Hz, 1H), 4.24 (d, *J =* 10.6 Hz, 1H), 4.17 (dd, *J =* 10.0, 3.9 Hz, 1H), 4.08 (t, *J =* 9.7 Hz, 1H), 3.91 - 3.83 (m, 2H), 3.78 - 3.63 (m, 3H), 3.52 - 3.47 (m, 1H), 3,33 (under solvent peak, 1H) 3.20 (dt, *J =* 8.3, 6.7 Hz, 19H, H5/Et₃N), 1.74 (s, 3H), 1.31 (td, *J =* 7.3, 1.4 Hz, 29H, Et₃N). ¹³C NMR (101 MHz, MeOD) δ 174.2, 172.9, 140.1, 139.8, 139.6, 139.0, 129.5, 129.5, 129.4, 129.3, 129.2, 129.0, 128.7, 128.6, 128.5, 128.5, 99.3, 84.8, 82.0, 81.4, 79.0, 76.2, 75.9, 75.2, 73.3, 72.8, 71.5, 66.8, 55.6, 54.2, 53.8, 49.4, 47.9 (Et₃N), 22.9, 9.3 (Et₃N). HRMS (ESI) m/z: [M+H]⁺ calculated for C₄₃H₄₇NO₁₄S 834.2790, found 834.2785.

### Intermediate 13: 2,3-di-O-benzyl-4-O-(2-Azido-2-deoxy-3,4-di-O-benzyl-6-O-tert-butyldiphenylsilyl-D-glucopyranosyl)-cyclophellitol alkene

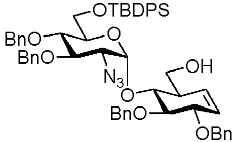

Intermediate 4 (0.98 g, 0.93 mmol, 1 eq) was dissolved in MeOH/DCM (1/1, v/v, 18 ml). NaOMe (25 wt%, 0.1 ml, 0.44 mmol) was added and the reaction mixture was stirred overnight. NH₄Cl was added and volatiles were removed under reduced pressure. The crude product was purified using column chromatography (EtOAc/pentane, 1/19 -> 3/17, v/v) this yielded the product as a colourless oil. (0.91 g, quant.). ¹H NMR (400 MHz, CDCl₃) δ 7.73 - 7.61 (m, 4H), 7.44 - 7.21 (m, 24H), 7.17 - 7.10 (m, 2H), 5.80 (dt, *J =* 10.2, 2.4 Hz, 1H), 5.65 (d, *J =* 4.0 Hz, 1H), 5.59 (dt, *J =* 10.2, 2.2 Hz, 1H), 5.06 (d, *J =* 10.9 Hz, 1H), 4.95 (d, *J =* 10.9 Hz, 1H), 4.89 - 4.79 (m, 3H), 4.71 - 4.57 (m, 3H), 4.23 (m, 1H), 4.02 - 3.89 (m, 3H), 3.88 - 3.80 (m, 3H), 3.76 - 3.57 (m, 3H), 3.29 (dd, *J =* 10.3, 4.0 Hz, 1H), 2.54 - 2.44 (m, 1H), 1.04 (s, 9H). ¹³C NMR (101 MHz, CDCl₃) δ 139.0, 138.3, 138.0, 136.0, 135.8, 133.4, 133.1, 129.9, 128.9, 128.6, 128.6, 128.6, 128.5, 128.5, 128.3, 128.1, 128.0, 127.9, 127.8, 127.7, 127.5, 98.2, 84.2, 80.9, 80.3, 78.4, 75.7, 75.3, 74.6, 74.4, 72.9, 71.7, 63.6, 63.0, 62.6, 45.5, 27.0, 19.4. HRMS (ESI) m/z: [M+Na]⁺ calculated for C₅₇H₆₃N₃O₈SiNa 968.4277, found 968.4274.

### Intermediate 14: 2,3-di-O-benzyl-4-O-(2-Azido-2-deoxy-3,4-di-O-benzyl-6-O-tert-butyldiphenylsilyl-D-glucopyranosyl)-6-O-tert-butyloxycarbonyl-cyclophellitol alkene

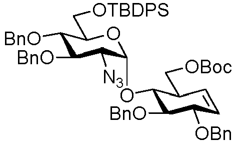

Intermediate 13 (0.92 g, 0.93 mmol) was co-evaporated with toluene and subsequently dissolved in THF (9.3 ml, 0.1 M). DMAP (91 mg, 0.74 mmol) and Boc₂O (0.41 g, 1.87 mmol) were added. The reaction was stirred at room temperature for 3 hours. Water was added and the mixture was stirred for 15 minutes. The mixture was extracted three times with Et₂O, the combined organic layers were washed with NH₄Cl (aq. sat.), NaHCO₃ (aq. sat.) and brine, dried over MgSO₄, filtered and concentrated under reduced pressure. The product was obtained after column chromatography (Et₂O/pentane, 0/1 -> 1/9, v/v) as a colorless oil. (0.80 g, 82%). ¹H NMR (500 MHz, CDCl₃) δ 7.73 - 7.59 (m, 4H), 7.44 - 7.12 (m, 26H), 5.76 (dt, *J =* 10.2, 2.4 Hz, 1H), 5.66 (d, *J =* 3.9 Hz, 1H), 5.57 (dt, *J =* 10.2, 2.3 Hz, 1H), 5.05 (d, *J =* 10.9 Hz, 1H), 4.94 (d, *J* = 10.9 Hz, 1H), 4.91 - 4.83 (m, 3H), 4.74 (d, *J =* 10.9 Hz, 1H), 4.70 - 4.60 (m, 2H), 4.26 - 4.16 (m, 2H), 4.03 - 3.94 (m, 3H), 3.93 - 3.81 (m, 4H), 3.75 - 3.69 (m, 1H), 3.28 (dd, *J =* 10.4, 3.9 Hz, 1H), 2.69 - 2.61 (m, 1H), 1.25 (s, 9H), 1.03 (s, 9H). ¹³C NMR (126 MHz, CDCl₃) δ 153.5, 139.0, 138.3, 138.1, 136.0, 135.7, 133.8, 133.2, 129.7, 129.7, 128.6, 128.6, 128.5, 128.5, 128.4, 128.0, 128.0, 127.9, 127.8, 127.7, 127.6, 127.5, 127.5, 98.0, 84.3, 82.1, 80.7, 80.3, 78.2, 75.7, 75.1, 74.6, 74.1, 72.7, 71.8, 66.6, 63.7, 62.3, 43.0, 27.7, 27.0, 19.4. HRMS (ESI) m/z: [M+Na]⁺ calculated for C₆₂H₇₁N₃O₁₀SiNa 1068.48009, found 1068.48007

### Intermediate 15: 1-iodo-2,3-di-O-benzyl-4-O-(2-azido-2-deoxy-3,4-di-O-benzyl-6-O-tert-butyldiphenylsilyl-D-glucopyranosyl)-6,7-O-carbonyl-cyclophellitol alkane

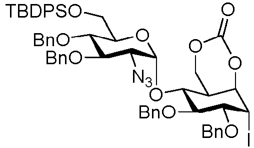

Intermediate 14 (0.80 g, 0.77 mmol) was dissolved in AcOH/DCM (1/2, v/v, 5.1 ml, 0.15 M). NIS (0.35 g , 1.54 mmol) was added and the reaction was stirred for 18 hours in the dark. The mixture was diluted with Et₂O, washed with Na₂S₂O₃ (aq.), NaHCO₃ (aq. sat.) and brine, dried over MgSO₄, filtered and concentrated under reduced pressure. The product was obtained after column chromatography (Et₂O/pentane, 1/4 -> 1/1, v/v) as a foam. (0,61 g, 71%). ¹H NMR (500 MHz, CDCl₃) δ = 7.64 (ddt, *J*=6.6, 2.8, 1.4, 4H), 7.45 - 7.22 (m, 24H), 7.21 - 7.11 (m, 2H), 5.50 (d, *J*=3.9, 1H), 5.03 (d, *J*=10.7, 1H), 4.93 - 4.83 (m, 5H), 4.72 - 4.63 (m, 2H), 4.63 - 4.54 (m, 3H), 4.27 (dd, *J*=11.6, 3.3, 1H), 4.00 - 3.93 (m, 2H), 3.94 - 3.82 (m, 4H), 3.63 (dt, *J*=9.7, 2.3, 1H), 3.36 (dd, *J*=10.2, 3.9, 1H), 3.13 (dd, *J*=8.8, 4.0, 1H), 2.79 - 2.73 (m, 1H), 1.06 (s, 9H). ¹³C NMR (126 MHz, CDCl₃) δ 146.7, 138.5, 137.9, 137.8, 137.2, 136.0, 135.8, 133.6, 132.9, 129.9, 129.9, 128.7, 128.5, 128.3, 128.3, 128.2, 128.2, 128.1, 128.1, 127.9, 127.8, 127.7, 127.4, 98.6, 83.4, 81.1, 80.2, 78.0, 77.2, 75.8, 75.4, 75.1, 74.0, 73.4, 72.5, 68.6, 63.6, 62.0, 34.0, 29.9, 27.1, 19.5. HRMS (ESI) m/z: [M+NH₄]⁺ calculated for C₅₈H₆₆IN₄O₁₀Si 1133.3587, found 1133.3593

### Intermediate 16: 2,3-di-O-benzyl-4-O-(2-azido-2-deoxy-3,4-di-O-benzyl-6-O-tert-butyldiphenylsilyl-D-glucopyranosyl)-cyclophellitol

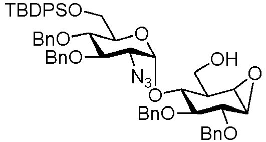

Intermediate 15 (0.606 g 0.543 mmol) was dissolved in DCM/MeOH (5.4 ml, 1/1, v/v). NaOMe (4.4M, 0.04 ml, 1.63 mmol) was added and the reaction was left to stir overnight. More NaOMe (4.4M, 0.04 ml, 1.63 mmol) was added and the reaction was stirred for 7 hours. Upon completion the reaction was quenched with NH₄Cl (35 mg, 0.65 mmol). The solvent was removed under reduced pressure and column chromatography (EtOAc/pentane, 3/17 -> 7/13, v/v) afforded the product as an oil (0.492 g, 0.511 mmol, 94%). ¹H NMR (400 MHz, CDCl₃) δ 7.71 - 7.62 (m, 4H), 7.44 - 7.24 (m, 24H), 7.18 - 7.12 (m, 2H), 5.61 (d, *J* = 4.0 Hz, 1H), 4.99 (d, *J* = 10.9 Hz, 1H), 4.91 - 4.76 (m, 5H), 4.69 - 4.61 (m, 2H), 3.97 - 3.78 (m, 6H), 3.78 - 3.65 (m, 4H), 3.41 - 3.36 (m, 1H), 3.24 (dd, *J =* 10.3, 4.0 Hz, 1H), 3.17 (d, *J =* 3.8 Hz, 1H), 2.23 - 2.16 (m, 1H), 1.05 (s, 9H). ¹³C NMR (101 MHz, CDCl₃) δ 138.9, 138.0, 137.5, 136.0, 135.8, 133.6, 133.2, 129.8, 128.7, 128.6, 128.6, 128.5, 128.3, 128.2, 128.1, 128.0, 128.0, 127.8, 127.7, 127.6, 127.5, 97.9, 84.8, 80.5, 80.3, 78.3, 75.7, 75.2, 74.6, 72.9, 70.4, 63.4, 62.7, 62.1, 56.0, 52.8, 43.2, 27.0, 19.4. HRMS (ESI) m/z: [M+NH₄]⁺ calculated for C₅₇H₆₇N₄O₉Si 979.4672, found 979.4669

### Intermediate 17: 2,3-di-O-benzyl-4-O-(2-azido-2-deoxy-3,4-di-O-benzyl-6-O-tert-butyldiphenylsilyl-D-glucopyranosyl)-glucurono-cyclophellitol

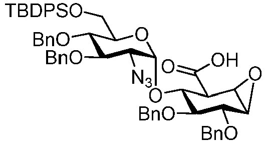

Intermediate 16 (0.492 g, 0.511 mmol) was dissolved in *t*-BuOH/ DCM/H₂O (3.5 ml, 6/4/1, v/v). TEMPO (0.016 g, 0.10 mmol) and BAIB (0.412 g, 1.28 mmol) were added and the reaction was stirred overnight. After TLC analysis (EtOAc/pentane, 1/3 + 0.5% Et₃N) showed full conversion of the starting material the reaction was diluted with DCM and water. The organic layer was washed with Na₂S₂O₃ and the layers were separated. The water layer was extracted four times with DCM. The combined organic layers were dried over MgSO₄, filtered and concentrated under reduced pressure. The product was obtained after column chromatography (EtOAc/pentane, 3/17 -> 1/4 + 0.5% AcOH, v/v) as an oil. (0.538 g, 0.525 mmol, quant.). ¹H NMR (400 MHz, CDCl₃) δ 7.72 - 7.63 (m, 4H), 7.43 - 7.25 (m, 24H), 7.21 - 7.16 (m, 2H), 5.38 (d, *J* = 3.7 Hz, 1H), 4.94 - 4.80 (m, 5H), 4.76 - 4.62 (m, 3H), 4.04 (t, *J =* 9.6 Hz, 1H), 4.00 - 3.89 (m, 2H), 3.86 - 3.79 (m, 3H), 3.64 (m, 1H), 3.54 (dd, *J =* 9.6, 8.2 Hz, 1H), 3.29 (dd, *J =* 10.2, 3.8 Hz, 1H), 3.21 - 3.18 (m, 1H), 3.09 (d, *J* = 3.6 Hz, 1H), 2.89 (dd, *J* = 9.4, 1.5 Hz, 1H), 1.04 (s, 9H). ¹³C NMR (101 MHz, CDCl₃) δ 172.2, 138.7, 138.2, 137.9, 137.4, 136.2, 135.8, 133.6, 133.3, 129.9, 129.8, 128.7, 128.6, 128.6, 128.5, 128.4, 128.2, 128.1, 128.1, 127.9, 127.9, 127.9, 127.8, 127.7, 98.4, 83.0, 80.3, 79.4, 78.1, 75.8, 75.2, 75.0, 73.6, 73.2, 72.5, 63.8, 62.3, 53.9, 53.5, 48.1, 27.0, 19.4. HRMS (ESI) m/z: [M+NH₄]⁺ calculated for C₅₇H₆₅N₄O₁₀Si 993.44645 found 993.44650

### Intermediate 18: 2,3-di-O-benzyl-4-O-(2-azido-2-deoxy-3,4-di-O-benzyl-D-glucopyranosyl)-glucurono-cyclophellitol

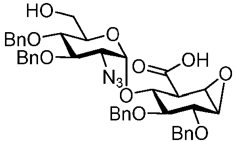

Intermediate 17 (0.525 g, 0.538 mmol) was dissolved in THF (2.7 ml), 3HF·Et₃N (0.44 ml, 2.69 mmol (8.07 mmol HF)) was added and the mixture was stirred for 45 hours. The reaction was poured over water and extracted four times with DCM. The combined organic layers were dried over MgSO₄ and concentrated under reduced pressure. Column chromatography (EtOAc/pentane 0.5% AcOH, 7/20 -> 9/20, v/v) afforded the product as a white solid. (0.340 g, 0.461 mmol, 86%). ¹H NMR (500 MHz, CDCl₃) δ 7.36 - 7.23 (m, 20H), 5.41 (d, *J* = 4.0 Hz, 1H), 4.90 (s, 2H), 4.87 - 4.79 (m, 3H), 4.74 (d, *J =* 11.3 Hz, 1H), 4.63 (dd, *J* = 14.7, 11.2 Hz, 2H), 4.12 (t, *J* = 9.7 Hz, 1H), 3.93 - 3.85 (m, 2H), 3.81 (dd, *J =* 11.8, 2.5 Hz, 1H), 3.72 (ddd, *J =* 10.1, 4.8, 2.5 Hz, 1H), 3.64 - 3.58 (m, 2H), 3.46 - 3.41 (m, 2H), 3.26 (dd, *J =* 10.3, 3.9 Hz, 1H), 3.20 (d, *J* = 3.7 Hz, 1H), 3.04 (dd, *J* = 9.5, 2.1 Hz, 1H). ¹³C NMR (126 MHz, CDCl₃) δ 173.8, 138.8, 137.8, 137.7, 137.4, 128.7, 128.7, 128.6, 128.4, 128.2, 128.2, 128.1, 128.1, 127.5, 127.4, 98.7, 83.0, 80.3, 79.6, 78.4, 75.6, 75.2, 74.9, 74.3, 73.2, 72.1, 63.6, 61.9, 54.3, 53.7, 48.4. HRMS (ESI) m/z: [M+NH₄]⁺ calculated for C₄₁H₄₇N₄O₁₀ 755.3287 found 755.3284.

### Intermediate 19: 2,3-di-O-benzyl-4-O-(2-amino-2-deoxy-3,4-di-O-benzyl-D-glucopyranosyl)-glucurono-cyclophellitol

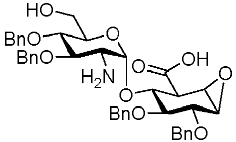

Intermediate 18 (50 mg, 0.068 mmol) was dissolved in MeOH/toluene (2.5 ml, 4/1, v/v). Zn dust (132 mg, 2.02 mmol) and NH₄Cl (144 mg, 2.69 mmol) were added portion wise over the 2 hours. The reaction was stirred for another hour followed by filtration over silica with MeOH/toluene (1/1, v/v). This afforded the poorly soluble product which was analyzed and used without further purification. ¹H NMR (400 MHz, MeOD) δ 7.39 - 7.22 (m, 20H), 5.52 (d, *J* = 3.6 Hz, 1H), 4.79 (d, *J =* 11.3 Hz, 1H), 4.75 (d, *J =* 11.9 Hz, 1H), 4.69 (d, *J =* 9.6 Hz, 2H), 4.54 (d, *J* = 11.3 Hz, 1H), 4.21 (t, *J* = 9.7 Hz, 1H), 4.00 (d, *J =* 7.1 Hz, 1H), 3.95 - 3.84 (m, 3H), 3.84 - 3.74 (m, 2H), 3.64 (t, *J =* 9.0 Hz, 1H), 3.50 (dd, *J =* 4.1, 1.6 Hz, 1H), 3.28 (d, *J =* 3.7 Hz, 1H), 3.23 (dd, *J =* 10.1, 3.6 Hz, 1H), 3.10 (d, *J =* 9.7 Hz, 1H). HRMS (ESI) m/z: [M+H]⁺ calculated for C₄₁H₄₆NO₁₀ 712.3116 found 712.3112

### Intermediate 20: 2,3-di-O-benzyl-6-O-(4-methoxybenzyl)-cyclophellitol alkene

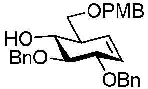

A solution of 5,6-bis(benzyloxy)-2-(hydroxymethyl)cyclohex-3-en-1-ol (1.00 g, 2.94 mmol) in anhydrous MeCN (15 mL, 0.2 M) was flushed with nitrogen and heated to 60 °C. PMB-Cl (0.59 ml, 4.41 mmol), KI (0.49 g, 2.94 mmol), K₂CO₃ (0.45 g, 3.23 mmol) and 2-aminoethyldiphenylborinate (0.07 g, 0.29 mmol) were added and the reaction mixture was stirred for 4 h at 60°C. After the mixture was cooled to room temperature, it was diluted with EtOAc. The organic phase was washed with H₂O and brine. The aqueous layers were extracted three times with EtOAc. The combined organic phases were dried over MgSO₄, filtered and concentrated under reduced pressure. Purification by column chromatography (EtOAc/PE: 1/4, v/v) afforded the product as yellow oil. (0.99 g, 2.16 mmol, 73%). ¹H NMR (400 MHz, CDCl₃) δ 7.37 - 7.27 (m, 10H), 7.25 - 7.21 (m, 2H), 6.89 - 6.83 (m, 2H), 5.72 (dt, *J =* 10.2, 2.4 Hz, 1H), 5.60 (dt, *J =* 10.2, 1.9 Hz, 1H), 4.99 (d, *J =* 11.3 Hz, 1H), 4.78 (d, *J =* 11.5 Hz, 1H), 4.66 (d, *J = 11.9* Hz, 2H), 4.46 (s, 2H), 4.22 - 4.16 (m, 1H), 3.78 (s, 3H), 3.73 - 3.51 (m, 4H), 2.98 (s, 1H); ¹³C NMR (101 MHz, CDCl₃): δ 159.3, 138.8, 138.4, 130.3, 129.4, 128.6, 128.5, 128.3, 128.1, 128.0, 127.9, 127.8, 127.8, 126.8, 113.9, 83.9, 80.2, 75.0, 73.1, 71.6, 71.3, 70.9, 55.4, 44.1.

### Intermediate 21: 8-azidooctyl 4-methylbenzenesulfonate

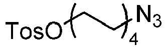

8-Chlorooctan-1-ol (22.4 ml, 67.7 mmol) was dissolved in DMSO (34 ml, 2 M), followed by the addition of NaN₃ (6.60 g, 102 mmol). After stirring for 22 hours at 80°C, NMR analysis of the reaction mixture showed total consumption of the starting material. The reaction mixture was cooled to room temperature and diluted with EtOAc. The organic layer was washed ten times with H₂O and the resulting aqueous layer was extracted three times with EtOAc. The combined organic phases were dried over MgSO₄, filtered and concentrated under reduced pressure. The resulting crude 8-Azidooctan-1-ol was dissolved in anhydrous DCM (335 ml, 0.2 M). The solution was flushed with nitrogen and Et₃N (13.9 ml, 100 mmol) was added. After 30 min, the mixture was cooled to 0 °C and TsCl (19.0 g, 100 mmol) was added dropwise. TLC monitoring after 1 hour indicated the presence of significant amount of starting material, so another portion of Et₃N (13.9 ml, 100 mmol) and and TsCl (19.0 g, 100 mmol) as well as a catalytic amount of DMAP (1.63 g, 13.4 mmol) were added to the mixture. After 18 hours, the reaction was quenched with H₂O and the mixture was diluted with DCM. The organic phase was washed with 1 M HCl, NaHCO₃ (aq. sat.) and brine, was dried over MgSO₄, filtered and concentrated under reduced pressure. Purification by column chromatography (EtOAc/PE: 1/20 -> 1/6, v/v) afforded the product as yellow oil. (19.9 g, 61.1 mmol, 92%). ¹H NMR (400 MHz, CDCl₃): δ 7.82 - 7.76 (m, 2H), 7.35 (d, *J =* 8.0 Hz, 2H), 4.02 (t, *J =* 6.5 Hz, 2H), 3.25 (t, *J =* 6.9 Hz, 2H), 2.45 (s, 3H), 1.59 (dq, *J =* 14.8, 6.8 Hz, 4H), 1.38 - 1.19 (m, 8H) ¹³C NMR (101 MHz, CDCl₃) δ 144.8, 133.3, 129.9, 128.0, 70.7, 51.5, 29.0, 28.9, 26.7, 25.3, 21.8.

### Intermediate 22: 2,3-di-O-benzyl-4-(8-azidooctyl)-6-O-(4-methoxybenzyl)-cyclophellitol alkene

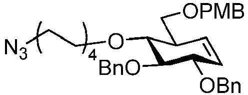

5,6-Bis(benzyloxy)-2-(((4-methoxybenzyl)oxy)methyl)cyclohex-3-en-1-ol (230 mg, 500 µmol) was co-evaporated with toluene and subsequently dissolved in THF (1 ml, 0.5 M). The solution was flushed with nitrogen and cooled to 0°C. KHMDS (0.5 M in toluene, 1.5 mL, 750 µmol) was added to the solution and the reaction mixture was stirred for 1 hour at 0°C. After 8-azidooctyl 4-methylbenzenesulfonate (23) (488 mg, 1.50 mmol) was added, the reaction mixture was warmed to room temperature and stirred for 18 h. The mixture was quenched by the addition of H₂O and diluted with EtOAc. The aqueous phase was extracted three times with EtOAc and the combined organic phases were washed with H₂O and brine. The organic phase was dried over MgSO₄, filtered and concentrated under reduced pressure. Purification by column chromatography (EtOAc/PE: 1/15 -> 1/4, v/v) afforded the product as colourless oil. (219 mg, 357 µmol, 71%). ¹H NMR (400 MHz, CDCl₃): δ 7.43 - 7.24 (m, 12H), 6.92 - 6.87 (m, 2H), 5.74 - 5.65 (m, 2H), 4.90 (s, 2H), 4.70 (s, 2H), 4.53 (d, *J =* 11.9 Hz, 1H), 4.43 (d, *J* = 11.9 Hz, 1H), 4.24 - 4.19 (m, 1H), 3.88 (dt, *J =* 8.9, 6.7 Hz, 1H), 3.82 (s, 3H), 3.74 (dd, *J =* 10.1, 7.8 Hz, 1H), 3.56 (d, *J =* 4.0 Hz, 2H), 3.47 (t, *J =* 9.9 Hz, 1H), 3.36 (dt, *J =* 8.9, 7.0 Hz, 1H), 3.27 (t, *J =* 7.0 Hz, 2H), 2.53 - 2.41 (m, 1H), 1.69 - 1.16 (m, 12H,) ¹³C NMR (101 MHz, CDCl₃): δ 159.3, 139.1, 138.6, 130.4, 129.5, 129.3, 128.5, 128.4, 127.9, 127.9, 127.7, 127.5, 127.0, 113.8, 85.3, 80.8, 78.7, 75.3, 73.6, 72.9, 72.2, 68.9, 55.3, 51.5, 44.5, 30.5, 29.5, 29.2, 28.9, 26.8, 26.2 HRMS (ESI) m/z: [M+Na]⁺ calculated for C₃₇H₄₇N₃NaO₅ 636.3413, found 636.3412.

### Intermediate 23: 2,3-di-O-benzyl-4-(8-azidooctyl)-cyclophellitol alkene

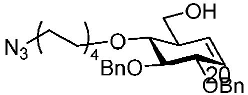

DDQ (39.0 mg, 175 µmol) was added to a solution of alkylated cyclohexene intermediate 22 (91.0 mg, 149 µmol) in a mixture of DCM/H₂O (3 ml, 19/1, v/v). After the reaction mixture was stirred for 18 hours at room temperature, the mixture was diluted with EtOAc. The organic layer was washed with NaHCO₃ (aq. sat.), H₂O and brine. The organic phase was dried over MgSO₄, filtered and concentrated under reduced pressure. Purification by column chromatography (EtOAc/PE: 1/3, v/v) afforded the product as colorless oil. (62.0 mg, 126 µmol, 85%). ¹H NMR (400 MHz, CDCl₃): δ 7.40 - 7.24 (m, 10H), 5.74 (dt, *J =* 10.1, 2.5 Hz, 1H), 5.53 (dt, *J* = 10.2, 1.8 Hz, 1H), 4.88 (d, *J* = 2.6 Hz, 2H), 4.68 (s, 2H), 4.21 - 4.13 (m, 1H), 3.97 (dt, *J* = 8.8, 6.9 Hz, 1H), 3.79 - 3.71 (m, 3H), 3.55 (dt, *J* = 8.8, 7.4 Hz, 1H), 3.47 (t, *J =* 9.7 Hz, 1H), 3.24 (t, *J = 7.0* Hz, 2H), 2.52 - 2.45 (m, 1H), 2.09 - 1.97 (m, 1H), 1.73 - 1.16 (m, 12H) ¹³C NMR (101 MHz, CDCl₃): δ 139.0, 138.5, 128.6, 128.5, 128.0, 127.9, 127.8, 127.7, 85.1, 80.8, 80.4, 75.3, 73.6, 72.3, 64.2, 51.6, 45.8, 30.6, 29.5, 29.2, 28.9, 26.8, 26.2 HRMS (ESI) m/z: [M+Na]⁺ calculated for C₂₉H₃₉N₃NaO₄ 516.2838, found 516.2834.

### Intermediate 24: 2,3-di-O-benzyl-4-(8-azidooctyl)-cyclophellitol

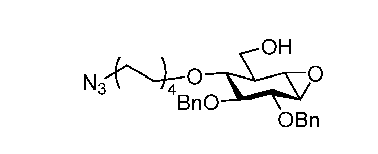

Intermediate 23 (631 mg, 1.28 mmol) was dissolved in CH₂Cl₂ (13 ml, 0.1 M), followed by the addition of mCPBA (442 mg, 1.97 mmol) at 0°C. After stirring for 18 hours at 0°C, TLC monitoring indicated the presence of significant amount of starting material therefore mCPBA (574 mg, 2.56 mmol) was added to the reaction mixture. The mixture was stirred for another 24 hours at 0°C, followed by the dilution with EtOAc. The organic layer was washed with a mixture of NaHCO₃/Na₂S₂O₃ (1/1, v/v, aq. sat.) and brine. The combined aqueous layers were extracted three times with EtOAc. The combined organic layers were dried over MgSO₄, filtered and concentrated under reduced pressure. Purification by column chromatography (EtOAc/PE: 2/5) afforded epoxide the product as colorless oil. (571 mg, 1.12 mmol, 87%). ¹H NMR (400 MHz, CDCl₃): δ 7.42 - 7.26 (m, 10H), 4.81 (d, *J =* 2.8 Hz, 2H), 4.74 (dd, *J =* 28.7, 11.4 Hz, 2H), 4.00 (dd, *J =* 10.8, 5.0 Hz, 1H), 3.92 - 3.85 (m, 2H), 3.80 (dd, *J =* 8.2, 0.5 Hz, 1H), 3.52 - 3.43 (m, 2H), 3.32 (dd, *J =* 3.6, 0.6 Hz, 1H), 3.28 - 3.21 (m, 3H), 3.15 (d, *J =* 3.8 Hz, 1H), 2.14 (dtd, *J* = 6.7, 5.3, 1.6 Hz, 1H), 1.62 - 1.21 (m, 12H) ¹³C NMR (101 MHz, CDCl₃): δ 138.7, 137.7, 128.6, 128.4, 128.1, 128.0, 127.8, 127.7, 84.9, 79.7, 76.3, 75.4, 73.8, 73.3, 63.1, 55.9, 53.1, 51.5, 44.0, 30.4, 29.4, 29.1, 28.9, 26.7, 26.1; HRMS (ESI) m/z: [M+Na]⁺ calculated for C₂₉H₃₉N₃NaO₅ 532.2787, found 532.2782.

### Intermediate 25: 2,3-di-O-benzyl-4-(8-azidooctyl)-glucurono-cyclophellitol

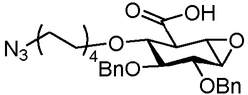

Intermediate 24 (200 mg, 393 µmol) was dissolved in a mixture of DCM/*t*-BuOH/H₂O (12 ml, 4/4/1, v/v/v). After the addition of TEMPO (12.3 mg, 78.6 µmol) and BAIB (316 mg, 982 µmol) at 0°C, the reaction mixture was stirred for 22 hours at 0°C. The reaction was quenched with Na₂S₂O₃ (aq. sat.), followed by the extraction of the acidified aqueous layer (pH = 3) three times with DCM. The combined organic layers were dried over MgSO₄, filtered and concentrated under reduced pressure. Purification by column chromatography (EtOAc/PE: 1/6, 1% AcOH) afforded the product as colorless oil. (185 mg, 354 µmol, 90%) To remove residual AcOH, the product was co-evaporated three times with EtOAc, toluene and chloroform. ¹H NMR (400 MHz, CDCl₃): δ 7.36 - 7.28 (m, 10H), 4.81 (d, *J =* 1.1 Hz, 2H), 4.74 (dd, *J* = 27.0, 11.3 Hz, 2H), 3.90 - 3.82 (m, 2H, H-2), 3.65 - 3.53 (m, 2H, H-4), 3.52 - 3.44 (m, 2H), 3.24 (t, *J =* 7.0 Hz, 2H), 3.20 (d, *J =* 3.6 Hz, 1H), 2.98 (dd, *J =* 10.0, 1.6 Hz, 1H), 1.61 - 1.18 (m, 12H); ¹³C NMR (101 MHz, CDCl₃): δ 175.3, 138.6, 137.7, 128.7, 128.5, 128.2, 128.1, 127.8, 127.8, 84.2, 79.3, 75.5, 74.9, 73.9, 73.5, 54.4, 53.8, 51.6, 48.6, 30.3, 29.3, 29.1, 28.9, 26.7, 25.9; HRMS (ESI) m/z: [M+Na]⁺ calculated for C₂₉H₃₇N₃NaO₆, 546.2580, found 546.2580.

### Intermediate 26: 4-(8-aminooctyl)-glucurono-cyclophellitol

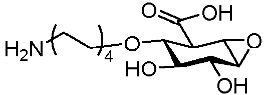

In a 25 ml-flask, intermediate 25 (40.0 mg, 76.5 µmol) was dissolved in anhydrous THF, followed by the addition of t-BuOH (73 µl, 765 µmol) and a glass-coated stirring bar. The flask was flushed with nitrogen and attached to a flow of gas ammonia. By cooling the reaction mixture to -78°C, 7 ml of liquid ammonia was condensed. Freshly cut sodium (35.0 mg, 1.53 mmol) was added portion wise at the same temperature under a flow of nitrogen. After stirring for 30 minutes, the reaction was quenched with AcOH (21 eq.), warmed to room temperature and stirred for 1 hour. The reaction mixture was concentrated under reduced pressure and co-evaporated three times with H₂O using a water-jet-pump. Preparative RP-HPLC (linear gradient, solutions used: A: 50 mM AcOH in H₂O, B: CH₃CN) and subsequent lyophilisation gave the amine product. (6.70 mg, 21.1 µmol, 28%). ¹H NMR (500 MHz, D₂O): δ 3.80 (d, *J* = 8.4 Hz, 1H), 3.73 (dt, *J* = 9.0, 6.2 Hz, 1H), 3.58 (dt, *J* = 9.0, 6.9 Hz, 1H), 3.49 - 3.44 (m, 2H), 3.37 (dd, *J =* 10.4, 8.5 Hz, 1H), 3.15 (d, *J* = 3.7 Hz, 1H), 2.97 (t, *J =* 7.6 Hz, 2H), 2.72 (dd, *J* = 9.8, 1.8 Hz, 1H), 1.67 - 1.24 (m, 12H) ¹³C NMR (126 MHz, D₂O): δ 178.0, 76.9, 75.8, 73.0, 71.2, 56.5, 55.5, 51.0, 39.5, 29.2, 28.0, 27.9, 26.6, 25.3, 25.0 HRMS (ESI) m/z: [M+H]⁺ calculated for C₁₅H₂₈NO₆ 318.1917, found 318.1913.

### Intermediate 27: 8-Benzyloxycarbonylamino-octan-1-ol

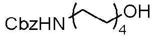

8-Amino-octan-1-ol (6.65 g, 45.8 mmol) was dissolved in acetone/water (700 ml, 2/1, v/v). NaHCO₃ (11.53 g, 137.3 mmol) was added followed by dropwise addition of carboxybenzyl chloride (9.78 ml, 68.7 mmol). After TLC showed full conversion the acetone was removed in vacuo. The remaining water layer was extracted with EtOAc (3x). The combined organic layers were washed with brine, dried over MgSO₄ and concentrated in vacuo. The product was obtained by column chromatography (EtOAc/pentane, 3/7, v/v) as an oil. (11.26 g, 40.30 mmol, 88%). ¹H NMR (400 MHz, CDCl₃) δ 7.39 - 7.28 (m, 5H), 5.09 (s, 2H), 3.63 (t, *J =* 6.6 Hz, 2H), 3.18 (q, *J* = 6.7 Hz, 2H), 1.62 - 1.40 (m, 4H), 1.41 - 1.23 (m, 8H). ¹³C NMR (101 MHz, CDCl₃) δ 156.5, 136.8, 128.6, 128.3, 128.2, 66.7, 63.1, 41.2, 32.8, 30.1, 29.4, 29.3, 26.7, 25.8. HRMS (ESI) m/z: [M+Na]⁺ calculated for C₁₆H₂₅NO₃Na 302.1727, found 302.1734.

### Intermediate 28: 8-Benzyloxycarbonylamino-1-trityloxy octane

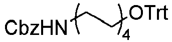

Intermediate 27 (9.8 g, 35 mmol) was dissolved in DMF (58 ml, 0.6 M). Trityl chloride (13.7 g, 175 mmol) and Et₃N (24.5 ml, 175 mmol) were added and the reaction was stirred for 24h. Trityl chloride (4.4 g, 17.5 mmol) and Et₃N (5.0 ml, 36 mmol) were added and the reaction was stirred for 48 h. Upon completion the mixture was diluted with H₂O and extracted three times with Et₂O. The combined organic layers were washed with H₂O (5x) and brine, dried over MgSO₄, filtered and concentrated in vacuo. The product was used without further purification. ¹H NMR (400 MHz, CDCl₃) δ 7.47 - 7.41 (m, 6H), 7.37 - 7.25 (m, 11H), 7.25 - 7.19 (m, 3H), 5.09 (s, 2H), 4.71 (s, 1H), 3.17 (q, *J =* 6.7 Hz, 2H), 3.03 (t, *J =* 6.6 Hz, 2H), 1.65 - 1.55 (m, 2H), 1.51 - 1.40 (m, 2H), 1.39 - 1.19 (m, 8H). ¹³C NMR (101 MHz, CDCl₃) δ 156.5, 144.6, 136.8, 128.8, 128.6, 128.3, 128.2, 127.8, 126.9, 86.4, 66.7, 63.7, 41.2, 30.1, 30.1, 29.5, 29.3, 26.8, 26.3. HRMS (ESI) m/z: [M+Na]⁺ calculated for C₃₅H₃₉NO₃Na 544.2822, found 544.2830.

### Intermediate 29: 8-N-benzyl(benzyloxycarbonyl)-1-trityloxy octane

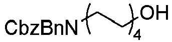

Carbamate 28b (18.3 g, 35 mmol) was dissolved in DMF (96 ml, 0.36 M) and the solution was cooled to 0°C. NaH (60 wt%, 2.9 g, 74 mmol) was added and the reaction was stirred at 0°C for 10 minutes. Benzyl bromide (5.9 ml, 49 mmol) and TBAI (1.3 g, 3.5 mmol) were added and the solution was stirred overnight at room temperature. The reaction mixture was cooled down to 0°C and quenched with H₂O. It was then further diluted with H₂O and extracted with Et₃O (3x). The combined organic layers were washed with H₂O (5x) and brine, dried over MgSO₄, filtered and concentrated under reduced pressure. Column chromatography (Et₂O/PE, 2/98 -> 1/4, v/v) yielded the product. (16.3 g, 26.7 mmol, 76%, over 2 steps). The resulting product (16.3 g, 26 mmol) was dissolved in DCM/MeOH (104 ml, 1/1, 0.25 M). TFA (3% in H₂O, 7.5 ml, 3.0 mmol) was added and the reaction was stirred for 27 hours. More TFA (0.23 ml, 3.0 mmol) was added and the reaction was stirred for another 24 hours. TLC showed complete conversion and the reaction was quenched with NaHCO₃, concentrated in vacuo, dissolved in EtOAc and washed three times with H₂O and brine, dried over MgSO₄, filtrated and concentrated in vacuo. Column chromatography (EtOAc/PE, 1/9 -> 3/7, v/v) afforded the product. (8.1 g, 21.9 mmol, 84%). ¹H NMR (400 MHz, CDCl₃) δ 7.40 - 7.12 (m, 10H), 5.16 (d, *J =* 13.1 Hz, 2H), 4.48 (d, 2H), 3.57 (t, *J* = 6.7 Hz, 2H), 3.29 - 3.14 (m, 2H), 2.32 (s, 1H), 1.57 - 1.43 (m, 4H), 1.38 - 1.14 (m, 8H). ¹³C NMR (101 MHz, CDCl₃) δ 156.8, 156.2, 137.9, 136.8, 136.7, 128.5, 128.4, 127.9, 127.8, 127.3, 67.1, 62.7, 50.4, 50.1, 47.2, 46.2, 32.6, 29.2, 29.2, 28.0, 27.6, 26.6, 25.6. HRMS (ESI) m/z: [M+Na]⁺ calculated for C₂₃H₃₁NO₃Na 392.2196, found 392.2200.

### Intermediate 30: 8-N-benzyl(benzyloxycarbonyl)-1-iodo-octane

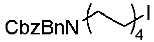

Intermediate 29 (8.1 g, 21.3 mmol) was dissolved in DCM (250 ml, 0.085 M) and cooled to 0°C. PPh₃ (6.7 g, 25.6 mmol), imidazole (1.7 g, 25.6 mmol) and I₂ (6.5 g, 26 mmol) were added to the solution. After stirring for 1 hour at 0 °C the reaction was warmed to rt and stirred for an additional 4 hours. The reaction was quenched with NaHCO₃ (aq. sat.) and the water layer was extracted three times with DCM. The combined organic layers were washed with NH₄Cl (aq. sat.), NaHCO₃ (aq. sat.), Na₂S₂O₃ (aq. sat.) and brine, dried over MgSO₄, filtrated and concentrated in vacuo. Column chromatography (EtOAc/PE, 1/24 -> 1/4, v/v) yielded the product. (10.4 g, 21.6 mmol, 97%). ¹H NMR (400 MHz, CDCl₃) δ 7.42 - 7.13 (m, 10H), 5.17 (d, *J = 11.8* Hz, 2H), 4.49 (d, *J =* 8.0 Hz, 2H), 3.30 - 3.11 (m, 4H), 1.83 - 1.70 (m, 2H), 1.58 - 1.41 (m, 2H), 1.41 - 1.15 (m, 8H). ¹³C NMR (101 MHz, CDCl₃) δ 156.8, 156.2, 138.0, 128.6, 128.5, 128.0, 127.9, 127.3, 67.2, 50.5, 50.2, 47.2, 46.3, 33.5, 30.4, 29.1, 28.4, 28.1, 27.7, 26.7, 7.4. HRMS (ESI) m/z: [M+Na]⁺ calculated for C₂₃H₃₀INO₂Na 502.1213, found 502.1216.

### Intermediate 31: Phenyl 2-azido-2-deoxy-3-O-benzyl-6-O-(2-Naphthylmethyl)-1-thio-α-D-glucopyranoside

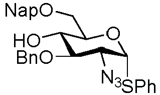

Phenyl 2-azido-2-deoxy-3-O-benzyl-4,6-benzylidene-1-thio-α-D-glucopyranoside (1.70 g, 3.6 mmol) was suspended in MeOH (30 mL). DCE (5 ml) was added to obtain a clear solution. The solution was heated to 50°C and camphorsulfonic acid (0.42 g, 1.8 mmol) was added. When TLC analysis showed full conversion of the starting material the reaction was cooled down to 0°C and quenched with Et₃N. Solvents were removed in vacuo and the crude residue was dissolved in EtOAc, washed with HCI (aq. 1M), NaHCO₃ (aq. sat.), brine and dried over MgSO₄. The solvent was evaporated and the crude diol was dissolved in anhydrous MeCN (15 ml). 2-aminoethyl diphenyl borinate (0.081 g, 0.36 mmol), 2-bromomethyl-naphtalene (1.2 g, 5.4 mmol), KI (0.60 g, 3.6 mmol) and K₂CO₃ (0.55 g, 4.0 mmol) were added. The mixture was heated to 60°C and stirred overnight. The reaction mixture was transferred to a separatory funnel containing EtOAc and H₂O, layers were separated and the water layer was re-extracted three times with EtOAc. The organic layers were combined, washed with brine, dried over MgSO₄, filtered and concentrated in vacuo. Column chromatography (EtOAc/pentane, 1/9 -> 1/3, v/v) afforded the product. (1.77 g, 3.35 mmol, 93%). ¹H NMR (400 MHz, CDCl₃) δ 7.83 - 7.75 (m, 3H), 7.73 - 7.69 (m, 1H), 7.52 - 7.26 (m, 10H), 7.24 - 7.17 (m, 3H), 5.54 (d, *J* = 5.4 Hz, 1H), 4.91 (d, *J =* 11.1 Hz, 1H), 4.80 (d, *J =* 11.1 Hz, 1H), 4.69 (d, *J =* 12.1 Hz, 1H), 4.61 (d, *J = 12.1* Hz, 1H), 4.34 (dt, *J =* 9.6, 4.2 Hz, 1H), 3.85 (dd, *J =* 10.0, 5.4 Hz, 1H), 3.78 - 3.60 (m, 4H), 2.66 (d, *J =* 3.1 Hz, 1H). ¹³C NMR (101 MHz, CDCl₃) δ 137.9, 135.2, 133.4, 133.2, 133.0, 132.2, 129.1, 128.7, 128.3, 128.2, 128.2, 127.9, 127.8, 127.8, 126.6, 126.2, 126.0, 125.6, 87.3, 81.4, 75.4, 73.7, 72.1, 71.2, 69.6, 63.6. HRMS (ESI) m/z: [M+Na]⁺ calculated for C₃₀H₂₉N₃O₄SNa 550.1771, found 550.1771.

### Intermediate 32: Phenyl 2-azido-2-deoxy-3-O-benzyl-4-O-(8-N-benzyl(benzyloxycarbonyl)-1-octyl)-6-O-(2-Naphthylmethyl)-1-thio-D-glucopyranoside

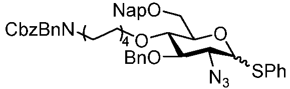

Intermediate 31 (α/β mixture) (2.3 g, 6.2 mmol) and intermediate 30 (7.7 g, 16 mmol) were dissolved in dry DMF (13 ml, 0.5 M) and the solution was cooled to 0°C. NaH (60% dispersion in mineral oil, 0.43 g, 10.7 mmol) was added and the reaction was stirred for 18 hours at room temperature. The reaction mixture was cooled to 0°C and quenched with water. The water layer was extracted with Et₂O (3x), the combined organic layers were washed five times with water and brine, dried over MgSO₄, filtrated and concentrated in vacuo. Column chromatography (Et₂O/PE, 1/49 -> 1/4, v/v) yielded the product. (4.4 g, 5.4 mmol, 88%). ¹H NMR (400 MHz, CDCl₃) δ 7.79 (d, *J* = 7.9 Hz, 3H), 7.74 (s, 1H), 7.54 - 7.12 (m, 23H), 5.60 (d, *J =* 5.4 Hz, 1H), 5.17 (d, *J =* 10.2 Hz, 2H), 4.88 (d, *J* = 10.5 Hz, 1H), 4.84 (d, *J =* 10.5 Hz, 1H), 4.78 (d, *J =* 12.1 Hz, 1H), 4.60 (d, *J =* 12.2 Hz, 1H), 4.48 (d, *J* = 9.4 Hz, 2H), 4.33 - 4.27 (m, 1H), 3.91 (dd, *J =* 10.3, 5.4 Hz, 1H), 3.79 (dd, *J =* 10.8, 3.7 Hz, 1H), 3.76 - 3.64 (m, 3H), 3.54 (t, *J* = 9.4 Hz, 1H), 3.39 (q, *J* = 7.0 Hz, 1H), 3.27 - 3.12 (m, 2H), 1.52 - 1.32 (m, 4H), 1.20 - 1.00 (m, 8H). ¹³C NMR (101 MHz, CDCl₃) δ 156.2, 138.0, 137.8, 135.3, 133.6, 133.2, 133.0, 132.1, 129.1, 128.6, 128.5, 128.5, 128.2, 128.0, 128.0, 127.9, 127.8, 127.7, 127.7, 126.7, 126.2, 126.0, 126.0, 87.3, 81.8, 78.4, 75.7, 75.7, 73.7, 73.6, 73.6, 73.4, 72.0, 68.3, 67.2, 64.0, 50.5, 50.2, 47.3, 46.3, 30.4, 29.8, 29.5, 29.3, 28.1, 27.7, 26.8, 26.1. HRMS (ESI) m/z: [M+Na]⁺ calculated for C₅₃H₅₈N₄O₆SNa 901.3969, found 901.3969.

### Intermediate 33: Phenyl 2-azido-2-deoxy-3-O-benzyl-4-O-(8-N-benzyl(benzyloxycarbonyl)-1-octyl)-1-thio-D-glucopyranoside

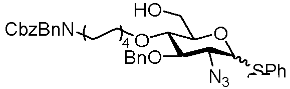

Intermediate 32 (4.8 g, 5.5 mmol) was dissolved in DCM/MeOH (38 ml, 0.15 M, 4/1, v/v) and flushed with N₂ for 15 min. DDQ (3.7 g, 16 mmol) was added and the reaction was stirred for 2.5 hours in the dark. The reaction mixture was concentrated in vacuo and the residue was dissolved in EtOAc. The organic layer was washed with NaHCO₃ (aq. sat. 3x) and brine, dried over MgSO₄, filtrated and concentrated in vacuo. Column chromatography (Et₂O/PE, 3/47 -> 2/3. v/v) yielded the product. (2.6 g, 3.9 mmol, 72%). ¹H NMR (400 MHz, CDCl₃) δ 7.49 (dd, *J* = 7.8, 1.7 Hz, 2H), 7.43 - 7.22 (m, 17H), 7.17 (d, *J =* 6.6 Hz, 1H), 5.54 (d, *J =* 5.3 Hz, 1H), 5.17 (d, *J* = 13.2 Hz, 2H), 4.88 (s, 2H), 4.49 (d, *J =* 8.3 Hz, 2H), 4.18 (dt, *J* = 9.9, 3.2 Hz, 1H), 3.84 (dd, *J* = 10.3, 5.3 Hz, 1H), 3.82 - 3.73 (m, 4H), 3.62 - 3.54 (m, 1H), 3.43 (t, *J* = 9.3 Hz, 1H), 3.31 - 3.15 (m, 2H), 1.60 - 1.43 (m, 4H), 1.32 - 1.14 (m, 8H). ¹³C NMR (101 MHz, CDCl₃) δ 137.8, 132.6, 129.3, 128.6, 128.6, 128.6, 128.2, 128.1, 128.0, 127.9, 87.2, 81.6, 78.5, 75.8, 73.6, 72.7, 67.3, 64.1, 61.6, 30.5, 29.5, 29.3, 26.8, 26.2. HRMS (ESI) m/z: [M+Na]⁺ calculated for C₄₂H₅₀N₄O₆SNa 761.3343, found 761.3365.

### Intermediate 34: Phenyl 2-azido-2-deoxy-3-O-benzyl-4-O-(8-N-benzyl(benzyloxycarbonyl)-1-octyl)-6-O-tert-butyldiphenylsilyl-1-thio-α-D-glucopyranoside

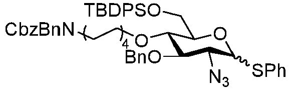

Intermediate 33 (3.4 g, 4.5 mmol) was dissolved in DMF (22.8 ml, 0.2 M). TBDPSCI (2.5 g, 9.1 mmol) and imidazole (1.6 g, 22.8 mmol) were added. After stirring for 17 hours TLC showed complete conversion and the reaction was quenched with H₂O. The reaction mixture was then diluted further with H₂O and extracted five times with Et₂O. The combined organic layers were washed five times with H₂O and brine, dried over MgSO₄, filtrated and concentrated in vacuo. Column chromatography (Et₂O/PE, 3/47, v/v) yielded the product. (4.3 g, 4.4 mmol, 95% contaminated with excess silyl reagent). ¹H NMR (400 MHz, CDCl₃) δ 7.75 - 7.64 (m, 6H), 7.48 - 7.19 (m, 23H), 7.19 - 7.13 (m, 1H), 5.59 (d, *J =* 5.4 Hz, 1H), 5.16 (d, *J* = 11.2 Hz, 2H), 4.88 (s, 2H), 4.48 (d, *J* = 6.8 Hz, 2H), 4.15 (d, *J =* 9.6 Hz, 1H), 3.94 (dd, *J =* 11.5, 3.1 Hz, 1H), 3.91 - 3.78 (m, 3H), 3.74 (t, *J =* 9.6 Hz, 1H), 3.68 - 3.60 (m, 2H), 3.27 - 3.14 (m, 2H), 1.57 - 1.44 (m, 4H), 1.30 - 1.12 (m, 8H), 1.03 (s, 9H). ¹³C NMR (101 MHz, CDCl₃) δ 137.8, 136.0, 135.6, 134.9, 134.3, 133.7, 133.1, 131.5, 129.8, 129.1, 128.6, 128.5, 128.2, 127.8, 127.7, 127.4, 87.2, 81.9, 78.2, 76.0, 73.6, 73.2, 67.3, 64.2, 62.3, 50.6, 50.2, 47.4, 46.4, 30.6, 29.7, 29.5, 26.9, 26.3, 19.5. HRMS (ESI) m/z: [M+Na]⁺ calculated for C₅₈H₆₈N₄O₆SSiNa 999.4521, found 999.4539.

### Intermediate 35: 2-azido-2-deoxy-3-O-benzyl-4-O-(8-N-benzyl(benzyloxycarbonyl)-1-octyl)-6-O-tert-butyldiphenylsilyl-D-glucopyranose (33a)

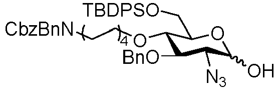

Intermediate 34 (6.2 g, 6.3 mmol) was dissolved in acetone/H₂O/DCM (49.5 ml, 0.13 M, 9/1/1, v/v). The reaction mixture was flushed with N₂. NIS (3.1 g, 13.5 mmol) was added and the reaction was stirred in the dark for 7 hours. The reaction was quenched with solid Na₂S₂O₃. The reaction mixture was concentrated in vacuo and dissolved in EtOAc. The organic layer was washed with Na₂S₂O₃ (aq. sat.) and brine, dried over MgSO₄, filtrated and concentrated in vacuo. Column chromatography (Et₂O/PE, 1/20 -> 1/4, v/v) yielded the product. (4.4 g, 5.0 mmol, 79%, α/β = 1/5). ¹H NMR (400 MHz, CDCl₃) δ 7.75 - 7.63 (m, 10H), 7.46 - 7.12 (m, 54H), 5.25 (t, *J =* 3.2 Hz, 1H), 5.17 (d, *J =* 11.4 Hz, 5H), 4.88 - 4.77 (m, 5H), 4.52 - 4.41 (m, 6H), 3.97 - 3.75 (m, 9H), 3.67 - 3.48 (m, 5H), 3.41 - 3.13 (m, 12H), 3.00 (s, 1H), 1.54 1.41 (m, 9H), 1.27 - 1.11 (m, 21H), 1.08 - 1.00 (m, 23H). ¹³C NMR (101 MHz, CDCl₃) δ 138.0, 136.1, 135.7, 129.8, 128.6, 128.3, 128.0, 127.8, 127.7, 96.1, 92.1, 83.1, 80.1, 78.2, 77.6, 76.3, 75.7, 73.4, 72.2, 67.6, 67.3, 64.2, 62.8, 62.6, 30.5, 29.6, 29.4, 27.0, 26.9, 26.2, 19.4. HRMS (ESI) m/z: [M+Na]⁺ calculated for C₅₂H₆₄N₄O₇SiNa 907.4436, found 907.4436.

### Intermediate 36: 2-azido-2-deoxy-3-O-benzyl-4-O-(8-N-benzyl(benzyloxycarbonyl)-1-octyl)-6-O-tert-butyldiphenylsilyl-D-glucopyranosyl N-phenyltrifluoroacetimidate

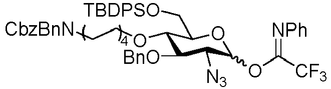

Intermediate 35 (3.8 g, 4.3 mmol) was dissolved in DCM (3.5 ml, 0.2 M). 2,2,2-trifluoro-N-phenylacetimidoyl chloride (1.0 ml, 6.4 mmol) and Cs₂CO₃ (2.1 g, 6.4 mmol) were added. And the mixture was stirred for 6.5 hours. Upon completion the suspension was filtrated over celite and the filtrate was concentrated under reduced pressure. Column chromatography (Et₂O/PE, 1/20 -> 9/10, v/v) yielded the product. (4.2 g, 4.0 mmol, 94%). ¹H NMR (400 MHz, CDCl₃) δ 7.74 - 7.64 (m, 4H), 7.46 - 7.02 (m, 26H), 6.80 (d, *J* = 7.7 Hz, 1H), 6.72 (d, *J =* 7.4 Hz, 1H), 5.17 (d, *J = 10.6* Hz, 2H), 4.94 - 4.79 (m, 2H), 4.49 (d, *J* = 7.4 Hz, 2H), 3.98 - 3.78 (m, 3H), 3.78 - 3.53 (m, 3H), 3.31 - 3.14 (m, 2H), 1.52 (s, 4H), 1.21 (d, *J =* 16.1 Hz, 8H), 1.05 (s, 9H). ¹³C NMR (101 MHz, CDCl₃) δ 138.0, 137.8, 135.9, 135.6, 133.7, 133.6, 132.9, 129.9, 129.8, 129.4, 128.9, 128.8, 128.7, 128.6, 128.5, 128.4, 128.3, 128.2, 128.0, 127.9, 127.8, 127.7, 127.4, 127.4, 127.3, 126.3, 124.5, 124.4, 120.6, 119.4, 82.9, 80.3, 77.6, 76.6, 75.9, 74.7, 73.7, 73.5, 67.2, 65.4, 63.0, 62.0, 50.7, 50.6, 50.2, 47.3, 46.4, 30.5, 30.5, 29.7, 29.6, 29.4, 28.2, 27.8, 26.9, 26.9, 26.3, 19.5.

### Intermediate 37: 2,3-di-O-benzyl-4-O-(2-Azido-2-deoxy-3-O-benzyl-4-O-(8-N-benzyl(benzyloxycarbonyl)-1-octyl)6-O-tert-butyldiphenylsilyl-α-D-glucopyranosyl)-6-O-benzoyl-cyclophellitol alkene

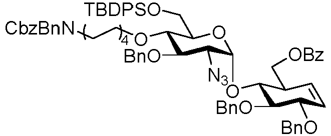

Intermediate 36 (1.3 g, 1.3 mmol) and intermediate 3 (0.39 g, 0.9 mmol) were co-evaporated thrice with toluene and dissolved in dry DCM (10.6 ml, 0.068 M). 3Å molecular sieves were added and the mixture was stirred for 1 hour. The mixture was cooled to -78°C and TfOH (0.1 M in DCM, 2.8 ml, 0.28 mmol) was added. The reaction mixture was slowly warmed to -30°C within 1.5 hours and stirred for an additional hour at this temperature. The reaction was quenched with Et₃N at -30°C and diluted with DCM. The organic layer was washed with water and the water layer was extracted with DCM (2x). The combined organic layers were washed with brine, dried over MgSO₄, filtrated and concentrated in vacuo. Column chromatography (EtOAc/pentane, 1/20 -> 1/3, v/v) yielded the product. (1.2 g, 0.88 mmol, 97%). ¹H NMR (400 MHz, CDCl₃) δ 7.83 (d, *J =* 7.3 Hz, 2H), 7.60 - 7.52 (m, 4H), 7.44 - 7.13 (m, 34H), 5.80 (dt, *J* = 10.2, 2.1 Hz, 1H), 5.68 (d, *J =* 3.9 Hz, 1H), 5.63 - 5.57 (m, 1H), 5.17 (d, *J* = 10.3 Hz, 2H), 5.07 (d, *J* = 10.7 Hz, 1H), 4.98 (d, *J =* 10.7 Hz, 1H), 4.88 (d, *J =* 10.5 Hz, 1H), 4.83 (d, *J =* 10.6 Hz, 1H), 4.71 (d, *J =* 11.5 Hz, 1H), 4.64 (d, *J =* 11.5 Hz, 1H), 4.53 - 4.44 (m, 3H), 4.32 - 4.24 (m, 2H), 4.02 - 3.89 (m, 3H), 3.83 - 3.73 (m, 2H), 3.71 - 3.54 (m, 5H), 3.29 - 3.14 (m, 3H), 2.82 - 2.75 (m, 1H), 1.55 - 1.42 (m, 4H), 1.25 - 1.11 (m, 8H), 0.97 (s, 9H). ¹³C NMR (101 MHz, CDCl₃) δ 166.2, 138.9, 138.2, 138.1, 135.9, 135.6, 133.7, 133.3, 133.0, 129.8, 129.6, 129.6, 129.5, 128.6, 128.6, 128.5, 128.4, 128.3, 127.9, 127.8, 127.7, 127.6, 127.6, 127.5, 98.1, 84.4, 81.0, 80.0, 77.9, 75.6, 74.8, 74.2, 73.3, 72.9, 71.8, 67.2, 64.4, 63.4, 62.1, 50.5, 43.2, 30.5, 29.6, 29.4, 27.0, 26.9, 26.2, 19.4. HRMS (ESI) m/z: [M+Na]⁺ calculated for C₈₀H₉₀N₄O₁₁SiNa 1333.6268, found 1333.6309.

### Intermediate 38: 2,3-di-O-benzyl-4-O-(2-N-acetyl-2-deoxy-3-O-benzyl-4-O-(8-N-benzyl(benzyloxycarbonyl)-1-octyl)6-O-tert-butyldiphenylsilyl-α-D-glucopyranosyl)-6-O-benzoyl-cyclophellitol alkene

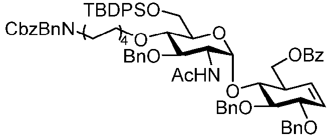

Intermediate 37 (1.2 g, 0.88 mmol) was dissolved in THF/H₂O (10 ml, 0.085 M, 17/3, v/v). PPh₃ (0.58 g, 2.2 mmol) and pyridine (0.080 ml, 1.0 mmol) were added. The reaction mixture was heated to 50°C and stirred for 3 hours. The reaction mixture was concentrated in vacuo. The residue was dissolved in DCM, dried with MgSO₄, and concentrated in vacuo. The crude product was co-evaporated with toluene and dissolved in DCM (8.8 ml, 0.1 M). Acetic anhydride (1.40 ml, 13.4 mmol) and pyridine (1.2 ml, 13.4 mmol) were added and the reaction mixture was stirred for 16.5 hours. The mixture was cooled to 0°C and quenched with water. The organic layer was washed with CuSO₄ (aq. sat.), NaHCO₃ (aq. sat.) and brine, dried over MgSO₄, filtrated and concentrated in vacuo. Column chromatography (EtOAc/PE, 1/9 -> 2/3, v/v) yielded the product. (1.1 g, 0.86 mmol, 98%). ¹H NMR (400 MHz, CDCl₃) δ 7.98 - 7.91 (m, 2H), 7.74 - 7.64 (m, 4H), 7.48 - 7.42 (m, 1H), 7.39 - 7.19 (m, 33H), 6.64 (d, *J =* 9.6 Hz, 1H), 5.83 (dt, *J =* 10.3, 2.5 Hz, 1H), 5.68 (dt, *J =* 10.3, 2.0 Hz, 1H), 5.18 (d, *J* = 12.6 Hz, 2H), 5.01 - 4.90 (m, 2H), 4.82 (d, *J* = 11.3 Hz, 1H), 4.74 - 4.44 (m, 8H), 4.38 (td, *J* = 9.6, 3.4 Hz, 1H), 4.23 - 4.17 (m, 1H), 3.97 - 3.78 (m, 6H), 3.78 - 3.58 (m, 4H), 3.23 (dt, *J* = 32.6, 7.5 Hz, 2H), 2.68 (t, *J* = 6.2 Hz, 1H), 1.58 - 1.44 (m, 7H), 1.29 - 1.13 (m, 8H), 1.05 (s, 9H). ¹³C NMR (101 MHz, CDCl₃) δ 170.0, 165.8, 156.6, 155.9, 138.5, 137.8, 137.6, 137.4, 136.7, 135.6, 135.4, 133.5, 132.9, 132.8, 129.7, 129.5, 129.3, 129.2, 128.4, 128.4, 128.3, 128.1, 128.0, 127.8, 127.8, 127.7, 127.6, 127.5, 127.4, 127.3, 127.1, 126.6, 100.1, 81.4, 81.2, 78.7, 77.5, 76.9, 74.9, 74.6, 73.4, 72.9, 71.2, 66.9, 63.8, 62.3, 53.0, 50.3, 50.0, 47.1, 46.1, 43.7, 30.3, 29.3, 29.1, 27.9, 27.6, 26.7, 26.6, 26.0, 22.6, 19.1. HRMS (ESI) m/z: [M+Na]⁺ calculated for C₈₂H₉₄N₂O₁₂SiNa 1349.6468, found 1349.6487.

### Intermediate 39: 2,3-di-O-benzyl-4-O-(2-N-acetyl-2-deoxy-3-O-benzyl-4-O-(8-N-benzyl(benzyloxycarbonyl)-1-octyl)6-O-tert-butyldiphenylsilyl-α-D-glucopyranosyl)-cyclophellitol alkene

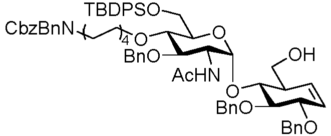

Intermediate 38 (1.1 g, 0.86 mmol) was dissolved in MeOH/DCM (21 ml, 0.04 M, 9.5/1, v/v), NaOMe (4.37 M in MeOH, 0.12 ml, 0.57 mmol) was added and the mixture was stirred for 21.5 hours. NH₄Cl was added and the reaction was concentrated in vacuo. The residue was dissolved in in EtOAc, washed with water and brine, dried over MgSO₄, filtrated and concentrated in vacuo. Column chromatography (EtOAc/PE, 1/4 -> 2/3, v/v) yielded the product. (1.0 g, 0.83 mmol, 97%). ¹H NMR (400 MHz, CDCl₃) δ 7.75 - 7.66 (m, 4H), 7.43 - 7.13 (m, 31H), 6.70 (d, *J =* 9.7 Hz, 1H), 5.88 - 5.81 (m, 1H), 5.66 - 5.60 (m, 1H), 5.17 (d, *J =* 10.8 Hz, 2H), 4.96 - 4.89 (m, 2H), 4.71 (dd, *J =* 11.2, 5.7 Hz, 2H), 4.65 (d, *J =* 11.6 Hz, 1H), 4.57 (d, *J* = 11.6 Hz, 1H), 4.54 - 4.46 (m, 3H), 4.34 - 4.25 (m, 1H), 4.21 - 4.16 (m, 1H), 3.96 - 3.75 (m, 7H), 3.67 - 3.51 (m, 4H), 3.31 - 3.14 (m, 2H), 2.42 - 2.34 (m, 1H), 1.55 - 1.46 (m, 7H), 1.20 (d, *J= 20.5* Hz, 8H), 1.05 (s, 9H). ¹³C NMR (101 MHz, CDCl₃) δ 170.3, 138.6, 138.0, 137.9, 137.5, 135.9, 135.8, 135.6, 133.7, 133.1, 129.7, 129.6, 129.5, 128.6, 128.5, 128.4, 128.3, 128.2, 128.2, 128.1, 127.9, 127.9, 127.9, 127.8, 127.7, 127.6, 127.5, 127.4, 127.3, 127.2, 126.6, 100.1, 81.4, 78.9, 77.9, 77.7, 74.9, 74.8, 73.6, 73.1, 71.4, 67.1, 62.7, 62.3, 60.4, 53.1, 50.5, 50.1, 47.2, 46.2, 46.1, 30.4, 29.5, 29.3, 28.1, 27.7, 26.8, 26.8, 26.2, 22.7, 19.3. HRMS (ESI) m/z: [M+Na]⁺ calculated for C₇₅H₉₀N₂O₁₁SiNa 1245.6206, found 1245.6245.

### Intermediate 40: 2,3-di-O-benzyl-4-O-(2-N-acetyl-2-deoxy-3-O-benzyl-4-O-(8-N-benzyl(benzyloxycarbonyl)-1-octyl)6-O-tert-butyldiphenylsilyl-α-D-glucopyranosyl)-6-O-tert-butyloxycarbonyl-cyclophellitol alkene

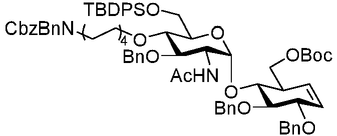

Intermediate 39 (0.53 g, 0.43 mmol) was co-evaporated with toluene (3x) and dissolved in THF (3.2 ml, 0.14 M). Boc₂O (0.38 g, 1.8 mmol) and DMAP (0.16 mM in THF, 0.32 ml, 0.05 mmol) were added. The reaction was stirred for 1 hour and quenched with H₂O. The reaction mixture was diluted with Et₂O and washed with NH₄Cl (aq. sat.), NaHCO₃ (aq. sat.) and brine, dried over MgSO₄, filtrated and concentrated in vacuo. Column chromatography (EtOAc/PE, 1/9 -> 3/7, v/v) yielded the product. (0.47 g, 0.35 mmol, 82%). ¹H NMR (300 MHz, CDCl₃) δ 7.76 - 7.65 (m, 4H), 7.43 - 7.14 (m, 31H), 6.73 (d, *J =* 9.7 Hz, 1H), 5.87 - 5.78 (m, 1H), 5.67 - 5.57 (m, 1H), 5.18 (d, *J* = 7.0 Hz, 2H), 4.94 (d, *J =* 11.1 Hz, 1H), 4.86 (d, *J* = 3.5 Hz, 1H), 4.78 (d, *J* = 11.3 Hz, 1H), 4.73 - 4.44 (m, 7H), 4.42 - 4.17 (m, 4H), 3.96 - 3.56 (m, 10H), 3.31 - 3.06 (m, 2H), 2.52 (s, 1H), 1.58 - 1.37 (m, 16H), 1.28 - 1.12 (m, 8H), 1.05 (s, 9H). ¹³C NMR (75 MHz, CDCl₃) δ 170.4, 153.4, 138.8, 138.0, 137.9, 137.6, 135.9, 135.7, 135.6, 133.9, 133.1, 129.6, 128.6, 128.6, 128.6, 128.5, 128.3, 128.3, 128.2, 128.0, 128.0, 127.9, 127.7, 127.6, 127.5, 127.3, 126.8, 100.5, 82.1, 81.7, 79.3, 77.7, 77.6, 77.5, 77.2, 76.8, 75.0, 74.9, 73.5, 73.2, 71.5, 67.2, 66.1, 62.5, 53.3, 50.5, 50.2, 47.3, 46.3, 44.0, 30.5, 29.6, 29.4, 27.8, 26.9, 26.8, 26.2, 22.7, 19.4. HRMS (ESI) m/z: [M+H]⁺ calculated for C₈₀H₉₉N₂O₁₃Si 1323.6911, found 1323.6910.

### Intermediate 41: 1-iodo-2,3-di-O-benzyl-4-O-(2-N-acetyl-2-deoxy-3-O-benzyl-4-O-(8-N-benzyl(benzyloxycarbonyl)-1-octyl)-6-O-tert-butyldiphenylsilyl-α-D-glucopyranosyl)-6,7-O-carbonyl-cyclophellitol alkane

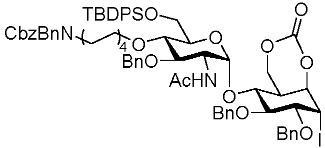

Intermediate 40 (0.47 g, 0.35 mmol) was dissolved in DCM/AcOH (1.8 ml, 0.2 M, 2/1, v/v). NIS (0.16 g, 0.71 mmol) was added and the reaction was stirred for 20 hours. Additional NIS (0.039 g, 0.18 mmol) was added and the reaction was stirred for 2 hours. The solution was diluted with Et₂O and quenched with Et₃N. The organic layer was washed with NH₄Cl (aq. sat.), NaHCO₃ (aq. sat.), Na₂S₂O₃ (aq. sat.) and brine, dried over MgSO₄, filtrated and concentrated in vacuo. Column chromatography (EtOAc/PE, 1/9 -> 2/3, v/v) yielded the product. (0.33 g, 0.24 mmol, 68%). ¹H NMR (400 MHz, CDCl₃) δ 7.74 - 7.62 (m, 4H), 7.46 - 7.11 (m, 31H), 6.37 (d, *J* = 9.7 Hz, 1H), 5.17 (d, *J =* 11.3 Hz, 2H), 5.08 (d, *J =* 10.5 Hz, 1H), 5.01 (d, *J =* 12.1 Hz, 1H), 4.87 - 4.77 (m, 3H), 4.71 - 4.44 (m, 7H), 4.34 (td, *J =* 9.9, 3.4 Hz, 1H), 4.04 (dd, *J =* 12.2, 2.7 Hz, 1H), 3.96 - 3.71 (m, 7H), 3.71 - 3.54 (m, 3H), 3.32 - 3.14 (m, 3H), 3.12 (dd, *J =* 9.3, 4.0 Hz, 1H), 2.66 - 2.57 (m, 1H), 1.56 - 1.44 (m, 4H), 1.24 (d, *J =* 4.8 Hz, 11H), 1.04 (d, *J =* 5.9 Hz, 9H). ¹³C NMR (101 MHz, CDCl₃) δ 170.5, 146.8, 138.6, 138.1, 137.1, 136.8, 135.9, 135.6, 133.9, 132.9, 130.0, 129.8, 128.8, 128.7, 128.6, 128.6, 128.5, 128.5, 128.4, 128.3, 128.0, 127.9, 127.7, 127.6, 101.5, 82.2, 81.7, 81.4, 77.8, 77.4, 76.7, 76.3, 75.0, 74.2, 73.4, 72.3, 68.4, 67.2, 62.7, 53.2, 50.6, 50.2, 47.3, 46.3, 35.2, 30.6, 30.5, 29.7, 29.5, 28.2, 27.8, 27.0, 26.9, 26.4, 22.5, 19.5. HRMS (ESI) m/z: [M+H]⁺ calculated for C₇₆H₉₀N₂O₁₃Sil 1393.5251, found 1393.5247.

### Intermediate 42: 2,3-di-O-benzyl-4-O-(2-N-acetyl-2-deoxy-3-O-benzyl-4-O-(8-N-benzyl(benzyloxycarbonyl)-1-octyl)6-O-tert-butyldiphenylsilyl-α-D-glucopyranosyl)-cyclophellitol

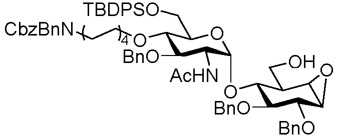

Intermediate 41 (0.28 g, 0.24 mmol) was dissolved in MeOH/DCM (3.4 ml, 0.7 M, 12:5, v/v). NaOMe (4.37 M in MeOH, 0.13 ml, 0.57 mmol) was added and the reaction was stirred for 15.5 hours. The reaction was quenched with Et₃N·HCl and concentrated in vacuo. The residue was dissolved in EtOAc, washed with H₂O and brine, dried over MgSO₄, filtrated and concentrated in vacuo. The product was used without further purification. (0.26 g, 0.21 mmol, 88%). ¹H NMR (400 MHz, CDCl₃) δ 7.75 - 7.66 (m, 4H), 7.45 - 7.14 (m, 31H), 6.80 (d, *J* = 9.7 Hz, 1H), 5.20 - 5.14 (m, 2H), 4.94 (d, *J =* 11.0 Hz, 1H), 4.86 (d, *J =* 3.5 Hz, 1H), 4.78 (d, *J =* 11.2 Hz, 1H), 4.72 - 4.62 (m, 3H), 4.52 - 4.43 (m, 3H), 4.32 - 4.25 (m, 1H), 4.09 - 4.01 (m, 1H), 3.99 - 3.86 (m, 3H), 3.85 - 3.74 (m, 3H), 3.58 (m, 5H), 3.35 - 3.31 (m, 1H), 3.30 - 3.13 (m, 3H), 2.03 - 1.96 (m, 1H), 1.56 - 1.44 (m, 4H), 1.43 (s, 3H), 1.30 - 1.14 (m, 8H), 1.05 (s, 9H). ¹³C NMR (101 MHz, CDCl₃) δ 170.2, 138.6, 138.0, 137.4, 137.2, 136.0, 135.6, 133.7, 133.1, 129.7, 128.7, 128.6, 128.6, 128.5, 128.3, 128.2, 128.2, 128.1, 127.9, 127.8, 127.7, 127.6, 127.5, 127.5, 127.3, 127.2, 100.6, 83.1, 81.3, 80.0, 77.5, 77.4, 77.1, 76.8, 75.8, 75.5, 74.7, 73.8, 73.0, 72.7, 67.2, 62.6, 61.7, 56.5, 53.5, 53.1, 52.2, 50.5, 50.2, 47.3, 46.3, 45.9, 44.5, 30.5, 29.7, 29.6, 29.4, 28.2, 26.9, 26.8, 26.2, 22.7, 19.3. HRMS (ESI) m/z: [M+Na]⁺ calculated for C₇₅H₉₀N₂O₁₂SiNa 1261.6155, found 1261.6184.

### Intermediate 43: 2,3-di-O-benzyl-4-O-(2-N-acetyl-2-deoxy-3-O-benzyl-4-O-(8-N-benzyl(benzyloxycarbonyl)-1-octyl)6-O-tert-butyldiphenylsilyl-α-D-glucopyranosyl)-glucurono-cyclophellitol (37a)

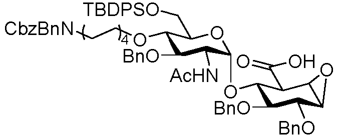

Intermediate 42 (0.25 g, 0.21 mmol) was dissolved in *t*-BuOH/DCM/H₂O (7.7 ml, 26 mM, 5/4/1, v/v). TEMPO (7 mg, 0.04 mmol) and BAIB (0.161 g, 0.50 mmol) were added and the solution was stirred for 24 hours. The reaction was diluted with DCM and H₂O and quenched with Na₂S₂O₃ (aq. sat.). The water layer was acidified with AcOH and extracted with DCM (4x). The combined organic layers were dried over MgSO₄, filtrated and concentrated in vacuo. Column chromatography (EtOAc/PE, 1/9 -> 1/1, v/v, 1% AcOH) yielded the product. (0.17 g, 0.13 mmol, 67%). ¹H NMR (400 MHz, CDCl₃) δ 7.76 - 7.65 (m, 4H), 7.46 - 7.14 (m, 31H), 6.07 (d, *J* = 9.7 Hz, 1H), 5.17 (d, *J =* 13.7 Hz, 2H), 4.93 (dd, *J =* 7.3, 3.7 Hz, 2H), 4.78 (t, *J =* 11.5 Hz, 2H), 4.64 (d, *J =* 11.3 Hz, 1H), 4.57 (dd, *J =* 11.1, 5.0 Hz, 2H), 4.49 (d, *J =* 7.4 Hz, 2H), 4.19 (td, *J =* 10.0, 3.6 Hz, 1H), 3.98 - 3.61 (m, 9H), 3.58 (dd, *J =* 10.4, 8.4 Hz, 1H), 3.47 (dd, *J =* 10.1, 8.1 Hz, 1H), 3.32 (t, *J* = 2.9 Hz, 1H), 3.29 - 3.14 (m, 3H), 2.80 (dd, *J =* 8.8, 2.2 Hz, 1H), 1.55 - 1.43 (m, 4H), 1.31 - 1.14 (m, 11H), 1.03 (s, 9H). ¹³C NMR (101 MHz, CDCl₃) δ 170.8, 170.6, 138.7, 138.0, 137.6, 137.1, 136.3, 135.8, 133.8, 133.6, 129.8, 129.7, 128.7, 128.6, 128.5, 128.4, 128.3, 128.2, 128.1, 128.0, 127.9, 127.8, 127.8, 127.7, 127.6, 127.4, 127.2, 100.0, 81.6, 81.5, 79.1, 77.5, 75.4, 75.2, 73.2, 73.2, 72.8, 67.2, 62.2, 54.2, 53.6, 53.2, 50.5, 50.2, 48.8, 47.3, 46.3, 30.6, 29.6, 29.4, 28.2, 27.8, 27.1, 27.0, 26.9, 26.8, 26.3, 22.5, 19.3. HRMS (ESI) m/z: [M+Na]⁺ calculated for C₇₅H₈₈N₂O₁₃SiNa 1275.5948, found 1275.5986.

### Intermediate 44: 2,3-di-O-benzyl-4-O-(2-N-acetyl-2-deoxy-3-O-benzyl-4-O-(8-N-benzyl(benzyloxycarbonyl)-1-octyl)-α-D-glucopyranosyl)-glucurono-cyclophellitol

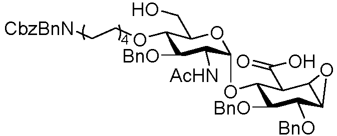

Intermediate 43 (0.60 g, 0.48 mmol) was dissolved in THF (7.2 ml, 0.05 M). Et₃N·3HF (0.29 ml, 1.8 mmol) was added and the reaction was stirred for 42 hours. The reaction mixture was diluted with DCM and water and the layers were separated. The water layer was extracted with DCM (4x) and the combined organic layers were dried over MgSO₄, filtrated and concentrated in vacuo. Column chromatography (EtOAc/PE, 2/3 -> 1/0, v/v, 1 % AcOH) yielded the product. (0.49 g, 0.48 mmol, quant.) ¹H NMR (400 MHz, CDCl₃) δ 7.40 - 7.11 (m, 25H), 6.27 (d, *J =* 9.7 Hz, 1H), 5.17 (d, *J =* 11.6 Hz, 2H), 4.96 (d, *J =* 10.8 Hz, 1H), 4.87 - 4.75 (m, 3H), 4.67 (d, *J =* 11.3 Hz, 1H), 4.56 (t, *J =* 10.8 Hz, 2H), 4.48 (d, *J* = 9.5 Hz, 2H), 4.22 (td, *J* = 10.0, 3.6 Hz, 1H), 3.99 - 3.87 (m, 3H), 3.79 - 3.66 (m, 4H), 3.60 - 3.39 (m, 6H), 3.28 - 3.14 (m, 4H), 2.89 (dd, *J* = 9.0, 2.1 Hz, 1H), 1.50 (d, *J* = 6.7 Hz, 4H), 1.20 (d, *J* = 19.5 Hz, 11H). ¹³C NMR (101 MHz, CDCl₃) δ 172.9, 172.6, 156.9, 156.3, 138.7, 138.0, 137.9, 137.7, 137.1, 137.0, 129.1, 128.7, 128.7, 128.7, 128.6, 128.5, 128.4, 128.3, 128.3, 128.2, 128.2, 128.0, 127.9, 127.6, 127.5, 127.5, 127.3, 125.4, 100.3, 81.6, 81.5, 79.3, 77.9, 77.5, 77.4, 77.2, 76.8, 76.8, 75.7, 75.0, 73.4, 73.2, 72.8, 67.2, 66.2, 64.3, 61.1, 54.5, 53.6, 53.4, 50.5, 50.2, 49.6, 47.3, 46.3, 43.4, 30.6, 29.8, 29.6, 29.4, 28.2, 27.8, 26.8, 26.2, 22.6. HRMS (ESI) m/z: [M+H]⁺ calc for C₅₉H₇₁N₂O₁₃ 1015.4951, found 1015.4954.

### Intermediate 45: 4-O-(2-N-acetyl-2-deoxy-4-O-(8-amino-1-octyl)-α-D-glucopyranosyl)-glucurono-cyclophellitol

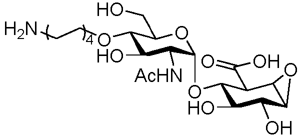

Intermediate 44 (0.10 g, 0.10 mmol) was dissolved in dioxane/water (10 ml, 0.01 M, 8/5) and flushed with N₂ for 5 min. 10% Pd/C (0.21 g, 0.2 mmol) was added and the suspension was flushed with N₂ for another 5 min. The N₂ balloon was replaced with an H₂ balloon and the solution was flushed with H₂ for 10 min and stirred at rt for 7 hours and 40 minutes. The H₂ balloon was replaced by an N₂ balloon, the reaction mixture was flushed for 5 min, filtrated over celite and concentrated under reduced pressure. Size exclusion chromatography over HW-40 eluting with H₂O 1% AcOH yielded a broad peak that was collected in three fractions. Based on NMR the pure product fraction was selected. (0.021 g, 0.041 mmol, 41%). ¹H NMR (500 MHz, MeOD) δ 5.11 (d, *J =* 3.8 Hz, 1H), 3.90 - 3.83 (m, 4H), 3.81 - 3.75 (m, 1H), 3.74 - 3.66 (m, 2H), 3.62 (dd, *J =* 11.6, 5.3 Hz, 1H), 3.58 - 3.50 (m, 1H), 3.42 (dd, *J =* 10.0, 8.2 Hz, 1H), 3.39 - 3.36 (m, 1H), 3.10 (dd, *J =* 10.0, 8.8 Hz, 1H), 3.01 (d, *J =* 3.6 Hz, 1H), 2.90 (t, *J =* 7.7 Hz, 2H), 2.74 (dd, *J* = 9.2, 2.1 Hz, 1H), 1.99 (s, 3H), 1.69 - 1.49 (m, 4H), 1.45 - 1.33 (m, 8H). ¹³C NMR (126 MHz, MeOD) δ 178.3 (weak), 174.0, 99.8, 80.2, 78.2, 77.8, 73.9, 73.7, 73.3, 73.2, 62.7, 57.0, 56.6, 56.1, 52.9, 40.8, 31.1, 29.9, 29.9, 28.7, 27.2, 27.2, 22.8. HRMS (ESI) m/z: [M+H]⁺ calc for C₂₃H₄₁N₂O₁₁ 521.2705, found 521.2703.

### Intermediate 46: (6-((tert-butyldimethylsilyl)oxy)-5-((4-methoxybenzyl)oxy)cyclohex-3-en-1-yl)methyl benzoate

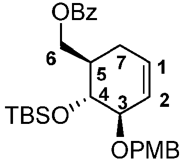

Alcohol S2 ( 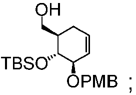 1.9 g, 5 mmol) was dissolved in anhydrous DCM (25 mL), the solution was then degassed with N₂ and cooled to 0 °C (ice bath). BzCl (872 µL, 7.5 mmol) and pyridine (604 µL, 7.5 mmol) were then added and the mixture was allowed to attain room temperature while stirring. Full conversion was observed after 1 h and 30 minutes, after which the reaction mixture was diluted with DCM (125 mL), transferred to a separatory funnel and washed two times with a saturated aqueous solution of NaHCO₃ (2x 50 mL). The organic layer was dried over Na₂SO₄, filtered and concentrated under reduced pressure to obtain a colourless oil. Purification by silica gel column chromatography (pentane/EtOAc 99:1→ 95:5) yielded the titled compound as a colourless oil (2.29 g, 4.75 mmol, 95% yield). ¹H NMR (400 MHz, CDCl₃): δ = 8.07 - 8.00 (m, 2H, 2H Ar, Bz), 7.58 - 7.46 (m, 1H, 1H Ar, Bz), 7.46 - 7.37 (m, 2H, 2H Ar, Bz), 7.32 - 7.24 (m, 2H, 2H Ar, PMB), 6.90 - 6.82 (m, 2H, 2H Ar, PMB), 5.80 - 5.66 (m, 2H, H-1/ H-2), 4.62 - 4.48 (m, 3H, C*H*H-6/ C*H*₂PhOMe), 4.41 - 4.32 (m, 1H, CH*H*-6), 3.95 - 3.87 (m, 2H, H-3/ H-4), 3.77 (s, 3H, OMe, PMB), 2.33 - 2.20 (m, 1H, C*H*H-7), 2.24 - 2.10 (m, 2H, CH*H*-7/ H-5), 0.89 (s, 9H, C(CH₃)₃,TBS) ppm. ¹³C NMR (101 MHz, CDCl₃): *δ* = 166.5, 159.1 (2C_{q}), 132.9 (CH Ar, Bz), 130.7 (C_{q}, PMB), 130.3 (C_{q}, PMB), 129.5 (2CH Ar, Bz), 129.3 (2CH Ar, PMB), 128.4 (2CH Ar, Bz), 127.8 (CH, C-2), 125.7 (CH, C-1), 113.7 (2CH Ar, PMB), 81.0 (CH, C-3), 72.3 (CH, C-4), 70.8 (CH₂, PMB), 65.4 (CH₂, C-6), 55.2 (OMe, PMB), 39.7 (CH, C-5), 27.9 (CH₂, C-7), 26.1 (C(CH₃)₃, TBS), 18.3 (C(CH₃)₃, TBS), -3.85, -4.9 (Si(CH₃)₂, TBS) ppm. HR-MS (ESI): *m*/*z* calcd for C₂₈H₃₈O₅SiNa⁺: 505.2381 [*M*+Na]⁺, found: 505.2378.

### Intermediate 47: 5,6-dihydroxycyclohex-3-en-1-yl)methyl benzoate

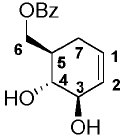

Intermediate 46 (6.76 g, 14 mmol) was dissolved in THF (70 mL) followed by addition of a 1 M solution of TBAF in THF (112 mL, 112 mmol). The reaction was left stirring for 1 h at room temperature, after which final conversion could be observed by TLC. The reaction mixture was quenched by addition of a saturated NaHCO₃ aqueous solution (100 mL) and Et₂O (900 mL), and transferred to a separatory funnel. The aqueous layer was the extracted twice with Et₂O (200 mL) The combined organic layers were then dried over Na₂SO₄, filtered and concentrated under reduced pressure. The crude was taken up in DCM (280 mL) and a pH 7.4 aqueous phosphate buffer (120 mL) was added. The solution was cooled on ice and 2,3-dichloro-5,6-dicyano-1,4-benzoquinone (DDQ) (19 g, 84 mmol) was subsequently added. After stirring at room temperature in darkness for 1 hour, full conversion was observed. The reaction was quenched by the addition of a saturated aqueous solution of NaHCO₃ (130 mL) and Na₂S₂O₃ (130 mL), diluted with DCM (550 mL) and transferred to a separatory funnel. The two layers were then separated and the aqueous layer was extracted twice with DCM (500 mL). The combined organic layers were dried over Na₂SO₄, filtered and concentrated under reduced pressure. The crude was purified by silica gel chromatography (pentane/EtOAc 20:1→1:1) and the title compound was collected as a thick colourless oil (2.73 g, 11.0 mmol, 79% yield over two steps). ¹H NMR (400 MHz, CDCl₃): *δ* = 8.07 - 8.00 (m, 2H, 2H Ar, Bz), 7.58 (ddt, *J =* 7.9, 7.0, 1.3 Hz, 1H, 1H Ar, Bz), 7.49 - 7.40 (m, 2H, 2H Ar, Bz), 5.75 - 5.66 (m, 1H, H-1), 5.64 - 5.55 (m, 1H, H-2), 4.84 (dd, *J =* 11.4, 4.2 Hz, 1H, C*H*H-6), 4.32 - 4.18 (m, 2H, H-3/CH*H*-6), 3.64 (s, 1H, 3-OH), 3.47 (dd, *J =* 11.1, 7.8 Hz, 1H, H-4), 2.70 (s, 1H, 4-OH), 2.34 - 2.18 (m, 2H, H-7), 2.18 - 2.02 (m, 1H, H-5) ppm. ¹³C NMR (101 MHz, CDCl₃): *δ* = 167. 6 (C_{q}, C=O, Bz), 133.6, 129.9, 128.6 (5CH Ar, Bz), 128.5 (CH, C-2), 127.5 (CH, C-1), 73.7 (CH, C-4), 73.5 (CH, C-3), 64.9 (CH₂, C-6), 39.6 (CH, C-5), 28.7 (CH₂, C-7) ppm. HR-MS (ESI): *m*/*z* calcd for C₁₄H₁₆O₄Na⁺: 271.0941 [*M*+Na]⁺, found: 271.0941.

### Intermediate 48: (4,5-dihydroxy-7-oxabicyclo[4.1.0]heptan-3-yl)methyl benzoate

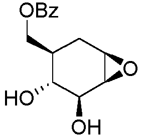

Intermediate 47 (0.51 g, 2.1 mmol) was dissolved in anhydrous DCM (20 mL) and the solution was cooled to -5 °C (ice/salt bath). mCPBA (<77% wt, 0.93 g, 4.1 mmol) was added at -5 °C, and the reaction mixture was stirred overnight while allowed to reach 4 °C. Upon full conversion the reaction was quenched by the addition of a saturated aqueous solution of Na₂S₂O₃ (50 mL), diluted with DCM (250 mL) and transferred to a separatory funnel. The two layers were separated and the organic solution was washed twice with a saturated aqueous solution of NaHCO₃ (50 mL) and brine solution. The organic layer was then dried using Na₂SO₄, filtered and concentrated under reduced pressure. The crude was purified by silica gel chromatography (pentane/EtOAc 2:3→1:4) to yield the title compound as a pale yellow oil (0.37 g, 1.4 mmol, 67% yield). ¹H NMR (400 MHz, CDCl₃): *δ* = 8.10 - 7.99 (m, 2H, 2H Ar, Bz), 7.63 - 7.54 (m, 1H, 1H Ar, Bz), 7.50 - 7.35 (m, 2H, 2H Ar, Bz), 4.72 (dd, *J* = 11.5, 4.0 Hz, 1H, C*H*H-6), 4.17 (dd, *J* = 11.4, 3.0 Hz, 1H, CH*H*-6), 3.91 (dd, *J =* 8.3, 1.9 Hz, 1H, H-3), 3.54 - 3.46 (m, 1H, H-4), 3.40 (ddd, *J* = 4.0, 1.8, 0.7 Hz, 1H, H-2), 3.32 (t, *J* = 4.3 Hz, 1H, H-1), 2.23 - 2.02 (m, 2H, H-7), 1.88 (m, *J* = 11.3, 7.0, 4.2, 2.9 Hz, 1H, H-5) ppm. ¹³C NMR (101 MHz, CDCl₃): δ = 167.7 (C_{q}, C=O, Bz), 134.0, 129.9, 128.6 (5CH Ar, Bz), 74.1 (CH, C-3), 70.2 (CH, C-4), 64.0 (CH₂, C-6), 56.5 (C-2), 53.1 (CH, C-1), 39.76 (CH, C-5), 26.3 (CH₂, C-7) ppm. HR-MS (ESI): *m*/*z* calcd for C₁₄H₁₆0sNa⁺: 287.0890 [*M*+Na]⁺, found: 287.0890.

### Intermediate 49: 4-((benzoyloxy)methyl)-7-oxabicyclo[4.1.0]heptane-2,3-diyl dibenzoate

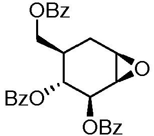

Intermediate 48 (832.2 mg, 0.315 mmol) was dissolved in dry pyridine (1.65 mL, 0.191 M) and cooled to 0 °C (ice bath). BzCl (99 wt%, 123.2 µL, 1.05 mmol, 3.5 eq.) was then added dropwise. The stirring continued at 0 °C for 2 h and 30 minutes, when full conversion could be observed. The reaction was then quenched by the addition of a saturated aqueous solution of NaHCO₃, while at 0 °C. The mixture was transferred to a separatory funnel, extracted two times with EtOAc (50 mL), then washed twice, first with H₂O (20 mL) then with brine solution (20 mL). The organic layers were then collected, dried over Na₂SO₄, filtered, and concentrated under reduced pressure. The crude was then purified by silica gel chromatography (pentane/EtOAc 9:1→2:3), affording the title compound as a dense milky oil (128.6 mg, 0.27 mmol, 84% yield). ¹H NMR (400 MHz, CDCl₃): δ = 8.09 - 7.47 (m, 6H, 6H Ar, Bz), 7.57 - 7.27 (m, 10H, 10H Ar, Bz), 5.78 (dd, *J =* 11.0, 8.9 Hz, 1H, H-4), 5.65 (dd, *J* = 8.9, 1.9 Hz, 1H, H-3), 4.39 (dd, *J* = 11.3, 4.0 Hz, 1H, C*H*H-6), 4.24 (dd, *J =* 11.3, 5.2 Hz, 1H, CH*H*-6), 3.66 (dd, *J =* 3.8, 2.1 Hz, 1H, H-2), 3.45 (t, *J* = 4.3 Hz, 1H, H-1), 2.54 - 2.35 (m, 2H, H-5/C*H*H-7), 2.26 - 2.13 (m, 1H, CH*H*-7) ppm. ¹³C NMR (101 MHz, CDCl₃): *δ* = 166.5, 166.0 (3C_{q}, 3C=O, Bz), 133.4, 133.3, 133.2, 130.1, 129.8, 128.5, 128.4 (15CH Ar, Bz), 74.4 (CH, C-3), 70.2 (CH, C-4), 64.1 (CH₂, C-6), 54.9 (CH, C-2), 52.9 (CH, C-1), 38.0 (CH, C-5), 26.6 (CH₂, C-7) ppm. HR-MS (ESI): *m*/*z* calcd for C₂₈H₂₅O₇⁺: 473.15948 [*M*+H]⁺, found 473.15910.

### Intermediate 50: 6-((benzoyloxy)methyl)-4-((5,6-bis(benzyloxy)-2-((naphthalen-2-ylmethoxy)methyl)cyclohex-3-en-1-yl)oxy)-3-hydroxycyclohexane-1,2-diyl dibenzoate

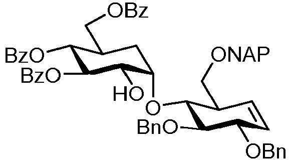

Intermediate 49 (2.28 g, 4.83 mmol) and 5,6-bis(benzyloxy)-2-((naphthalen-2-yloxy)methyl)cyclohex-3-en-1-ol (9.29 g, 19.3 mmol) were combined in a flask, co-evaporated thrice with dry distilled toluene and dissolved in dry DCM (19.3 mL). The mixture was cooled to 0 °C, and BF₃.OEt₂ (298 µL, 2.42 mmol) was added. The reaction was stirred for 16 h whilst allowing the cooling bath to slowly warm to room temperature. Next, the reaction was cooled to 0 °C and quenched with Et₃N (506 µL, 3.62 mmol). The mixture was diluted with sat. aq. NaHCO₃ (50 mL) and extracted with DCM (3 x 250 mL). The combined organic layers were dried over MgSO₄, filtered and concentrated. Flash purification by silica column chromatography (pentane:Et₂O, 10:1→1.5:1) with wet load (distilled toluene) afforded first the 5,6-bis(benzyloxy)-2-((naphthalen-2-yloxy)methyl)cyclohex-3-en-1-ol as white solid (5.85 g) and then desired product as an oil (3.45 g, 3.62 mmol, 75 % yield). ¹H NMR (400 MHz, CDCl₃): δ = 7.98 - 7.66 (m, 11H, 11CH Ar/toluene traces), 7.51 - 7.08 (m, 26H, 21CH Ar/toluene traces/CDCl₃), 5.81 - 5.68 (m, 3H, H-7, H-1, H-4'), 5.54 (dd, *J =* 9.4, 2.9 Hz, 1H, H-3'), 5.14 (d, *J =* 11.4 Hz, 1H, 1CH*H*Ar), 4.83 - 4.74 (d, *J =* 11.4 Hz, 1H, 1CH*H*Ar), 4.74 - 4.56 (m, 4H, 4CH*H*Ar), 4.29 (ddd, *J* = 5.3, 3.3, 1.6 Hz, 1H, H-2), 4.24 (dd, *J =* 11.3, 4.8 Hz, 2H, C*H*HOBz, H-9), 4.19 - 4.14 (m, 2H, H-2', CH*H*OBz), 4.13 - 4.06 (m, 1H, H-1'), 3.83 (dd, *J* = 9.9, 7.5 Hz, 1H, H-3), 3.73 (t, *J* = 9.5 Hz, 1H, H-4), 3.66 (d, *J* = 4.0 Hz, 2H, CH₂ONAP), 2.64 - 2.55 (m, 1H, H-5), 2.55 - 2.46 (m, 1H, H-5') 1.92 - 1.71 (m, 2H, CH₂, H-7') ppm. ¹³C NMR (101 MHz, CDCl₃): *δ* = 166.4, 166.0, 165.8, 139.0, 138.3, 135.6, 133.2 (7C_{q}), 133.1, 133.0, 132.9, 130.0, 129.8, 129.7, 129.5, 128.5, 128.4, 128.3, 128.0, 127.9, 127.8, 127.7, 127.6, 127.5, 126.6, 126.5, 126.3, 126.2, 126.0, 125.7 (32CH Ar), 85.0 (CH, C-3), 81.5 (CH, C-2), 78.2 (CH, C-1'), 77.9 (CH, C-4), 75.2 (CH₂Ar), 74.5 (CH, C-3'), 73.3 (CH₂Ar), 71.5 (CH₂Ar), 71.0 (CH, C-2'), 70.8 (CH, C-4'), 69.6 (CH₂, C-6'), 65.0 (CH₂, C-6), 44.2 (CH, C-5), 36.2 (CH, C-5'), 27.1 (CH₂, C-7') ppm. HR-MS (ESI): *m*/*z* calcd for C₆₀H₅₆O₁₁Na⁺: 975.37148 [*M*+Na]⁺, found 975.37129.

### Intermediate 51: 6-((benzoyloxy)methyl)-4-((5,6-bis(benzyloxy)-2-((naphthalen-2-ylmethoxy)methyl)cyclohex-3-en-1-yl)oxy)-3-oxocyclohexane-1,2-diyl dibenzoate

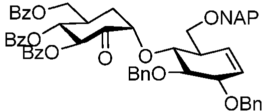

Intermediate 50 (0.84 g, 0.88 mmol) was coevaporated with dry distilled toluene, and dissolved in dry DCM (13 mL). At 0 °C, DMP (0.74 g, 1.8 mmol) was added portion-wise. The reaction mixture was allowed to gradually attain rt overnight. After 17 h, celite was added, and the solution was concentrated to dryness. Crude was purified by silica column chromatography with dry-load (pentane/Et₂O 10:1→1.5:1), affording the desired product as off-white solid (0.72 g, 0.76 mmol, 86% yield). ¹H NMR (400 MHz, CDCl₃): δ = 8.05 - 7.61 (m, 11H, 11CH Ar, Bz/NAP), 7.61 - 6.88 (m, 25H, 21H Ar/CDCl₃), 6.28 (dd, *J =* 10.2, 1.2 Hz, 1H, H-3'), 5.79 (dt, *J* = 10.3, 2.2 Hz, 1H, H-1), 5.69 (dt, *J =* 10.0, 2.1 Hz, 1H, H-7), 5.63 (t, *J =* 10.4 Hz, 1H, H-4'), 4.89 (dd, *J =* 21.1, 9.9 Hz, 2H, 2CH*H*Ar), 4.76 - 4.52 (m, 4H, 4CH*H*Ar), 4.38 (t, *J =* 3.0 Hz, 1H, H-1'), 4.26 (q, *J* = 2.8 Hz, 1H, H-2), 4.17 (ddd, *J* = 26.3, 15.0, 3.8 Hz, 2H, CH₂-6'), 3.95 - 3.82 (m, 2H, H-3, H-4), 3.66 (ddd, *J =* 13.6, 9.3, 3.3 Hz, 2H, CH₂, H-6), 2.96 - 2.84 (m, 1H, H-5'), 2.70 - 2.61 (m, 1H, H-5), 1.99 (dt, *J* = 15.0, 3.6 Hz, 1H, C*H*H-7'), 1.52 - 1.43 (m, 1H, CHH-7') ppm. ¹³C NMR (101 MHz, CDCl₃): δ = 199.1 (C=O, C-2'), 166.2, 165.3, 138.9, 138.3, 135.4 (5C_{q}), 133.4, 133.3 (2CH Ar), 133.2 (C_{q}), 133.2 (CH Ar), 133.1 (C_{q}), 130.1, 130.0, 129.9, 129.7 (4CH Ar), 129.2, 129.1 (2C_{q}), 129.0, 128.6, 128.5, 128.4, 128.3, 128.2, 128.0, 127.9, 127.7, 127.3, 126.9, 126.8, 126.4, 126.1, 125.9 (25CH Ar), 82.8 (CH, C-3), 81.2 (CH, C-1'), 80.8 (CH, C-2), 77.7 (CH, C-4), 77.6 (CH, C-3'), 74.0 (CH₂Ar), 73.4 (CH₂Ar), 72.8 (CH, C-4'), 71.6 (CH₂Ar), 69.6 (CH₂, C-6), 63.6 (CH₂, C-6'), 43.8 (CH, C-5), 35.2 (CH, C-5'), 30.4 (CH₂, C-7') ppm. HR-MS (ESI): m/z calcd for C₆₀H₅₅O₁₁⁺ 951.37389 [*M*+H]⁺, found 951.37383.

### Intermediate 52: 4-((5,6-bis(benzyloxy)-2-((naphthalen-2-ylmethoxy)methyl)cyclohex-3-en-1-yl)oxy)-6-(hydroxymethyl)cyclohexane-1,2,3-triol

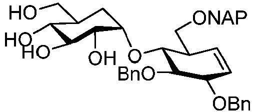

Intermediate 51 (0.39 g, 0.41 mmol) was coevaporated thrice with dry distilled toluene, dissolved in dry THF/MeOH (1:1 v/v, 4 mL) and the resulting solution was cooled to -60 °C. At - 60 °C, NaBH₄ (54 mg, 1.4 mmol) was added portion-wise. The reaction mixture was allowed to progressively warm up to 0 °C over 4 h, and stirred at this temperature overnight. The reaction was quenched by addition of acetone, then Celite was added and the mixture was concentrated to dryness. The crude was dry-loaded on a silica gel column and flash purification (pentane/EtOAc, 7:1→1:4) afforded the title product (0.24 mg, 0.38 mmol, 92%) as a pale yellow oil. ¹H NMR (600 MHz, CDCl₃): δ = 7.87 - 7.77 (m, 4H, 4H Ar), 7.52 - 7.25 (m, 16H, 13H Ar/CDCl₃), 5.79 (dt, J=10.6, 2.6 Hz, 1H, H-7), 5.68 (dt, *J =* 10.2, 2.0 Hz, 1H, H-1), 5.12 (d, *J =* 10.8 Hz, 1H, 1CH*H*Ar), 4.74 - 4.69 (m, 2H, 2CH*H*Ar), 4.65 - 4.58 (m, 3H, 3CH*H*Ar), 4.34 - 4.27 (m, 1H, H-2), 3.91 - 3.83 (m, 1H, H-4), 3.75 (dd, *J* = 9.9, 7.1 Hz, 1H, H-3), 3.56 - 3.51 (m, 2H, CH₂, H-6), 3.49 - 3.45 (m, 1H, H-1'), 3.42 - 3.36 (m, 2H, CH₂, H-6'), 3.25 (t, *J =* 9.4 Hz, 1H, H-3'), 3.09 (t, *J* = 9.9 Hz, 1H, H-4'), 2.85 - 2.76 (m, 1H, H-2'), 2.46 - 2.40 (m, 1H, H-5), 1.91 - 1.82 (m, 1H, H-5'), 1.30 (dt, *J =* 13.9, 3.3 Hz, 1H, ½ CH₂, CHH-7'), 0.51 (td, *J =* 13.9, 2.1 Hz, 1H, CHH-7') ppm. ¹³C NMR (151 MHz, CDCl₃): δ = 138.0, 137.5, 135.2, 133.3, 133.2 (5 C_{q}), 129.7 (CHsp², C-1), 128.8, 128.7, 128.6, 128.4, 128.3, 128.2, 128.0, 127.9, 127.8, 127.4, 126.4, 126.3 (17CH Ar), 126.1 (CHsp², C-7), 126.0 (CH Ar), 83.5 (CH, C-3), 80.8 (CH, C-2), 79.5 (CH, C-1'), 79.0 (CH, C-4), 76.8 (CH, C-4'), 75.9 (CH, C-2'/C-3'), 75.6 (CH, C-2'/C-3'), 75.5, 73.6, 71.6 (3CH₂Ar), 69.0 (CH₂, C-6), 66.8 (CH₂, C-6'), 45.8 (CH, C-5), 37.9 (CH, C-5'), 29.9 (CH₂, C-7') ppm. HR-MS (ESI): *m*/*z* calcd for C₃₉H₄₅O₈⁺: 641.31089 [*M*+H]⁺, found 641.31067

### Intermediate 53: 2-(((4,5-bis(benzyloxy)-6-((2,3,4-tris(benzyloxy)-5-((benzyloxy)methyl)cyclohexyl)oxy)cyclohex-2-en-1-yl)methoxy)methyl)naphthalene

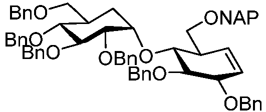

Intermediate 52 (0.37 g, 0.57 mmol) was dissolved in dry DMF (2.9 ml, 0.2 M) and cooled to 0 °C. At 0 °C, NaH (60% wt, 0.18 g, 4.6 mmol) was added portion-wise and the solution was stirred for 15 minutes. BnBr (0.57 ml, 4.5 mmol) was added, and the solution was allowed to slowly warm up to room temperature. After 18 h, reaction showed complete conversion. The reaction mixture was quenched at 0 °C with water, diluted with Et₂O (20 ml) and washed with brine (2x 5 ml). The organic layers were dried over Na₂SO₄, filtered, and concentrated to dryness. The crude was wet-loaded onto a silica gel column as concentrated solution in distilled toluene, and purified by silica gel column chromatography (pentane:Et₂O 50:1→1:1), affording the titled compound as a pale yellow oil (0.42 g, 0.42 mmol, 73%). ¹H NMR (500 MHz, CDCl₃): *δ* = 7.84 - 7.64 (m, 4H, 4H Ar), 7.49 - 7.11 (m, 36H, 28H Ar/toluene traces/CDCl₃), 5.77 (dt, *J =* 10.2, 2.3 Hz, 1H, H-7), 5.67 (dt, *J =* 10.0, 2.1 Hz, 1H, H-1), 5.08 - 4.79 (m, 4H, 4CHHAr), 4.75 - 4.52 (m, 10H, 10CHHAr), 4.26 - 4.22 (m, 2H, H-2/H-1'), 4.03 (t, *J =* 8.6 Hz, 1H, H-4), 3.98 (t, *J* = 9.3 Hz, 1H, H-3'), 3.89 (dd, *J =* 9.1, 6.9 Hz, 1H, H-3), 3.72 (dd, *J =* 9.0, 4.3 Hz, 1H, ½ CH₂), 3.68 - 3.60 (m, 1H), 3.54 (ddd, *J* = 11.0, 9.0, 3.7 Hz, 2H, CH₂), 3.46 (dd, *J =* 10.8, 8.9 Hz, 1H, H-4'), 3.30 (dd, *J* = 9.8, 2.9 Hz, 1H, H-2'), 3.19 (dd, *J* = 9.0, 2.5 Hz, 1H), 2.68 - 2.60 (m, 1H, H-5), 2.09 (dd, *J* = 14.7, 3.9 Hz, 1H, CHH-7'), 2.03 (t, *J* = 11.8 Hz, 1H, H-5'), 1.36 - 1.31 (m, 1H, CHH-7') ppm. ¹³C NMR (101 MHz, CDCl₃): *δ* = 139.4, 138.7, 138.6, 138.5, 135.9, 133.3, 133.0 (7C_{q}), 130.0 (CHsp², C-1), 128.5, 128.4, 128.3, 128.2, 128.0, 127.9, 127.8, 127.7, 127.6, 127.5, 127.4, 127.2, 127.0 (18CH Ar), 126.6 (CHsp², C-7), 126.2, 125.9, 125.7 (4CH Ar), 84.8 (CH, C-3), 83.8 (CH, C-3'), 83.0 (CH, C-2'), 81.0, 80.8 (2CH, C-2, C-4'), 75.4, 75.2 (2CH₂Ar), 73.2 (CH, C-1'), 73.0 (CH₂Ar), 72.4 (CH, C-4), 72.4, 71.6, 70.0, 69.9 (4CH₂Ar), 44.1 (CH, C-5) 37.1 (CH, C-5'), 27.9 (CH₂, C-7') ppm. HR-MS (ESI): *m*/*z* calcd for C₆₇H₆₉O₈⁺: 1001.49870 [*M*+H]⁺, found 1001.49801.

### Intermediate 54: (4,5-bis(benzyloxy)-6-((2,3,4-tris(benzyloxy)-5-((benzyloxy)methyl)cyclohexyl)oxy)cyclohex-2-en-1-yl)methanol

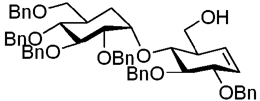

Intermediate 53 (0.42 g, 0.42 mmol) was dissolved in a mixture of DCM/H₂O (10:1 v/v, 8.4 mL) and stirred vigorously in darkness. DDQ (0.11 g, 0.47 mmol) was added at rt. After 90 minutes, reaction showed complete conversion. The reaction mixture was quenched with saturated NaHCO₃ aqueous solution (1 mL), and diluted with DCM (60 mL). The solution was washed with saturated NaHCO₃ aqueous solution (4 x 15 mL), then brine (15 mL). The organic layer was dried over Na₂SO₄, filtered, and concentrated to dryness. Purification by silica gel column chromatography (pentane/Et₂O 50:1→1:4), yielded the titled product as an oil (0.21 g, 0.24 mmol, 58% yield). ¹H NMR (500 MHz, CDCl₃): *δ* = 7.40 - 7.08 (m, 37H, H-1', 25H Ar, CDCl₃), 5.81 (dt, *J* = 10.2, 2.5 Hz, 1H, H-1), 5.65 (dt, *J* = 10.1, 2.2 Hz, 1H, H-7), 5.05 - 4.80 (m, 4H), 4.73 (d, *J* = 10.9 Hz, 1H), 4.48 - 4.40 (m, 3H, ), 4.25 - 4.18 (m, 1H, H-2), 4.04 - 3.89 (m, 4H, CHH-6, H-4, H-3, H-3'), 3.64 (dd, *J =* 11.0, 3.0 Hz, 1H, CHH-6), 3.55 (dd, *J =* 8.5, 2.7 Hz, 1H, CHH-6'), 3.37 - 3.18 (m, 3H, H-2', H-4', CHH-6'), 2.73 (s, 1H, OH), 2.57 - 2.49 (m, 1H, H-5), 2.35 (dt, *J =* 14.8, 3.3 Hz, 1H, CHH-7'), 2.16 - 2.05 (m, 1H, H-5'), 1.13 (t, *J* = 13.1 Hz, 1H, CHH-7') ppm. ¹³C NMR (126 MHz, CDCl₃): *δ* = 139.4, 139.2, 138.7, 138.6, 138.4, 138.1 (6C_{q}), 129.9, 128.5, 128.4, 128.3, 128.2, 128.0, 127.9, 127.8, 127.7, 127.6, 127.5, 127.4, 127.1, 126.8, 125.6 (30CH Ar), 84.1, 84.0 (2CH, C-4, C-3), 82.6 (CH, C-2'), 81.4, 80.7 (2CH, C-2, C-4'), 75.6, 75.3 (2CH₂Ar), 73.5 (CH, C-1'), 73.4, 73.3 (2CH₂Ar), 72.6 (CH-3' and CH₂Ar), 71.4 (CH₂Ar), 70.2 (CH₂, C-6'), 62.2 (CH₂, C-6), 45.2 (CH, C-5), 37.7 (CH, C-5'), 30.4 (CH/CH₃, grease), 28.3 (2CH₂, C-7') ppm. HR-MS (ESI): *m*/*z* calcd for C₅₆H₆₁O₈⁺: 861.43610 [*M*+H]⁺, found 861.43622.

### Intermediate 55: (4,5-bis(benzyloxy)-3-((2,3,4-tris(benzyloxy)-5-((benzyloxy)methyl)cyclohexyl)oxy)-7-oxabicyclo[4.1.0]heptan-2-yl)methanol

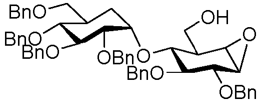

To a solution of intermediate 54 (208 mg, 0.24 mmol) in dry DCM (2.7 mL) at -20 °C, mCPBA (<75%, 664 mg, 1.88 mmol) was added portion-wise. The solution was allowed to warm up to 4 °C, and it was stirred for 18 hours at this temperature. Next, the solution was quenched with aqueous saturated NaHCO₃ solution and diluted with DCM. The organic layer was washed with further aqueous saturated NaHCO₃ solution, water and then brine. The combined organic layers were dried over Na₂SO₄, filtered and concentrated. Silica gel column chromatography of crude (P/Et₂O 50:1→1:4) afforded titled product (98 mg, 46%) as pale yellow oil and impure α-epoxide (10 mg, 5%). ¹H NMR (500 MHz, CDCl₃): *δ* = 7.59 - 6.87 (m, 30H, 30H Ar), 4.98 - 4.34 (m, 13H, 12CH*H*Ar, H-1'), 4.03 (dd, *J* = 11.0, 4.8 Hz, 1H, *CHH*-6), 3.97 - 3.92 (m, 1H, CH*H*-6), 3.92 - 3.55 (m, 4H), 3.52 - 3.40 (m, 1H, C*H*H-6'), 3.39 - 3.35 (m, 1H, H-7), 3.36 - 3.25 (m, 2H, CH*H*-6', CH), 3.15 (d, *J* = 3.9 Hz, 1H, H-1), 2.29 - 2.17 (m, 2H, H-5, C*H*H-7'), 2.14 - 1.97 (m, 1H, H-5'), 1.23 - 1.15 (m, 3H, CH*H*-7', grease) ppm. ¹³C NMR (101 MHz, CDCl₃): *δ* = 139.2, 138.8, 138.7, 138.4, 137.6 (6C_{q}), 133.8, 130.4, 129.9, 128.6, 128.5, 128.4, 128.3, 128.2, 128.1, 128.0, 127.9, 127.8, 127.7, 127.6, 127.4, 127.2, 126.6, 126.5 (30CH Ar), 85.0, 83.8, 82.5, 81.2, 80.2 (5CH), 75.4, 75.3 (2CH₂Ar), 73.5 (CH), 73.2, 72.7 (4CH₂Ar), 70.1 (CH₂, C-6'), 69.3 (CH), 62.2 (CH₂, C-6), 56.1 (CH, C-7, epoxide), 52.2 (CH, C-1, epoxide), 42.8 (CH, C-5), 37.6 (CH, C-5'), 27.8 (CH₂, C-7') ppm. HR-MS (ESI): *m*/*z* calcd for C₅₆H₆₁O₉⁺: 877.43101 [*M*+H]⁺, found 877.43007.

### Intermediate 56: 4,5-bis(benzyloxy)-3-((2,3,4-tris(benzyloxy)-5-((benzyloxy)methyl)cyclohexyl)oxy)-7-oxabicyclo[4.1.0]heptane-2-carboxylic acid

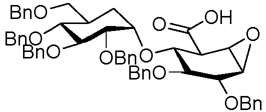

Intermediate 55 (83.3 mg, 0.0950 mmol) was co-evaporated twice with dry distilled toluene, and dissolved in DCM/*^{t}*BuOH/H₂O (4:5:1 v/v/v, 650 µL). The solution was cooled to 0 °C. TEMPO (2.96 mg, 0.0185 mmol) and BAIB (78.1 mg, 0.238 mmol) were added at 0 °C. The solution was kept at this temperature during the reaction. After 3 h, TLC showed full consumption of the starting material, and the reaction was quenched with a saturated aqueous Na₂S₂O₃ solution (1 mL), diluted with H₂O (5 mL) and washed with DCM (40 mL). The collected aqueous layer was washed thrice with DCM (40 mL). Combined organic layers were dried over Na₂SO₄, filtered, and volatiles were removed. Crude was purified by flash chromatography over silica gel (pentane/EtOAc 20:1→3:2 +0.5% AcOH), affording the desired product as a pale yellow oil (60.2 mg, 71% yield). ¹H NMR (400 MHz, CDCl₃): *δ* = 7.44 - 6.88 (m, 30, CH Ar), 4.95 - 4.28 (m, 13H, 12CHHAr, H-1'), 4.04 (t, *J* = 9.3 Hz, 1H, H-4), 3.98 - 3.82 (m, 2H, H-2/H-2'), 3.65 (dd, *J* = 9.1, 6.9 Hz, 1H, H-3), 3.48 (ddd, *J* = 23.0, 8.8, 3.6 Hz, 2H, CH₂-6'), 3.39 - 3.30 (m, 2H, H-7/H-4'), 3.26 (dd, *J =* 10.0, 3.2 Hz, 1H, H-3'), 3.16 (d, *J* = 3.8 Hz, 1H, H-1), 3.11 (dd, *J* = 9.5, 1.8 Hz, 1H, H-5), 2.17 (dt, *J =* 14.9, 3.8 Hz, 1H, CHH-7'), 2.05 - 1.90 (m, 1H, H-5'), 1.31 - 1.18 (m, 1H, CHH-7') .¹³C NMR (101 MHz, CDCl₃): *δ* = 173.6 (C_{q}, C-6), 139.2, 139.0, 138.7, 138.4, 137.4 (5 C_{q}), 128.6, 128.6, 128.5, 128.4, 128.3, 128.2, 128.1, 128.0, 127.9, 127.8, 127.7, 127.5, 127.4, 127.3, 127.2, 126.8, 126.7 (17 CH Ar), 83.7, 83.5 (CH, C-2/C-2'), 82.5 (CH, C-3'), 81.3 (CH, C-4'), 81.1, 79.6 (CH, C-2/C-2'), 75.5, 75.2 (2 CH₂Ar), 74.3 (CH, C-1'), 73.4, 73.3, 72.9, 72.7 (4CH₂Ar), 71.6 (CH, C-4), 70.3 (CH₂, C-6'), 53.9 (CH, C-7, epoxide), 52.9 (CH, C-1, epoxide), 48.3 (CH, C-5), 36.8 (CH, C-5'), 29.8 (CH₂, C-7') ppm. HR-MS (ESI): *m*/*z* calcd for C₅₆H₅₉O₁₀⁺: 891.41027 [*M*+H]⁺, found 891.40978.

### Intermediate 57: (4,5-bis(benzyloxy)-6-((3,4,5-tris(benzyloxy)-6-((benzyloxy)methyl)tetrahydro-2H-pyran-2-yl)oxy)cyclohex-2-en-1-yl)methyl tert-butyl carbonate

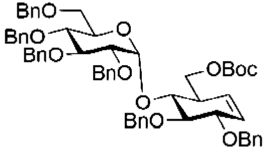

(4,5-Bis(benzyloxy)-6-((3,4,5-tris(benzyloxy)-6-((benzyloxy)methyl)tetrahydro-2H-pyran-2-yl)oxy)cyclohex-2-en-1-yl)methanol (0.12 g, 0.13 mmol) was co-evaporated thrice with anhydrous toluene and subsequently dissolved in 0.9 mL anhydrous THF. Boc₂O (0.062 mL, 0.26 mmol) and DMAP (0.013 g, 0.11 mmol) were added and the reaction was stirred overnight. The reaction mixture was quenched and diluted with H₂O. The water layer was extracted thrice with Et₂O, the combined organic layers were washed with sat. NH₄Cl (aq), sat. NaHCO₃ (aq), brine, dried over MgSO₄, filtered and concentrated *in vacuo.* The crude product was used without purification in the next step.

### Intermediate 58: (4,5-bis(benzyloxy)-3-((3,4,5-tris(benzyloxy)-6-((benzyloxy)methyl)tetrahydro-2H-pyran-2-yl)oxy)-7-oxabicyclo[4.1.0]heptan-2-yl)methanol 20

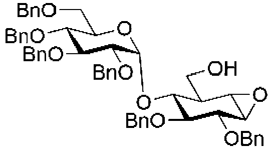

Intermediate 57 (crude, 0.13 mmol) was dissolved in 0.65 mL AcOH. NIS (0.058 g, 0.26 mmol) was added and the reaction was stirred overnight. The reaction mixture was diluted with Et₂O, quenched with Et₃N, washed with sat. NaHCO₃ (aq), 10% Na₂S₂O₃, brine, dried over MgSO₄, filtered and concentrated in vacuo. The crude product was used without further purification in the next step. Crude was next dissolved in 0.65 mL MeOH/DCM (1/1, v/v, 0.65 mL) and NaOMe (5.4M in MeOH) was added catalytically. The reaction was stirred overnight. The reaction mixture was diluted with EtOAc, washed with H₂O, brine, dried over MgSO₄, filtered and concentrated in vacuo. The crude product was purified using flash silica column chromatography (pentane/EtOAc, 10:1→5:1) to obtain the intermediate (0.077 g, 0.088 mmol, 67% over 3 steps). ¹H NMR (400 MHz, CDCl₃): *δ* = 7.38 - 7.02 (m, 30H), 5.69 (d, *J* = 3.9 Hz, 1H), 4.88 (t, *J* = 11.4 Hz, 2H), 4.82 - 4.65 (m, 4H), 4.60 - 4.39 (m, 6H), 4.03 - 3.85 (m, 6H), 3.73 - 3.64 (m, 2H), 3.63 - 3.57 (m, 1H), 3.52 - 3.45 (m, 2H), 3.41 - 3.34 (m, 1H), 3.15 (d, *J* = 3.8 Hz, 1H), 2.60 - 2.52 (m, 1H), 2.28 - 2.19 (m, 1H) ppm. ¹³C NMR (101 MHz, CDCl₃): *δ* = 139.1, 138.7, 138.1, 137.6, 137.5, 129.1, 128.6, 128.5, 128.4, 128.4, 128.3, 128.3, 128.2, 128.1, 128.1, 128.0, 128.0, 127.9, 127.8, 127.6, 127.5, 127.1, 126.4, 125.4, 97.3, 84.8, 81.9, 80.1, 79.2, 78.0, 75.5, 75.1, 73.7, 73.6, 73.1, 72.8, 71.4, 70.0, 68.7, 61.8, 56.5 (CH, epoxide), 52.4 (CH, epoxide), 43.2 ppm. HR-MS: *m*/*z* calcd for C₅₅H₆₂O₁₀N⁺: 896.4368 [*M*+NH₄]⁺, found 896.4368

### Intermediate 59: 4,5-bis(benzyloxy)-3-((3,4,5-tris(benzyloxy)-6-((benzyloxy)methyl)tetrahydro-2H-pyran-2-yl)oxy)-7-oxabicyclo[4.1.0]heptane-2-carboxylic acid

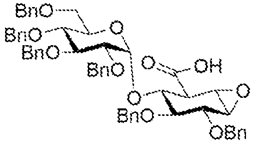

Intermediate 58 (0.07 g, 0.08 mmol) was dissolved in DCM/*^{t}*BuOH/H₂O (4:4:1, v/v/v, 2.65 mL) and cooled down to 0 °C. TEMPO (0.0025 g, 0.016 mmol) and BAIB (0.064 g, 0.20 mmol) were added and the reaction was left to stir at 0 °C overnight. After TLC analysis showed full conversion of the starting material the reaction was quenched by adding solid Na₂S₂O₃. The mixture was then further diluted with H₂O, acidified with AcOH and extracted with DCM thrice. The combined organic layers were washed with brine, dried over MgSO₄, filtered and concentrated *in vacuo.* The crude product was purified with flash silica column chromatography (pentane/acetone, 10:1→20:3 with 1% AcOH, v/v), to obtain the title compound (0.058 g, 0.065 mmol, 82%). ¹H NMR (400 MHz, CDCl₃): *δ* = 7.34 - 7.08 (m, 30H), 5.46 (d, *J* = 3.7 Hz, 1H), 4.97 (d, *J* = 11.6 Hz, 1H), 4.89 (d, *J =* 10.9 Hz, 1H), 4.82 - 4.67 (m, 4H), 4.65 - 4.59 (m, 2H), 4.52 - 4.48 (m, 2H), 4.46 - 4.41 (m, 2H), 4.19 (t, *J* = 9.5 Hz, 1H), 3.94 (t, *J* = 9.4 Hz, 1H), 3.87 - 3.79 (m, 2H), 3.77 - 3.53 (m, 5H), 3.48 (dd, *J* = 9.9, 3.7 Hz, 1H), 3.21 - 3.18 (m, 1H), 3.09 (d, *J* = 3.6 Hz, 1H), 2.99 (dd, *J =* 9.3, 2.0 Hz, 1H) ppm. ¹³C NMR (101 MHz, CDCl₃): *δ* = 174.6, 139.1, 138.8, 138.4, 138.2, 138.0, 137.5, 129.2, 128.6, 128.5, 128.4, 128.3, 128.3, 128.2, 128.1, 128.1, 128.0, 128.0, 127.8, 127.8, 127.7, 127.6, 127.5, 127.2, 127.2, 125.4, 97.8, 83.0, 81.7, 79.7, 79.0, 77.7, 75.7, 75.1, 74.8, 74.5, 73.6, 73.3, 72.8, 71.2, 68.5, 54.4 (CH, epoxide), 53.7 (CH, epoxide), 48.8 ppm. HR-MS: *m*/*z* calcd for C₅₅H₆₀O₁₁N⁺: 910.4161 [*M*+NH₄]⁺, found 910.4161.

### Example 2: 4-O-(2-N-acetyl-2-deoxy-D-glucopyranosyl)-glucurono-cyclophellitol (Compound 2)

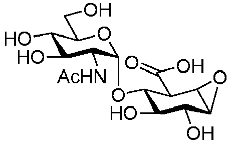

Ammonia (10 ml) was condensed at -70 °C. Sodium (17 mg, 0.72 mmol) was added and the solution turned blue. Intermediate 11 (14 mg, 0.018 mmol), dissolved in anhydrous THF (2ml) and t-BuOH (0.068 ml, 0.72 mmol) was added. After stirring at -60°C for 15 minutes the blue color faded and more sodium (12 mg, 0.51 mmol) was added. After another 30 minutes the blue color faded again the reaction was quenched with NH₄Cl (66 mg, 1.23 mmol) The ammonia was evaporated, water was added and the compound was desalted by size exclusion over HW-40 (1% AcOH in water). Lyophilization afforded the compound as a white solid. (6.42 mg, 0.016 mmol, 91%).
¹H NMR (500 MHz, D₂O) δ 5.30 (d, *J* = 3.7 Hz, 1H), 3.97 - 3.93 (m, 1H), 3.93 - 3.88 (m, 4H), 3.85 - 3.81 (m, 2H), 3.64 - 3.56 (m, 3H), 3.32 - 3.29 (m, 1H), 2.99 (d, *J =* 9.6 Hz, 1H), 2.13 (s, 3H). ¹³C NMR (126 MHz, D₂O) δ 177.6, 174.4, 97.7, 76.2, 75.0, 72.0, 71.5, 71.0, 69.7, 60.1, 56.2, 55.5, 54.0, 50.8, 22.0. HRMS (ESI) m/z: [M+Na]⁺ calculated for C₁₅H₂₃NO₁₁Na 416.1163, found 416.1164.

### Example 3: 4-O-(2-N-acetyl-2-deoxy-6-O-sulfo-D-glucopyranosyl)-glucurono-cyclophellitol (Compound 3)

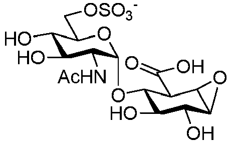

Ammonia was condensed at -60°C under an inert atmosphere and Na (37 mg, 1.6 mmol) was added. The solution turned dark blue. Intermediate 12 (35 µmol, 33 mg) was dissolved in THF/t-BuOH (0.6 ml, 2/3, v/v) and added to the solution. After stirring for 45 minutes the reaction was quenched with NH₄Cl (0.10 g, 1.92 mmol). Ammonia was evaporated at room temperature, diluted in water and concentrated under reduced pressure. The compound was desalted by size exclusion over HW-40 (1% AcOH in water). Lyophilization afforded the compound as a white solid. (14.9 mg, 0.03 mmol, 87% over 2 steps).
¹H NMR (400 MHz, D₂O) δ 5.22 (d, *J* = 3.7 Hz, 1H), 4.33 (dd, *J* = 11.0, 2.2 Hz, 1H), 4.15 (dd, J = 11.1, 2.1 Hz, 1H), 3.88 - 3.75 (m, 4H), 3.70 (dd, *J* = 10.7, 9.1 Hz, 1H), 3.56 (dd, *J* = 10.1, 9.1 Hz, 1H), 3.50 - 3.44 (m, 2H), 3.15 (d, *J* = 3.7 Hz, 1H), 2.83 (dd, *J =* 9.7, 1.9 Hz, 1H), 1.99 (s, 3H). ¹³C NMR (101 MHz, D₂O) δ 180.6, 177.7, 174.3, 97.4, 76.5, 74.4, 71.6, 70.8, 69.9, 69.0, 66.3, 56.1, 55.3, 53.7, 50.9, 22.7 (AcOH), 21.9. HRMS (ESI) m/z: [M+2H]⁺ calculated for C₁₅H₂₄NO₁₄S 474.09120 found 474.09129

### Example 4: 4-O-(2-deoxy-D-glucopyranosyl)-glucurono-cyclophellitol (Compound 4)

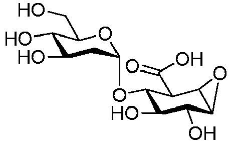

Ammonia (8 ml) was condensed at -70 °C. Sodium (31 mg, 1.35 mmol) was added and the solution turned blue. Intermediate 18 (50 mg, 0.068 mmol), dissolved in anhydrous THF (2ml) and t-BuOH (0.16 ml, 1.69 mmol) was added. After stirring at -60 °C for 45 minutes the reaction was quenched with NH₄Cl (109 mg, 2.04 mmol) The ammonia was evaporated, water was added and the compound was desalted by size exclusion over HW-40 (1% AcOH in water). Lyophilization afforded the compound as a white solid. (22.7 mg, 0.068 mmol, quant.).
¹H NMR (500 MHz, D₂O) δ 5.31 (d, *J =* 3.7 Hz, 1H), 3.88 (ddd, *J =* 11.9, 9.1, 4.9 Hz, 1H), 3.84 - 3.76 (m, 4H), 3.67 (dt, *J* = 10.0, 3.1 Hz, 1H), 3.48 - 3.45 (m, 1H), 3.43 (dd, *J* = 10.2, 8.7 Hz), 3.35 (dd, *J =* 10.0, 9.2 Hz, 1H), 3.18 (d, *J =* 3.7 Hz, 1H), 2.80 (dd, *J =* 9.8, 1.9 Hz, 1H), 2.19 (ddd, *J =* 13.3, 5.0, 1.3 Hz, 1H), 1.64 (ddd, *J =* 13.2, 11.9, 3.9 Hz, 1H). ¹³C NMR (126 MHz, D₂O) δ 179.8, 97.7, 75.7, 73.7, 71.6, 70.4, 70.3, 67.6, 59.7, 55.6, 54.7, 50.4, 36.4. HRMS (ESI) m/z: [M+H]⁺ calculated for C₁₃H₂₄O₁₀ 354.13947 found 354.13937.

### Example 5: 4-O-(2-amino-2-deoxy-D-glucopyranosyl)-glucurono-cyclophellitol (Compound 5)

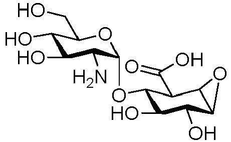

Anhydrous THF (1 ml). and *t*-BuOH (0.32 ml, 3.4 mmol) were added to intermediate 19 (crude, 0.068 mmol). The flask was cooled to -70°C and ammonia (5 ml) was condensed directly in the flask. Sodium (63 mg, 2.72 mmol) was added and the solution turned blue. After stirring at - 60°C for 45 minutes the reaction was quenched with NH₄Cl (0.18 g, 3.4 mmol) The ammonia was evaporated, water was added and the compound was desalted by size exclusion over HW-40 (1% AcOH in water). Lyophilization afforded the compound as a white solid. (12.1 mg, 0.035 mmol, 51% over 2 steps.)
¹H NMR (500 MHz, D₂O) δ 5.49 (d, *J* = 3.8 Hz, 1H), 3.93 - 3.85 (m, 3H), 3.83 (d, *J* = 2.9 Hz, 2H), 3.75 (dt, *J =* 10.1, 2.9 Hz, 1H), 3.58 - 3.50 (m, 3H), 3.30 (dd, *J* = 10.7, 3.8 Hz, 1H), 3.21 (d, *J* = 3.7 Hz, 1H), 2.90 (dd, *J* = 9.8, 1.8 Hz, 1H). ¹³C NMR (126 MHz, D₂O) δ 177.7, 96.1, 76.4, 75.2, 72.1, 71.4, 69.7, 69.2, 59.7, 56.2, 55.4, 54.4, 50.9. HRMS (ESI) m/z: [M+H]⁺ calculated for C₁₃H₂₂NO₁₀ 352.1235, found 352.1238.

### Example 6: 4-O-(2-N-azidoacetyl-2-deoxy-D-glucopyranosyl)-glucurono-cyclophellitol (Compound 6)

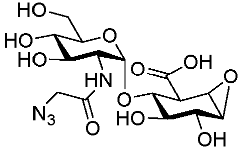

Azidoacetic acid (50 µmol) was dissolved in DMF (1 ml), 2,3,4,5,6-pentafluorophenol (46 mg, 0.26 mmol), Et₃N (20 µl, 0.26 mmol) and DIC (7.8 µl, 50 µmol) were added and the mixture was stirred for 90 minutes. Of this stock solution 1.5 eq compared to amine (0.25 ml, 13 µmol) was added to Compound 5 and stirred overnight. LC-MS indicated full conversion and the product was purified over HW-40 eluting with AcOH in water (1%, v/v). The fractions were concentrated under reduced pressure and lyophilized to yield the product as a white solid (0.45 mg, 1.04 µmol, 12%).
¹H NMR (600 MHz, D₂O) δ 5.17 (d, *J* = 3.8 Hz, 1H), 4.03 (d, *J* = 1.1 Hz, 2H), 3.89 (dd, *J* = 10.7, 3.8 Hz, 1H), 3.79 - 3.71 (m, 5H), 3.69 (dt, *J =* 10.1, 3.1 Hz, 1H), 3.50 - 3.45 (m, 3H), 3.19 - 3.13 (m, 1H), 2.87 (dd, *J =* 9.5, 1.9 Hz, 1H). ¹³C NMR (151 MHz, D₂O) δ 178.3, 171.6, 98.5, 77.0, 76.0, 72.9, 72.2, 71.7, 70.4, 60.9, 57.0, 56.4, 54.9, 52.7, 51.5. HRMS (ESI) m/z: [M+H]⁺ calculated for C₁₅H₂₃N₄O₁₁ 435.1358, found 435.1358.

### Example 7: 4-(8-Cy5-aminooctyl)-glucurono-cyclophellitol (Compound 8)

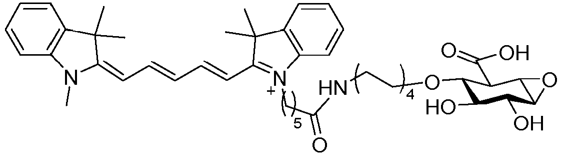

Intermediate 26 (10.3 mg, 32.5 µmol) was dissolved in anhydrous DMF (0.5 mL), followed by the addition of Cy5-NHS ester (30.0 mg, 48.8 µmol) and DIPEA (10.6 µl, 65.0 µmol). After the reaction mixture was stirred for 18 hours, it was purified by preparative RP-HPLC (linear gradient, solutions used: A: 50 mM AcOH in H₂O, B: CH₃CN) and lyophilized. The product was obtained as blue solid. (3.50 mg, 4.48 µmol, 14%)
¹H NMR (500 MHz, MeOD): δ 8.25 (t, *J* = 13.0 Hz, 2H), 7.49 (d, *J* = 7.5 Hz, 2H), 7.44 - 7.38 (m, 2H), 7.32 - 7.24 (m, 4H), 6.64 (t, *J =* 12.4 Hz, 1H), 6.29 (dd, *J* = 13.7, 6.5 Hz, 2H), 4.11 (t, *J* = 7.4 Hz, 2H), 3.79 - 3.71 (m, 1H), 3.69 (d, *J* = 8.2 Hz, 1H), 3.67 - 3.59 (m, 4H), 3.48 (t, *J* = 9.8 Hz, 1H), 3.39 (m, 1H), 3.27 (dd, *J =* 10.1, 8.3 Hz, 1H), 3.12 (t, *J =* 7.0 Hz, 2H), 2.99 (d, *J* = 3.6 Hz, 1H), 2.69 (dd, *J* = 8.1, 1.5 Hz, 1H), 2.20 (t, *J* = 7.2 Hz), 1.87 - 1.79 (m, 2H), 1.73 (s, 12H), 1.71 - 1.66 (m, 2H), 1.47 - 1.24 (m, 14H) ¹³C NMR (126 MHz, MeOD): δ 155.5, 144.3, 143.6, 142.7, 142.5, 129.8, 129.7, 126.7, 126.3, 123.4, 123.3, 112.1, 111.9, 104.4, 78.2, 77.9, 73.9, 73.0, 56.9, 56.7, 52.0, 50.6, 50.5, 44.8, 40.4, 36.7, 31.6, 31.3, 30.5, 30.3, 30.3, 28.2, 28.0, 27.9, 27.8, 27.4, 27.0, 26.6 HRMS (ESI) m/z: [M]⁺ calculated for C₄₇H₆₄N₃O₇ 782.4739, found 782.4772.

### Example 8: 4-O-(2-N-acetyl-2-deoxy-4-O-(8-amino-Cy5-1-octyl)-α-D-glucopyranosyl)-glucurono-cyclophellitol (Compound 9)

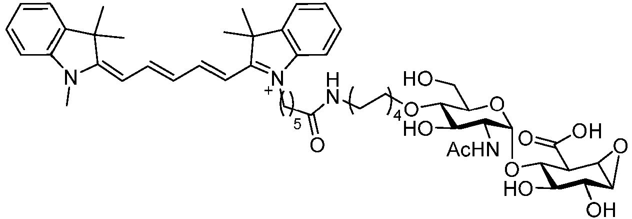

Cy5 carboxylic acid (11.6 mg, 22.3 µmol) was dissolved in DMF (0.25 ml). DIPEA (12 µl, 70 µmol), and pentafluorophenyl trifluoroacetate (3.8 µl, 22.3 µmol) were added and the mixture was stirred for one hour. LC-MS indicated the presence of starting material so more DIPEA (6 µl, 35 µmol) and pentaflurophenyl trifluoroacetate (3.8 µl, 22.3 µmol) were added. After stirring for 30 minutes water (2 µl) and DMF (0.25 ml) were added and the solution was added to intermediate 45 (12.2 mg, 23µmol). The reaction was stirred overnight and the product was purified on semi-preparative HPLC eluting with a linear gradient of solution A (MeCN) in solution B (50mM AcOH in H₂O). The fractions were concentrated under reduced pressure, coevaporated with water, diluted with water and lyophilized to yield the product as a blue solid. (2,88 mg, 2,82 µmol, 13%).
¹H NMR (850 MHz, CD₃CN) δ 8.02 (t, *J* = 13.1 Hz, 2H), 7.47 - 7.42 (m, 2H), 7.40 - 7.34 (m, 2H), 7.26 - 7.19 (m, 4H), 6.50 (t, *J* = 12.4 Hz, 1H), 6.16 (t, *J* = 13.1 Hz, 2H), 5.01 (d, *J* = 3.9 Hz, 1H), 3.96 (t, *J* = 7.5 Hz, 2H), 3.74 - 3.65 (m, 7H), 3.64 - 3.58 (m, 2H), 3.53 (dd, *J* = 12.0, 5.2 Hz, 1H), 3.49 (s, 5H), 3.38 (dd, *J* = 10.0, 8.4 Hz, 1H), 3.35 (dd, *J* = 3.8, 2.1 Hz, 1H), 3.10 (t, *J* = 9.6 Hz, 1H), 3.06 - 2.99 (m, 3H), 2.69 (dd, *J* = 9.3, 2.1 Hz, 1H), 2.08 (t, *J =* 7.3 Hz, 3H), 1.90 (s, 3H), 1.74 - 1.69 (m, 2H), 1.62 (s, 12H), 1.58 - 1.52 (m, 2H), 1.50 - 1.42 (m, 2H), 1.37 - 1.30 (m, 4H), 1.20 (s, 8H). ¹³C NMR (214 MHz, CD₃CN) δ 175.5, 174.8, 174.4, 174.2, 154.6, 143.9, 143.2, 142.2, 142.1, 129.6, 126.0, 125.4, 123.2, 123.1, 112.0, 111.8, 103.9, 103.8, 98.3, 98.3, 79.5, 76.8, 76.7, 74.1, 72.5, 72.2, 72.1, 61.6, 57.1, 56.3, 56.3, 55.1, 51.7, 50.0, 50.0, 44.7, 40.0, 36.5, 31.8, 30.5, 29.9, 29.7, 27.7, 27.6, 27.5, 27.3, 26.7, 26.3, 26.1, 23.0. HRMS (ESI) m/z: [M]⁺ calculated for C₅₅H₇₇N₄O₁₂ 985.5523, found 985.5533.

### Example 9: 4,5-dihydroxy-3-((2,3,4-trihydroxy-5-(hydroxymethyl)cyclohexyl)oxy)-7-oxabicyclo[4.1.0]heptane-2-carboxylic acid (Compound 12)

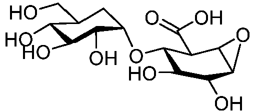

Intermediate 52 (59 mg, 67 µmol) was dissolved in a mixture of MeOH/*^{t}*BuOH/dioxane (2:1:2 v/v/v, 6.7 ml) under Nitrogen, then Pd(OH)₂/C (20% wt, 61 mg, 87 µmol) was added. Under vigorous stirring, the mixture was flushed with a H₂ balloon. After stirring for 5 h under H₂ atmosphere, the mixture was filtered over a Whatman pad, concentrated, and purified by reverse-phase HPLC chromatography with HILIC column (gradient: 70-65% eluent B for 9 minutes, then 65-50% eluent B for 9 minutes). After lyophilisation, the desired product (7.3 mg, 21 µmol, 31%) was obtained as white powder. ¹H NMR (400 MHz, D₂O): *δ* = 3.91 - 3.82 (m, 1H, H-1'), 3.71 (d, *J =* 8.4 Hz, 1H, H-2), 3.58 (dd, *J =* 11.4, 4.8 Hz, 1H, CHH-6'), 3.50 (dd, *J =* 11.4, 3.3 Hz, 1H, CHH-6'), 3.47 - 3.25 (m, 5H, H-2', H-3, H-3', H-4, H-7), 3.11 (dd, *J* = 10.8, 9.1 Hz, 1H, H-4'), 3.03 (d, *J* = 3.7 Hz, 1H, H-1), 2.67 (dd, *J* = 9.6, 1.7 Hz, 1H, H-5), 1.79 (dt, *J* = 14.5, 3.7 Hz, 1H, CHH-7'), 1.71 - 1.60 (m, 1H, H-5'), 1.14 (td, *J* = 14.1, 1.7 Hz, 1H, CHH-7') ppm. ¹³C NMR (101 MHz, D₂O): *δ* = 178.0 (C_{q}, C-6), 80.2 (CH, C-1'), 78.2, 76.5, 75.1 (3 CH, C-3, C-4, C-3'), 74.2 (CH, C-2'), 72.8 (CH, C-4'), 70.9 (CH, C-2), 62.0 (CH₂, C-6'), 56.2 (CH, C-7, epoxide), 55.3 (CH, C-1, epoxide), 51.6 (CH, C-5), 38.2 (CH, C-5'), 28.8 (CH₂, C-7') ppm. HR-MS (ESI): *m*/*z* calcd for C₁₄H₂₃O₁₀⁺: 351.12857 [*M*+H]⁺, found 351.12845.

### Example 10: 4,5-dihydroxy-3-((3,4,5-trihydroxy-6-(hydroxymethyl)tetrahydro-2H-pyran-2-yl)oxy)-7-oxabicyclo[4.1.0]heptane-2-carboxylic acid (Compound 13)

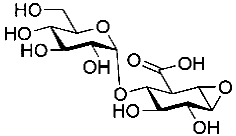

Intermediate 59 (0.027 g, 0.030 mmol) was co-evaporated with anhydrous toluene thrice and dissolved in 1.25 mL anhydrous THF. *t*-BuOH ( 0.17 mL, 1.8 mmol) was added to this solution. Liquid ammonia was prepared fresh by cooling an empty round bottom flask to -60 °C while maintaining a gentle gaseous flow of ammonia in the flask. Upon reaching the desired amount of liquid ammonia the flask was sealed off and placed under nitrogen atmosphere. Solid sodium (0.042 g, 1.8 mmol) was added to the liquid ammonia and the resulting solution turned blue. Compound **21** was added to the blue solution and the reaction was left to stir for 10 minutes. The reaction was quenched with AcOH (0.1 mL, 1.8 mmol) in H₂O (1 mL) after which the blue color disappeared. The reaction flask was transferred to a warm water bath and stirred for 30 minutes until all ammonia had evaporated. The remaining solution was concentrated in vacuo. The crude residue was purified using gel filtration to obtain compound 3 (0.0088 g, 0.024 mmol, 80%). ¹H NMR (850 MHz, D₂O): *δ* = 5.23 (d, *J* = 4.0 Hz, 1H), 3.87 (d, *J =* 8.7, 0.8 Hz, 1H), 3.84 - 3.77 (m, 3H), 3.74 - 3.68 (m, 2H), 3.59 - 3.57 (m, 1H), 3.54 (dd, *J* = 9.9, 4.0 Hz, 1H), 3.51 - 3.49 (m, 1H), 3.43 (t, *J =* 10.2, 9.2 Hz, 1H), 3.20 (d, *J =* 3.7 Hz, 1H), 2.88 (dd, *J* = 9.7, 1.9 Hz, 1H) ppm. ¹³C NMR (214 MHz, D₂O): *δ* = 177.7, 99.6, 76.6, 76.2, 73.0, 71.8, 71.7, 70.7, 69.2, 60.0, 56.2 (CH, epoxide), 55.3 (CH, epoxide), 50.9 ppm. HR-MS: *m*/*z* calcd for C₁₃H₂₄O₁₁N ⁺: 370.1344 [*M*+NH₄]⁺, found 370.1344.

### Example 11: Inhibition trials

Inhibition trials were conducted using a competitive activity-based protein profiling (cABPP) assay, which measures the ability of inhibitors to abrogate enzyme labelling by the fluorescent pan-β-glucuronidase activity-based probe (ABP) **7** (Wu et al., Nat Chem Biol, 13, 867-873 (2017)).

Using cABPP Assay 1, we found pseudo-monosaccharide **1** to be an ineffective HPSE inhibitor up to 10 µM, whilst compounds **2, 3** and **4** all inhibited HPSE at nanomolar concentrations (Figure 5a). Compound **3** showed greater potency against HPSE than **2** and **4,** likely due to electrostatic interactions from its 6O'-sulfate. HPSE inhibition by **4** indicates that some non-natural modifications of the 2' position can be tolerated, although positively charged 2' groups such as -NH³⁺ (as per compound **5**), or sterically larger 2' groups such as -NHCH₂N₃ (as per compound **6**) do not inhibit HPSE well, suggesting they are poorly accommodated by the enzyme active site. As expected, no compounds tested inhibited proHPSE, consistent with the requirement of an extended binding-cleft for pseudo-disaccharide binding. Similarly, 1, but not **2-6** effectively inhibited the *bona fide exo*-β-glucuronidase EcGUSB. Inhibition of BpHep and AcGH79 followed similar patterns to those observed for HPSE, with all compounds apart from **5** showing activity against one or both of these enzymes (Figure 6a).

Competitive activity of compounds **2, 12** and **13** were investigated using cABPP Assay 2. The inhibitory potency of **12** was assessed against recombinant *endo*-β-D-glucuronidase (AcGH79, hHPSE) via in-gel competitive profiling using as fluorescent read-out Cy5-tagged β-D-glucuronidase ABP; **12** displayed low micromolar inhibition of AcGH79 (IC₅₀: 1.2 ± 0.3 µM, Figure 6b) at the tested enzyme concentration, suggesting that **12** is able to partially block the *endo*-activity of AcGH79.

To gain insight into HPSE structure-activity relationships, apparent HPSE inhibition was tested for cyclitol **12,** its disaccharidic analogue **13** and parent HPSE inhibitor **2** (Figure 7a). Each of **12, 2** and **13** scored apparent HPSE IC₅₀ values in the low to sub-micromolar range (Table 1). In a qualitative comparison, **12** exhibited moderately lower inhibitory potency compared to **2.** Conversely, **13** could inactivate rHPSE down to 2 µM inhibitor concentration similar to **2.** Moreover, the collected evidence indicates that replacement of the 2'-*N*-Ac moiety in **2** for 2'-OH group with or without stabilisation of the α-1,4-glycosidic linkage does not significantly impair HPSE binding affinity. These results suggest that the HPSE -2 subsite can accommodate moderate modifications of the 2' position provided that the electron density, the steric properties and the spatial occupancy are not drastically altered.

**Table 1**

| ***In vitro* IC₅₀ (µM)** | **12** | **2** | **13** |
|---|---|---|---|
| **rHPSE^{a}** | 0.5 ± 0.5 | 0.3 ± 0.2 | 1 ± 1 |
| **HPSE (platelet)^{a}** | 0.4 ± 0.2 | 0.9 ± 0.3 | 5 ± 1 |

| | | | |
|---|---|---|---|
| ^{a} In-gel competitive ABPP-based assay | | | |

In another cABPP study, we observed selective inhibition of human platelet HPSE by both **2** and **3,** consistent with recombinant enzyme results, whilst **1** exclusively inhibited GUSB. Fluorescence quantitation gave IC50 values of ~530 nM and ~56 nM for platelet HPSE inhibition by **2** and **3** respectively, confirming the high potency and β-glucuronidase specificity of these inhibitors within complex physiological milieu (Figure 7b).

The trend in apparent HPSE IC₅₀ observed in platelet extracts for **2, 12** and **13** differs from the one found with recombinant HPSE. As shown in Figure 7c, preincubation with inhibitors **2, 12** and **13** effectively abrogates HPSE signal down to 2.5 µM inhibitor concentration without altering GUS signals. This demonstrates the inherent HPSE selectivity of **2, 12** and **13** over exo-acting isoforms. Moreover, candidate **12** scored a half order of magnitude lower apparent HPSE IC₅₀ value compared to **2** and **13,** both of which inactivated HPSE with similar low micromolar inhibitory potency under the tested conditions. Without wishing to be bound by any theory, the observed IC₅₀ value obtained for **12** in platelets may be due to an enhancement in metabolic stability rather than to an increase in HPSE binding affinity compared to precedent non-stabilised inhibitors

In a final cABPP study, fluorescent ABPs **7, 8** and **9** were applied to whole cell lysates from a panel of human cancer cell lines. Fluorescent pseudo-disaccharide 9 displayed markedly improved HPSE specificity compared to both **7** and **8** in all lysates tested, with the most substantial off-target of **9** being serum albumin at 10 µM ABP concentration (Figure 7d).

### Example 12: Molecular interaction between inhibitors and HPSE active site

To better understand the molecular basis of interaction between our inhibitors and their targets, we turned to X-ray crystallography, using ligand soaking to derivatize enzymes *in crystallo*.

Following soaking, **2, 3** and **4** were all observed to occupy the extended HPSE active site cleft, with covalent linkages between their pseudo-anomeric carbons and the enzyme nucleophile Glu343. The 6O'-sulfate of **3** adopted two equally populated configurations, both well placed for electrostatic interactions with basic residues around the HPSE substrate binding cleft (Lys158, Lys159, Lys161, Arg303). Non-covalent interactions for **2-4** within the HPSE cleft were similar to those previously characterized for HS¹⁵, albeit with a minor shift in the position of the NRE deoxy-sugar of **4,** due to its lack of 2' substituent (Figure 5b). Complexes of **2** and **3** with AcGH79 and BpHep displayed similar interaction modes as found for HPSE, indicating general GH79 family reactivity for HS-configured pseudo-disaccharides; HPSE is the sole active GH79 enzyme in humans. Consistent with cABPP profiles, we were only able to obtain an EcGUSB complex with **1,** which showed expected active site occupation and nucleophile labelling (Figure 8).

### Example 13: Effect of compounds on cleavage of HS oligosaccharide substrates

A colorimetric assay that detects sugar reducing ends liberated upon enzymatic cleavage of the synthetic HS pentasaccharide fondaparinux was used to investigate the ability of the compounds of the invention to abrogate HPSE cleavage of HS oligosaccharide substrates (Hammond et al., Anal Biochem, 396, 112-6, 2010).

Clear inhibition of HPSE mediated fondaparinux cleavage by both **2** (IC50 = ~0.46 µM) and **3** (IC50 = ~0.51 µM) was observed, both of which were approximately 2-fold more potent than reference small molecule HPSE inhibitors #98 (**10;** IC50 = ~0.80 µM; Figure 9) and OGT2115 (**11;** IC50 = ~0.95 µM; Figure 9). In contrast to cABPP assays, we did not observe improved potency for **3** compared to **2** against fondaparinux cleavage. Little HPSE inhibition was produced by **1,** even at 50 µM concentration (Figure 7e).

### Example 14: Effect of compounds on HPSE degradation of HSPGs

The compounds of the invention were assessed against HPSE degradation of bona fide HSPGs in a physiologically relevant ECM-like environment. Cultured bovine corneal epithelial cells secrete a subendothelial basement membrane material rich in HSPGs, and their growth in the presence of ³⁵SO₄²⁻ results in ³⁵S radiolabeled HSPGs (Vlodavsky et al., Curr Protoc Cell Biol, Chapter 10, Unit 10, 4, 2001). H³⁵S chains attached to these HSPGs are detectable after solubilization by HPSE, with enzyme inhibition leading to a corresponding reduction of signal.

Both **2** and **3** produced robust, dose dependent inhibition of recombinant HPSE in this assay, with comparable levels of inhibition at 1 µM **2** or **3** compared to 4.6 µM **10.** As with the fondaparinux assay, no HPSE inhibition was produced by **1,** and we observed no difference in potency between **2** and **3** (Figure 7f). Inhibition of endogenous cell lysate HPSE was also tested using this HSPG digest assay. We observed similar H³⁵S solubilization profiles effected by HPSE in U87 glioma-cell lysates whether inhibitors were applied before or after lysis, indicating our compounds are able to penetrate cells to inhibit the intracellular HPSE pool (Figure 10).

### Example 15: Effect of the compounds on cancer cell aggression

The Matrigel invasion assay was used to assess the effects of the compounds of the invention on cancer cell aggression. This is an *in cellulo* model of cell invasion through a HSPG rich basement membrane. The ability of cells to migrate through transwell inserts coated with Matrigel (an ECM secretion from Engelbreth-Holm-Swarm mouse sarcoma cells) correlates well with their *in vivo* invasive potential.

Using U87 glioblastoma cells, which show a strong HPSE dependent metastatic phenotype, we found that both **2** and **3** significantly reduced Matrigel invasion, suggesting potential efficacy as anti-metastatic agents (Figure 11a).

### Example 16: Effect of the compounds on metastasis of melanoma and breast cancer in vivo

The compounds of the invention were tested against a B16 murine melanoma experimental metastasis model, which measures the formation of metastatic lung tumors by B16 cancer cells after intravenous (i.v.) injection. Additionally, the compounds of the invention were tested against a second cancer model - 4T1 murine mammary carcinoma, which also metastasizes to the lungs upon injection.

In the B16 model, after 14 days, a marked reduction in lung growths for mice treated with **2** was observed compared to a PBS-only control group (Figure 11b). Remarkably, this reduction was similar in magnitude to that elicited by SST0001 (Roneparstat), a current 'best-in-class' phase Ilb clinical trial HPSE inhibitor (Figure 9). In the 4T1 model, a marked reduction in the incidence of lung metastases following treatment was observed, with similar efficacy to that achieved by SST0001 (Figure 11c). These murine models demonstrate that covalent HPSE inhibitors such as **2** can elicit anti-metastatic effects *in vivo,* with comparable efficacy to current HPSE inhibitor compounds.

In the CAG myeloma model, a marked reduction in metastatic burden was observed in mice treated with **2,** similar in magnitude to that achieved by bortezomib, albeit with lower efficacy following prolonged treatment (33 day timepoint). The reduction in antimetastatic efficacy at longer timepoints may reflect some metabolic instability in the structure of **2.** In contrast, a combined treatment regimen of **2+**bortezomib showed improved efficacy after 33 days compared to both monotherapies, highlighting clear potential for combined proteasomal inhibition and covalent HPSE inhibition over prolonged timecourses (Figure 11d).

## Claims

1. A compound of formula **(I),** or a pharmaceutically acceptable salt, solvate, or ester prodrug thereof: 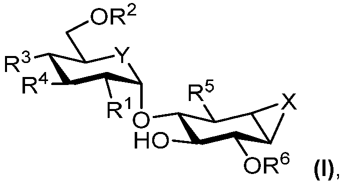 wherein
X is -O-;
Y is selected from -O-, -(CH₂)-, or -S-;
R' is selected from -H, -OH, -NHC(O)CH₃, -NHSO₃⁻;
R² is selected from -H or -SO₃⁻;
R³ is selected from -H, -OH, -O-C₁₋₆ alkyl, -L₂-fluorophore, or -L₂-biotin;
R⁴ is selected from -H, -OH, -O-C₁₋₆ alkyl or -SO₃⁻;
R⁵ is selected from: -C(O)O⁻, -C(O)OC₁₋₆ alkyl, -C(O)NH(C₁₋₆ alkyl), -OPO₃²⁻, -PO₃²⁻;
R⁶ is selected from -H or -SO₃⁻;
R^{a} is selected from C₁₋₆ alkyl, or C₁₋₆ alkyl interrupted by one or more ether linkages;
R^{b} is selected from C₁₋₆ alkyl, or C₁₋₆ alkyl interrupted by one or more ether linkages; and
L₂ is a linker which, when present, comprises a -O-C₂₋₂₄-alkyl or C₂₋₂₄-alkyl, optionally interrupted by one, two, or three of -NC(O)-, -NH-, -O-, -C(O)-, -C(OH)-, -C(O)O-, and heterocyclic ring system comprising 1,2,3-triazole;
fluorophore, when present, is selected from cyanine 2, cyanine 3, cyanine 5, BODIPY, fluorescein, tetramethylrhodamine.

2. The compound of claim 1, wherein R³ is selected from -H, -OH, -O-C₁₋₆ alkyl, optionally wherein R³ is -OH; and/or
wherein R⁴ is -OH; and/or
wherein R⁶ is -H.

3. The compound of any preceding claim, wherein R¹ is -NHC(O)Me, or wherein R' is - OH.

4. The compound of any preceding claim, wherein Y is -O-, or wherein Y is -(CH₂)-.

5. The compound of any preceding claim, wherein R² is -H, or wherein R² is -SO₃⁻; and/or
wherein R³ is -L₂-fluorophore, or -L₂-biotin.

6. The compound according to claim 1 of formula (XXIVa) or (XXIVb), or a pharmaceutically acceptable salt, solvate, or ester prodrug thereof: 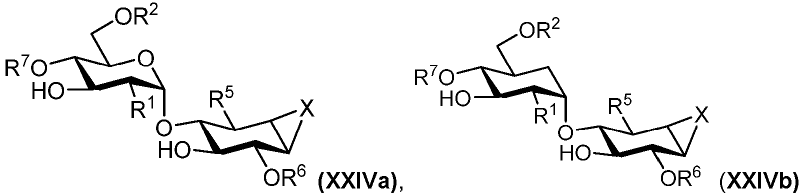 wherein
X is -O-;
R' is selected from -H, -OH, -NHC(O)CH₃, -NHSO₃⁻;
R² is selected from -H or -SO₃⁻;
R⁵ is selected from: -C(O)O⁻, -C(O)OC₁₋₆ alkyl, -C(O)NH(C₁₋₆ alkyl), -OPO₃²⁻, -PO₃²⁻;
R⁶ is selected from -H or -SO₃⁻;
R⁷ is selected from -H, -C₁₋₆ alkyl, -L₃-fluorophore, or -L₃-biotin;
R^{a} is selected from C₁₋₆ alkyl, or C₁₋₆ alkyl interrupted by one or more ether linkages;
R^{b} is selected from C₁₋₆ alkyl, or C₁₋₆ alkyl interrupted by one or more ether linkages; and
-L₃- is a linker which, when present, comprises a C₂₋₂₄-alkyl, optionally interrupted by one, two, or three of -NC(O)-, -NH-, -O-, -C(O)-, -C(OH)-, -C(O)O-, and heterocyclic ring system comprising 1,2,3-triazole;
fluorophore, when present, is selected from cyanine 2, cyanine 3, cyanine 5, BODIPY, fluorescein, tetramethylrhodamine.

7. The compound of claim 6, wherein R⁷ is selected from -L₃-fluorophore, or -L₃-biotin, optionally wherein R⁷ is -L₃-fluorophore; and/or
wherein the fluorophore is selected from cyanine 5 or BODIPY.

8. The compound of any preceding claim, wherein the compound is selected from the following, or a pharmaceutically acceptable salt, solvate, or ester prodrug thereof:

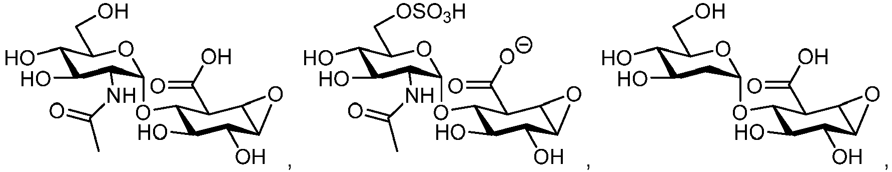

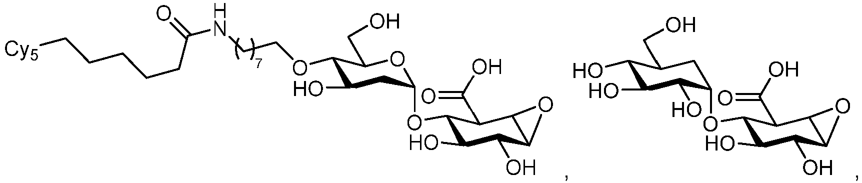

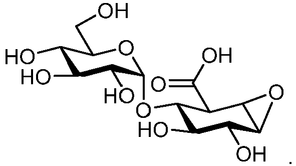

9. A pharmaceutical composition comprising a compound of any of preceding claim; optionally further comprising a pharmaceutically acceptable excipient.

10. The compound of any of claims 1 to 8, or the pharmaceutical composition of claim 9, for use as a medicament.

11. The compound of any of claims 1 to 8, or the pharmaceutical composition of claim 9, for use in the treatment of a condition which is modulated by heparanase (HPSE).

12. The compound of any of claims 1 to 8, or the pharmaceutical composition of claim 9, for use in the treatment of a condition selected from: cancer, inflammation, inflammatory lung injury, rheumatoid arthritis, chronic colitis, neuroinflammation, sepsis-associated lung injury, inflammatory bowel disease, diabetes, diabetic nephropathy, bone osteolysis, thrombosis, artherosclerosis, inflammatory kidney disease, acute kidney injury, and viral infection (e.g. viral infection caused by herpes simplex virus, dengue virus, human papillomavirus, respiratory syncytial virus, adenovirus, hepatitis C virus, porcine respiratory virus, reproductive syncytial virus, or retroviruses);
optionally wherein the condition is cancer.

13. A method of inhibiting heparanase (HPSE), comprising administration to a cell of an effective amount of a compound of any of claims 1 to 8, or a pharmaceutical composition of claim 9; wherein the administration is *in vitro.*

14. Use of the compound of any of claims 1 to 8, or of the pharmaceutical composition of claim 9, for the inhibition of heparanase (HPSE); wherein the use is *in vitro.*

15. Use of the compound of any of claims 1 to 8, or the pharmaceutical composition of claim 9, for the enzymatic labelling of heparanase (HPSE), wherein said compound comprises a fluorophore or a biotin; wherein the use is not a method for treatment of the human or animal body by therapy.

## Patentansprüche

1. Verbindung der Formel (I) oder pharmazeutisch unbedenkliches Salz, Solvat oder Ester-Prodrug davon: 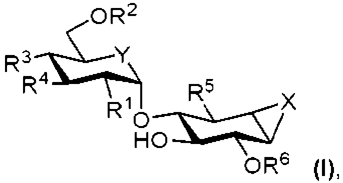 wobei
X -O- ist;
Y ausgewählt ist aus -O-, -(CH₂)- oder -S-;
R¹ ausgewählt ist aus -H, -OH, -NHC(O)CH₃, -NHSO₃⁻;
R² ausgewählt ist aus -H oder -SO₃⁻;
R³ ausgewählt ist aus -H, -OH, -O-C₁₋₆-Alkyl, -L₂-Fluorophor oder -L₂-Biotin;
R⁴ ausgewählt ist aus -H, -OH, -O-C₁₋₆-Alkyl oder -SO₃⁻;
R⁵ ausgewählt ist aus: -C(O)O⁻, -C(O)OC₁₋₆-Alkyl, -C(O)NH(C₁₋₆-Alkyl), -OPO₃²⁻, -PO₃²⁻;
R⁶ ausgewählt ist aus -H oder -SO₃⁻;
R^{a} ausgewählt ist aus C₁₋₆-Alkyl oder C₁₋₆-Alkyl, unterbrochen durch eine oder mehrere Etherbindungen;
R^{b} ausgewählt ist aus C₁₋₆-Alkyl oder C₁₋₆-Alkyl, unterbrochen durch eine oder mehrere Etherbindungen; und
L₂ ein Linker ist, der, wenn vorhanden, ein -O-C₂₋₂₄-Alkyl oder C₂₋₂₄-Alkyl, optional unterbrochen durch ein, zwei oder drei von -NC(O)-, -NH-, -O-, -C(O)-, -C(OH)-, -C(O)O- und einem heterocyclischen Ringsystem, das 1,2,3-Triazol umfasst, umfasst;
Fluorophor, wenn vorhanden, ausgewählt ist aus Cyanin 2, Cyanin 3, Cyanin 5, BODIPY, Fluorescein, Tetramethylrhodamin.

2. Verbindung nach Anspruch 1, wobei R³ ausgewählt ist aus -H, -OH, -O-C₁₋₆-Alkyl, wobei R³ optional -OH ist; und/oder
wobei R⁴ -OH ist; und/oder
wobei R⁶ -H ist.

3. Verbindung nach einem vorhergehenden Anspruch, wobei R¹ -NHC(O)Me ist oder wobei R¹ -OH ist.

4. Verbindung nach einem vorhergehenden Anspruch, wobei Y -O- ist oder wobei Y -(CH₂)-ist.

5. Verbindung nach einem vorhergehenden Anspruch, wobei R² -H ist oder wobei R² -SO₃⁻ist;
und/oder
wobei R³ -L₂-Fluorophor oder -L₂-Biotin ist.

6. Verbindung gemäß Anspruch 1 der Formel **(XXIVa)** oder **(XXIVb)** oder pharmazeutisch unbedenkliches Salz, Solvat oder Ester-Prodrug davon: 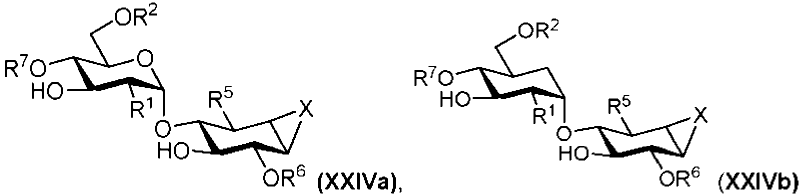 wobei
X -O- ist;
R¹ ausgewählt ist aus -H, -OH, -NHC(O)CH₃, -NHSO₃⁻;
R² ausgewählt ist aus -H oder -SO₃⁻;
R⁵ ausgewählt ist aus: -C(O)O⁻, -C(O)OC₁₋₆-Alkyl, -C(O)NH(C₁₋₆-Alkyl), -OPO₃²⁻, -PO₃²⁻;
R⁶ ausgewählt ist aus -H oder -SO₃⁻;
R⁷ ausgewählt ist aus -H, -C₁₋₆-Alkyl, -L₃-Fluorophor oder -L₃-Biotin;
R^{a} ausgewählt ist aus C₁₋₆-Alkyl oder C₁₋₆-Alkyl, unterbrochen durch eine oder mehrere Etherbindungen;
R^{b} ausgewählt ist aus C₁₋₆-Alkyl oder C₁₋₆-Alkyl, unterbrochen durch eine oder mehrere Etherbindungen; und
-L₃- ein Linker ist, der, wenn vorhanden, ein C₂₋₂₄-Alkyl, optional unterbrochen durch ein, zwei oder drei von -NC(O)-, -NH-, -O-, -C(O)-, -C(OH)-, -C(O)O- und ein heterocyclisches Ringsystem, das 1,2,3-Triazol umfasst, umfasst;
Fluorophor, wenn vorhanden, ausgewählt ist aus Cyanin 2, Cyanin 3, Cyanin 5, BODIPY, Fluorescein, Tetramethylrhodamin.

7. Verbindung nach Anspruch 6, wobei R⁷ ausgewählt ist aus -L₃-Fluorophor oder -L₃-Biotin,
wobei optional R⁷ -L₃-Fluorophor ist; und/oder
wobei das Fluorophor ausgewählt ist aus Cyanin 5 oder BODIPY.

8. Verbindung nach einem vorhergehenden Anspruch, wobei die Verbindung aus den folgenden ausgewählt ist, oder pharmazeutisch unbedenkliches Salz, Solvat oder Ester-Prodrug davon:

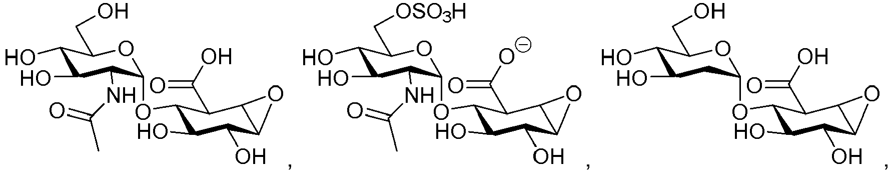

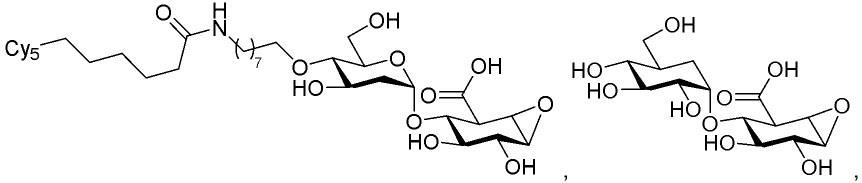

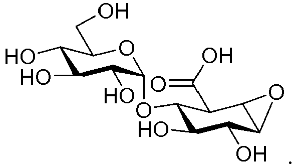

9. Pharmazeutische Zusammensetzung, umfassend eine Verbindung nach einem vorhergehenden Anspruch; optional ferner umfassend einen pharmazeutisch unbedenklichen Hilfsstoff.

10. Verbindung nach einem der Ansprüche 1 bis 8 oder pharmazeutische Zusammensetzung nach Anspruch 9 zur Verwendung als ein Medikament.

11. Verbindung nach einem der Ansprüche 1 bis 8 oder pharmazeutische Zusammensetzung nach Anspruch 9 zur Verwendung bei der Behandlung eines Leidens, das durch Heparanase (HPSE) moduliert ist.

12. Verbindung nach einem der Ansprüche 1 bis 8 oder pharmazeutische Zusammensetzung nach Anspruch 9 zur Verwendung bei der Behandlung eines Leidens, das ausgewählt ist aus Folgenden: Krebs, Entzündung, entzündlicher Lungenschädigung, rheumatoider Arthritis, chronischer Kolitis, Neuroinflammation, Sepsis-assoziierter Lungenschädigung, entzündlicher Darmkrankheit, Diabetes, diabetischer Nephropathie, Knochenosteolyse, Thrombose, Artherosklerose, entzündlicher Nierenkrankheit, akuter Nierenschädigung und Virusinfektion (z.
B. Virusinfektion, die durch Herpes-simplex-Virus, Dengue-Virus, Humanes Papillomvirus, Respiratorisches Synzytial-Virus, Adenovirus, Hepatitis-C-Virus, Porcines Respiratorisches Virus, Reproduktives Synzytial-Virus oder Retroviren verursacht wird); optional wobei das Leiden Krebs ist.

13. Verfahren zum Hemmen von Heparanase (HPSE), umfassend Verabreichung einer wirksamen Menge einer Verbindung nach einem der Ansprüche 1 bis 8 oder einer pharmazeutischen Zusammensetzung nach Anspruch 9 an eine Zelle; wobei die Verabreichung *in vitro* erfolgt.

14. Verwendung der Verbindung nach einem der Ansprüche 1 bis 8 oder der pharmazeutischen Zusammensetzung nach Anspruch 9, zur Hemmung von Heparanase (HPSE); wobei die Verwendung *in vitro* erfolgt.

15. Verwendung der Verbindung nach einem der Ansprüche 1 bis 8 oder der pharmazeutischen Zusammensetzung nach Anspruch 9 zur enzymatischen Markierung von Heparanase (HPSE), wobei die Verbindung ein Fluorophor oder ein Biotin umfasst; wobei die Verwendung kein Verfahren zur Behandlung des menschlichen oder tierischen Körpers durch Therapie ist.

## Revendications

1. Composé de formule (I), ou sel, solvate ou promédicament ester pharmaceutiquement acceptable de celui-ci : 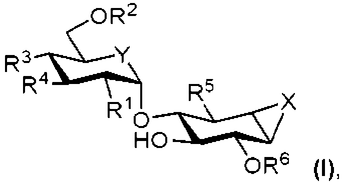 dans lequel
X est -O- ;
Y est choisi parmi -O-, -(CH₂)- ou -S- ;
R¹ est choisi parmi -H, -OH, -NHC(O)CH₃, -NHSO₃⁻ ;
R² est choisi parmi -H ou -SO₃⁻ ;
R³ est choisi parmi -H, -OH, -O-alkyle en C₁₋₆, -L₂-fluorophore ou -L₂-biotine ;
R⁴ est choisi parmi -H, -OH, -O-alkyle en C₁₋₆ ou -SO₃⁻ ;
R⁵ est choisi parmi : -C(O)O⁻, -C(O)O-alkyle en C₁₋₆, -C(O)NH(alkyle en C₁₋₆), -OPO₃²⁻, -PO₃²⁻ ;
R⁶ est choisi parmi -H ou -SO₃⁻ ;
R^{a} est choisi parmi un alkyle en C₁₋₆ ou un alkyle en C₁₋₆ interrompu par une ou plusieurs liaisons éther ;
R^{b} est choisi parmi un alkyle en C₁₋₆ ou un alkyle en C₁₋₆ interrompu par une ou plusieurs liaisons éther ; et
L₂ est un coupleur qui, lorsqu'il est présent, comprend un -O-alkyle en C₂₋₂₄ ou un alkyle en C₂₋₂₄, éventuellement interrompu par un, deux ou trois parmi -NC(O)-, -NH-, -O-, -C(O)-, -C(OH)-, - C(O)O- et un système de cycle hétérocyclique comprenant du 1,2,3-triazole ;
un fluorophore, lorsqu'il est présent, est choisi parmi la cyanine 2, la cyanine 3, la cyanine 5, BODIPY, la fluorescéine, la tétraméthylrhodamine.

2. Composé de la revendication 1, dans lequel R³ est choisi parmi -H, -OH, -O-alkyle en C₁₋₆, éventuellement dans lequel R³ est -OH ; et/ou
dans lequel R⁴ est -OH ; et/ou
dans lequel R⁶ est -H.

3. Composé d'une quelconque revendication précédente, dans lequel R¹ est -NHC(O)Me, ou dans lequel R¹ est -OH.

4. Composé d'une quelconque revendication précédente, dans lequel Y est -O-, ou dans lequel Y est -(CH₂)-.

5. Composé d'une quelconque revendication précédente, dans lequel R² est -H, ou dans lequel
R² est -SO₃⁻ ; et/ou
dans lequel R³ est -L₂-fluorophore ou -L₂-biotine.

6. Composé selon la revendication 1 de formule (XXIVa) ou (XXIVb), ou sel, solvate ou promédicament ester pharmaceutiquement acceptable de celui-ci : 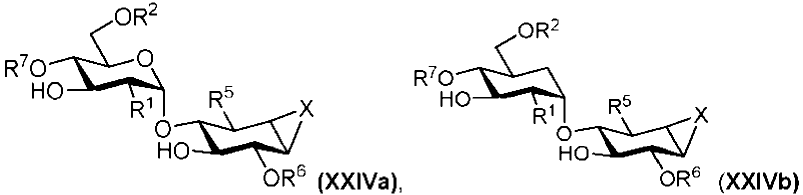 dans lequel
X est -O- ;
R¹ est choisi parmi -H, -OH, -NHC(O)CH₃, -NHSO₃⁻ ;
R² est choisi parmi -H ou -SO₃⁻ ;
R⁵ est choisi parmi : -C(O)O⁻, -C(O)O-alkyle en C₁₋₆, -C(O)NH(alkyle en C₁₋₆), -OPO₃²⁻, -PO₃²⁻;
R⁶ est choisi parmi -H ou -SO₃⁻ ;
R⁷ est choisi parmi -H, -alkyle en C₁₋₆, -L₃-fluorophore ou -L₃-biotine ;
R^{a} est choisi parmi un alkyle en C₁₋₆ ou un alkyle en C₁₋₆ interrompu par une ou plusieurs liaisons éther ;
R^{b} est choisi parmi un alkyle en C₁₋₆ ou un alkyle en C₁₋₆ interrompu par une ou plusieurs liaisons éther ; et -L₃- est un coupleur qui, lorsqu'il est présent, comprend un alkyle en C₂₋₂₄, éventuellement interrompu par un, deux ou trois parmi -NC(O)-, -NH-, -O-, -C(O)-, -C(OH)-, - C(O)O- et un système de cycle hétérocyclique comprenant du 1,2,3-triazole ;
un fluorophore, lorsqu'il est présent, est choisi parmi la cyanine 2, la cyanine 3, la cyanine 5, BODIPY, la fluorescéine, la tétraméthylrhodamine.

7. Composé de la revendication 6, dans lequel R⁷ est choisi parmi -L₃-fluorophore ou -L₃-biotine, éventuellement dans lequel R⁷ est -L₃-fluorophore ; et/ou
dans lequel le fluorophore est choisi parmi la cyanine 5 ou BODIPY.

8. Composé d'une quelconque revendication précédente, ledit composé étant choisi parmi les composés suivants, ou sel, solvate ou promédicament ester pharmaceutiquement acceptable de celui-ci :

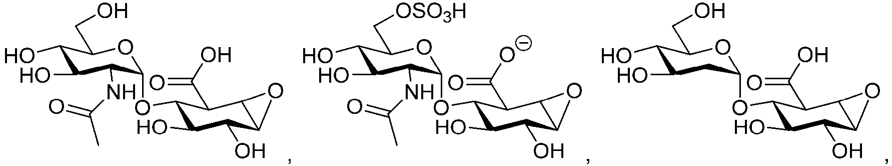

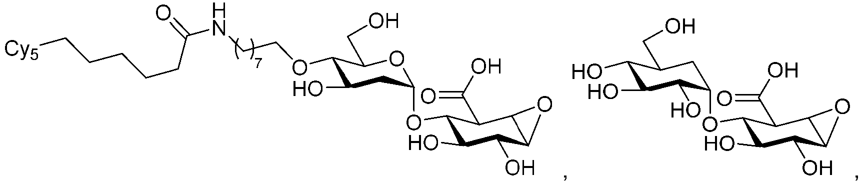

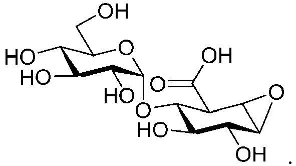

9. Composition pharmaceutique comprenant un composé d'une quelconque revendication précédente ; comprenant en outre éventuellement un excipient pharmaceutiquement acceptable.

10. Composé de l'une quelconque des revendications 1 à 8, ou composition pharmaceutique de la revendication 9, destiné(e) à être utilisé(e) en tant que médicament.

11. Composé de l'une quelconque des revendications 1 à 8, ou composition pharmaceutique de la revendication 9, destiné(e) à être utilisé(e) dans le traitement d'une affection qui est modulée par l'héparanase (HPSE).

12. Composé de l'une quelconque des revendications 1 à 8, ou composition pharmaceutique de la revendication 9, destiné(e) à être utilisé(e) dans le traitement d'une affection choisie parmi : un cancer, une inflammation, une lésion pulmonaire inflammatoire, la polyarthrite rhumatoïde, la colite chronique, une neuroinflammation, une lésion pulmonaire associée à la sepsie, une maladie inflammatoire de l'intestin, le diabète, la néphropathie diabétique, l'ostéolyse osseuse, la thrombose, l'athérosclérose, une maladie rénale inflammatoire, une lésion rénale aiguë et une infection virale (par exemple, une infection virale causée par le virus de l'herpès simplex, le virus de la dengue, le papillomavirus humain, le virus respiratoire syncytial, l'adénovirus, le virus de l'hépatite C, le virus respiratoire porcin, le virus syncytial reproducteur ou les rétrovirus) ; éventuellement, ladite affection étant un cancer.

13. Procédé d'inhibition de l'héparanase (HPSE), comprenant l'administration à une cellule d'une quantité efficace d'un composé de l'une quelconque des revendications 1 à 8, ou d'une composition pharmaceutique de la revendication 9 ; dans lequel l'administration se fait *in vitro.*

14. Utilisation du composé de l'une quelconque des revendications 1 à 8, ou de la composition pharmaceutique de la revendication 9, pour l'inhibition de l'héparanase (HPSE) ; ladite utilisation étant *in vitro.*

15. Utilisation du composé de l'une quelconque des revendications 1 à 8, ou de la composition pharmaceutique de la revendication 9, pour le marquage enzymatique de l'héparanase (HPSE), ledit composé comprenant un fluorophore ou une biotine ; ladite utilisation n'étant pas un procédé de traitement du corps humain ou animal par thérapie.
